# EUROPEAN PATENT APPLICATION

(11) **EP 4 782 438 A1**
(43) Date of publication of application: **29.07.2026**
(21) Application number: 24884827.7
(22) Date of filing: 30.10.2024
(51) Int. Cl.: C07D 303/36, C07D 405/12, C07D 407/12, A61K 31/336, A61K 31/16, C07K 5/097, A61P 29/00

(54) **EPOXY COMPOUND AND USE THEREOF**

(30) Priority: 30.10.2023 CN 202311420632; 05.07.2024 CN 202410901865
(71) Applicant: Shanghai Huilun Pharmaceutical Co., Ltd., Shanghai 200241 (CN)
(72) Inventor: TANG, Shuangqi, Shanghai 200241 (CN); WANG, Xinfeng, Shanghai 200241 (CN); HUANG, Shengcai, Shanghai 200241 (CN); HUANG, Qibin, Shanghai 200241 (CN); HUANG, Guoqi, Shanghai 200241 (CN); DU, Shuqi, Shanghai 200241 (CN); CHEN, Yeying, Shanghai 200241 (CN); CHEN, Lei, Shanghai 200241 (CN); QIN, Jihong, Shanghai 200241 (CN)
(74) Representative: IK-IP LTD
(86) International application number: PCT/CN2024/128540
(87) International publication number: WO 2025/092827

(57) **Abstract**

An epoxy compound and a use thereof. Specifically disclosed are an epoxy compound as shown in formula I, a pharmaceutically acceptable salt or stereoisomer and use thereof. The compound has high inhibitory activity on β5i and β1i, but has a low inhibition level on β5c and β1c. The compound has relatively high selectivity and can be used for treating autoimmune diseases.

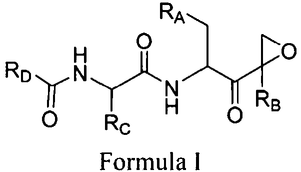

## Description

The present application claims priority to Chinese Patent Application No. 2023114206329 filed on October 30, 2023, and priority to Chinese Patent Application No. 2024109018659 filed on July 5, 2024. The contents of the above Chinese Patent Applications are incorporated herein by reference in their entireties.

### TECHNICAL FIELD

The present invention relates to an epoxy compound and use thereof.

### BACKGROUND

The ubiquitin proteasome system (UPS) includes ubiquitin, ubiquitin-activating enzyme (E1), ubiquitin-conjugating enzyme (E3), ubiquitin ligase (E3), 26S proteasome and deubiquitinating enzyme. The degradation of proteins by UPS includes two key steps: ubiquitination and degradation of a target protein, in which the degradation of the target protein requires the participation of 26S proteasome.

The 26S proteasome includes the catalytic core 20S proteasome and 2 19S lids, is widely expressed in mammalian cells, and is also called constitutive proteasome. The 20S proteasome is a barrel-shaped structure formed by stacking 2 outer-ring α chains and 2 inner-ring β chains, where each chain consists of 7 α subunits or 7 β subunits respectively. The outer ring has no catalytic activity and the inner ring is the catalytic center. The specificity of the 20S proteasome for a substrate depends on the cleavage mode of the peptide bonds on N2 terminal threonine residues of β1, β2 and β5 subunits. The protease activities corresponding to β1, β2 and β5 subunits are caspase activity (cleaving acidic amino acid residues), trypsin activity (cleaving basic amino acid residues), and chymotrypsin activity (cleaving hydrophobic amino acid residues) respectively. The structure of the 20S proteasome is not constant. In some cells, under the induction by stimulus such as IFN-γ, TNF-α, NO, oxidative stress and, inflammatory reaction, the β1, β2 and β5 subunits in the constitutive proteasome can be replaced by β1i (LMP2), β21 (MECL-1) and β5i (LMP7), respectively to form a new 20S proteasome. Because LMP2, MECL-1 and LMP7 can enhance the proteasome's ability to produce a polypeptide that binds to major histocompatibility complex (MHC-1), the 20S proteasome containing these three subunits is also known as immunoproteasome. The immunoproteasome is constitutively expressed in immune cells (lymphocytes, and monocytes, etc.), and other non-immune cells such as retinal pigment epithelial cells, vascular endothelial cells and nerve cells can also express immunoproteasome under the induction of cytokines.

The immunoproteasome is not only involved in the processing of antigens, but also responsible for regulating the production of cytokines. The immunoproteasome is related to the pathogenesis of autoimmune diseases. After the LMP7 selective inhibitor ONX0914 (PR-957) is administered, IL-23 secreted by monocytes and IL-2 secreted by T cells decrease significantly. In the disease model of rheumatoid arthritis in mice, the anti-inflammatory effect of inhibiting LMP7 is remarkable, and the joint pathological score is obviously improved. It is found in the gene sequencing study of autosomal recessive diseases in the human that the deletion of *PSMB8* gene (encoding LMP7) is related to the symptoms of various autoimmune diseases.

WO2014152134 discloses a tripeptide cyclooxygenase inhibitor that can be used to treat inflammation and neurodegenerative diseases. However, there is still a further need to find a protease inhibitor with high selectivity and high activity.

### SUMMARY

The present invention provides an epoxy compound of Formula I, or a pharmaceutically acceptable salt or a stereoisomer thereof, 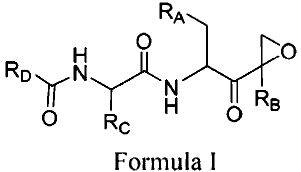 wherein
R_{A} is C₁₋₆ alkyl, cyclopentenyl, cyclohexenyl, cyclohexyl, cyclopentyl, or C₆₋₁₄ aryl, in which the C₁₋₆ alkyl, cyclopentenyl, cyclohexenyl, cyclohexyl, cyclopentyl and C₆₋₁₄ aryl are optionally substituted with one or more R_{A1};
R_{A1} is independently C₁₋₆ alkyl, C₁₋₆ alkoxy, halogen, hydroxyl, or C₁₋₆ haloalkyl;
R_{B} is C₁₋₆ alkyl or C₁₋₆ haloalkyl;
R_{C} is 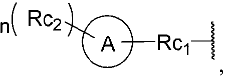 C₁₋₆ alkyl, or C₁₋₆ alkyl substituted with one or more Rc₃;
ring A is phenyl, 4- to 7-membered heterocycloalkyl, 8- to 10-membered heterocycloalkyl, 5- to 8-membered heteroaryl, C₄₋₇ cycloalkyl, pyridinonyl, or C₄₋₇ cycloalkenyl;
Rc₁ is C₁₋₆ alkylene optionally substituted with one or more R_{C1-1}; R_{C1-1} is hydroxyl, C₁₋₆ alkoxy, or halogen;
Rc₂ is C₁₋₆ alkyl, C₁₋₆ alkoxy, halogen, hydroxyl, C₃₋₆ cycloalkoxy, NH(C₁₋₆ alkyl), or N(C₁₋₆ alkyl)₂; or two adjacent Rc₂ form, together with the atoms to which they are attached, a 4- to 6-membered ring;
Rc₃ is independently hydroxyl, halogen, C₁₋₆ alkoxy, or C₁₋₆ alkoxy substituted with one or more Rc₃₋₁;
Rc₃₋₁ is independently C₁₋₆ haloalkyl or C₃₋₈cycloalkyl;
R_{D} is 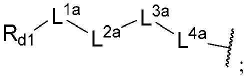
L^{4a} is a bond, C₁₋₆ alkylene, C₂₋₆ alkenylene, C₃₋₈ cycloalkylene, or 3- to 8-membered heterocycloalkylene, in which the C₁₋₆ alkylene, C₂₋₆ alkenylene, C₃₋₈ cycloalkylene, and 3- to 8-membered heterocycloalkylene are optionally substituted with one or more L^{4a-1};
L^{4a-1} is hydroxyl, halogen, oxo (=O), C₁₋₆ alkylsulfonamido, or C₁₋₆ alkoxy;
L^{3a}, L^{2a}, and L^{1a} are each independently a bond, -O-, -NH-, -N(C₁₋₆ alkyl)-, carbonyl (-C=O-), C₁₋₆ alkylene, sulfonyl (-SO₂-), -C(=O)NH-, -NHC(=O)-, or -NHC(=O)NH-;
R_{d1} is C₆₋₁₄ aryl, C₁₋₆ alkyl, C₃₋₁₀ cycloalkyl, 3- to 10-membered heterocycloalkyl, C₃₋₆ cycloalkyloxy, 3- to 8-membered heterocycloalkyloxy, 5- to 8-membered heteroaryl, or C₁₋₆ alkylsulfonyl, in which the C₆₋₁₄ aryl, C₁₋₆ alkyl, C₃₋₁₀ cycloalkyl, 3- to 10-membered heterocycloalkyl, C₃₋₆ cycloalkyloxy, 3- to 8-membered heterocycloalkyloxy, 5- to 8-membered heteroaryl, and C₁₋₆ alkylsulfonyl are optionally substituted with one or more R_{d1-1};
R_{d1-1} is hydroxyl, halogen, C₁₋₆ alkyl, C₁₋₆ alkoxy, C₁₋₆ haloalkyl, C₁₋₆ haloalkoxy, oxo (=O), C₁₋₆ alkylcarbonyl, C₁₋₆ alkoxycarbonyl, C₁₋₆ alkylsulfonyl, C₃₋₆cycloalkylsulfonyl, 3- to 6-membered heterocycloalkyl, 3- to 6-membered heterocycloalkylmethylene, C₃₋₆cycloalkyl, or C₆₋₁₄ aryl-C₁₋₆ alkylene-;
n is 0, 1, 2 or 3; and
in the 3- to 8-membered heterocycloalkylene, 3- to 10-membered heterocycloalkyl, 3- to 8-membered heterocycloalkyloxy, 4- to 7-membered heterocycloalkyl, 8- to 10-membered heterocycloalkyl, 3- to 6-membered heterocycloalkyl, 3- to 6-membered heterocycloalkylmethylene, and 5- to 8-membered heteroaryl, the number of the heteroatom or heteroatom group is independently 1, 2, 3, or 4, and the heteroatom or heteroatom group is independently one or more of N, O, S, and -SO₂-.

In some preferred embodiments of the present invention, R_{A} is cyclopentenyl, cyclohexenyl, cyclohexyl, cyclopentyl, or C₆₋₁₄ aryl, in which the cyclopentenyl, cyclohexenyl, cyclohexyl, cyclopentyl and C₆₋₁₄ aryl are optionally substituted with one or more R_{A1};
R_{A1} is C₁₋₆ alkyl, C₁₋₆ alkoxy, halogen, or hydroxyl;
R_{B} is C₁₋₆ alkyl or C₁₋₆ haloalkyl;
R_{C} is 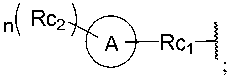
ring A is phenyl, 4- to 7-membered heterocycloalkyl, 5- to 8-membered heteroaryl, or C₄₋₇cycloalkyl;
Rc₁ is C₁₋₆ alkylene optionally substituted with one or more R_{C1-1}; R_{C1-1} is hydroxyl, C₁₋₆ alkoxy, or halogen;
Rc₂ is C₁₋₆ alkyl, C₁₋₆ alkoxy, halogen, hydroxyl, C₃₋₆ cycloalkoxy, NH(C₁₋₆ alkyl), or N(C₁₋₆ alkyl)₂; or two adjacent Rc₂ form, together with the atoms to which they are attached, a 4- to 6-membered ring;
R_{D} is 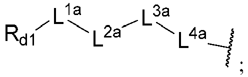
L^{4a} is C₁₋₆ alkylene, C₂₋₆ alkenylene, C₃₋₈ cycloalkylene, or 3- to 8-membered heterocycloalkylene, in which the C₁₋₆ alkylene, C₂₋₆ alkenylene, C₃₋₈ cycloalkylene, and 3- to 8-membered heterocycloalkylene are optionally substituted with one or more L^{4a-1};
L^{4a-1} is hydroxyl, halogen, oxo (=O), C₁₋₆ alkylsulfonamido, or C₁₋₆ alkoxy;
L^{3a}, L^{2a}, and L^{1a} are each independently a bond, -O-, -NH-, -N(C₁₋₆ alkyl)-, carbonyl (-C=O-), C₁₋₆ alkylene, sulfonyl (-SO₂-), -C(=O)NH-, -NHC(=O)-, or -NHC(=O)NH-;
R_{d1} is C₆₋₁₄ aryl, C₁₋₆ alkyl, C₃₋₁₀ cycloalkyl, 3- to 10-membered heterocycloalkyl, C₃₋₆ cycloalkyloxy, 3- to 8-membered heterocycloalkyloxy, 5- to 8-membered heteroaryl, or C₁₋₆ alkylsulfonyl, in which the C₆₋₁₄ aryl, C₁₋₆ alkyl, C₃₋₁₀ cycloalkyl, 3- to 10-membered heterocycloalkyl, C₃₋₆ cycloalkyloxy, 3- to 8-membered heterocycloalkyloxy, 5- to 8-membered heteroaryl, and C₁₋₆ alkylsulfonyl are optionally substituted with one or more R_{d1-1};
R_{d1-1} is hydroxyl, halogen, C₁₋₆ alkyl, C₁₋₆ alkoxy, C₁₋₆ haloalkyl, C₁₋₆ haloalkoxy, oxo (=O), C₁₋₆ alkylcarbonyl, C₁₋₆ alkoxycarbonyl, C₁₋₆ alkylsulfonyl, C₃₋₆cycloalkylsulfonyl, 3- to 6-membered heterocycloalkyl, 3- to 6-membered heterocycloalkylmethylene, C₃₋₆cycloalkyl, or C₆₋₁₄ aryl-C₁₋₆ alkylene-;
n is 0, 1, 2 or 3; and
in the 3- to 8-membered heterocycloalkylene, 3- to 10-membered heterocycloalkyl, 3- to 8-membered heterocycloalkyloxy, 3- to 6-membered heterocycloalkyl, and 3- to 6-membered heterocycloalkylmethylene, the number of the heteroatom or heteroatom group is independently 1, 2, 3, or 4, and the heteroatom or heteroatom group is independently one or more of N, O, S, and -SO₂-.
In some preferred embodiments of the present invention, R_{A} is cyclopentenyl, cyclohexenyl, cyclohexyl, cyclopentyl or phenyl, in which the cyclopentenyl, cyclohexenyl, cyclohexyl, cyclopentyl and phenyl are optionally substituted with one or more R_{A1}; and R_{A1} is C₁₋₆ haloalkyl; or R_{A} is C₁₋₆ alkyl;
R_{C} is C₁₋₆ alkyl, or C₁₋₆ alkyl substituted with one or more Rc₃;
ring A is 8- to 10-membered heterocycloalkyl, pyridinonyl, or C₄₋₇ cycloalkenyl;
Rc₃ is independently hydroxyl, halogen, C₁₋₆ alkoxy, or C₁₋₆ alkoxy substituted with one or more Rc₃₋₁;
Rc₃₋₁ is independently C₁₋₆ haloalkyl or C₃₋₈cycloalkyl;
L^{4a} is a bond; and
in the 8- to 10-membered heterocycloalkylene, the number of the heteroatom or heteroatom group is independently 1, 2, 3, or 4, and the heteroatom or heteroatom group is independently one or more of N, O, S, and -SO₂-.

In some preferred embodiments of the present invention, Formula I has a spatial configuration below: 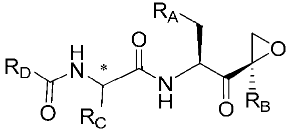 in which * means that the carbon atom is a chiral atom having R configuration or S configuration.

In some preferred embodiments of the present invention, R_{A} is cyclopentenyl, cyclohexenyl, cyclohexyl, cyclopentyl or phenyl, in which the cyclopentenyl, cyclohexenyl, cyclohexyl, cyclopentyl and phenyl are optionally substituted with one or more R_{A1}; and R_{A1} is C₁₋₆ alkyl, C₁₋₆ alkoxy, halogen, or hydroxyl.

In some preferred embodiments of the present invention, R_{A} is cyclopentenyl, cyclohexenyl, cyclohexyl, cyclopentyl or phenyl, in which the cyclopentenyl, cyclohexenyl, cyclohexyl, cyclopentyl and phenyl are optionally substituted with one or more R_{A1}; and R_{A1} is C₁₋₆ haloalkyl.

In some preferred embodiments of the present invention, R_{A} is cyclopentenyl, cyclohexenyl, cyclohexyl, cyclopentyl or phenyl, in which the cyclopentenyl, cyclohexenyl, cyclohexyl, cyclopentyl and phenyl are optionally substituted with one or more R_{A1}; and R_{A1} is methyl, halogen, or hydroxyl.

In some preferred embodiments of the present invention, R_{A} is cyclopentenyl, cyclohexenyl, cyclohexyl, cyclopentyl or phenyl, in which the cyclopentenyl, cyclohexenyl, cyclohexyl, cyclopentyl and phenyl are optionally substituted with one or more R_{A1}; and R_{A1} is ethyl, isopropyl, methoxy, ethoxy, or trifluoromethyl.

In some preferred embodiments of the present invention, R_{A} is cyclopentenyl, cyclohexenyl, cyclohexyl, cyclopentyl or phenyl, in which the cyclopentenyl, cyclohexenyl, cyclohexyl, cyclopentyl and phenyl are optionally substituted with one or more R_{A1}; and R_{A1} is methyl, ethyl, isopropyl, methoxy, ethoxy, halogen, hydroxyl, or trifluoromethyl.

In some preferred embodiments of the present invention, R_{A} is

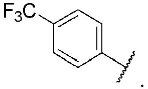

In some preferred embodiments of the present invention, R_{A} is cyclopentenyl (for example, 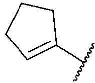 ).

In some preferred embodiments of the present invention, R_{A} is cyclohexenyl (for example, 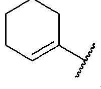 ).

In some preferred embodiments of the present invention, R_{A} is cyclohexyl.

In some preferred embodiments of the present invention, R_{A} is cyclopentyl.

In some preferred embodiments of the present invention, R_{A} is phenyl, phenyl substituted with one or more methyl (for example, 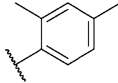 ), or phenyl substituted with one or more halogens (for example, 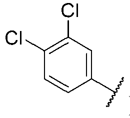 ).

In some preferred embodiments of the present invention, R_{A} is

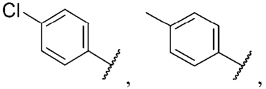

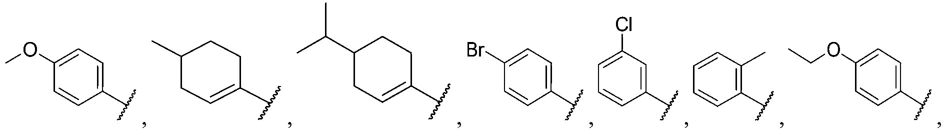

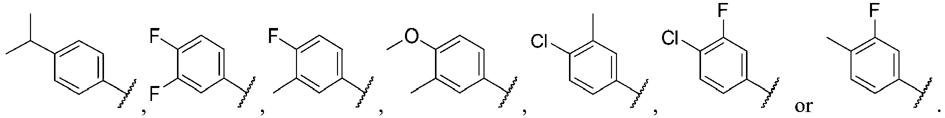

In some preferred embodiments of the present invention, R_{A} is C₁₋₆ alkyl.

In some preferred embodiments of the present invention, R_{A} is

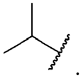

In some preferred embodiments of the present invention, R_{A1} is independently fluoro, chloro, bromo, methyl, methoxy, ethoxy, isopropyl, hydroxyl, or trifluoromethyl.

In some preferred embodiments of the present invention, R_{B} is methyl, ethyl, or trifluoromethyl.

In some preferred embodiments of the present invention, Formula I has a structure of Formula I-a1:

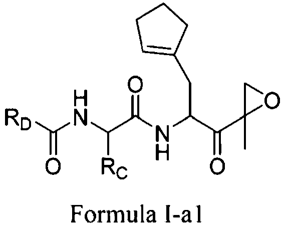

In Formula I-a1, R_{D} and R_{C} have the same definitions as in Formula I.

In some preferred embodiments of the present invention, R_{C} is

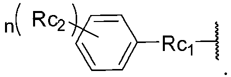

In some preferred embodiments of the present invention, ring A is 8- to 10-membered heterocycloalkyl, pyridinonyl, or C₄₋₇ cycloalkenyl.

In some preferred embodiments of the present invention, R_{C1} is methylene substituted with one or more R_{C1-1}, and R_{C1-1} is hydroxyl or halogen.

In some preferred embodiments of the present invention, Rc₁ is methylene optionally substituted with one or more R_{C1-1}, and R_{C1-1} is hydroxyl or halogen.

In some preferred embodiments of the present invention, Rc₁ is methylene.

In some preferred embodiments of the present invention, R_{C} is C₁₋₆ alkyl, or C₁₋₆ alkyl substituted with one or more Rc₃; Rc₃ is independently hydroxyl, halogen, C₁₋₆ alkoxy, or C₁₋₆ alkoxy substituted with one or more Rc₃₋₁; and
Rc₃₋₁ is independently C₁₋₆ haloalkyl or C₃₋₈ cycloalkyl.

In some preferred embodiments of the present invention, R_{C} is methyl, ethyl, or t-butyl, in which the methyl, ethyl and t-butyl are optionally substituted with one or more Rc₃.

In some preferred embodiments of the present invention, R_{C3} is independently hydroxyl, or methoxy optionally substituted with one or more R_{C3-1}.

In some preferred embodiments of the present invention, R_{C3-1} is independently trifluoromethyl or cyclopropyl.

In some preferred embodiments of the present invention, Formula I or Formula I-a1 has a structure of Formula I-c1:

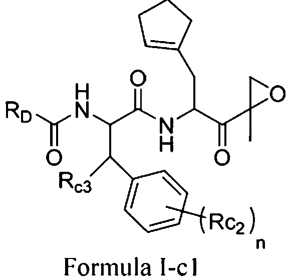

In Formula I-c1, R_{D} and Rc₂ have the same definitions as in Formula I; and Rc₃ is hydrogen, hydroxyl, C₁₋₆ alkoxy, or halogen.

In some preferred embodiments of the present invention, Rc₂ is methyl, methoxy, halogen, hydroxyl, NH(C₁₋₆ alkyl), or N(C₁₋₆ alkyl)₂.

In some preferred embodiments of the present invention, Rc₂ is methoxy, cyclopropoxy or chloro.

In some preferred embodiments of the present invention, two Rc₂ form, together with the atoms to which they are attached, a 4-membered ring, a 5-membered ring or a 6-membered ring.

In some preferred embodiments of the present invention, two Rc₂ form, together with the atoms to which they are attached, 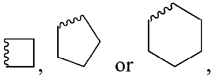 in which " 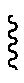 " indicates a position for forming a fused ring with ring A (e.g. benzene ring).

In some preferred embodiments of the present invention, R_{C} is 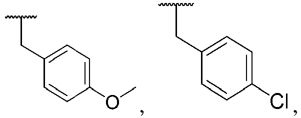 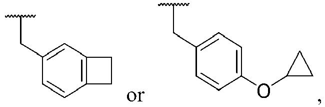 and preferably

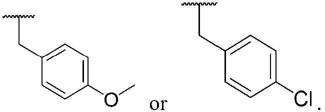

In some preferred embodiments of the present invention, R_{C} is

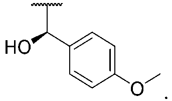

In some preferred embodiments of the present invention, R_{C} is 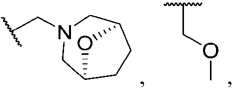 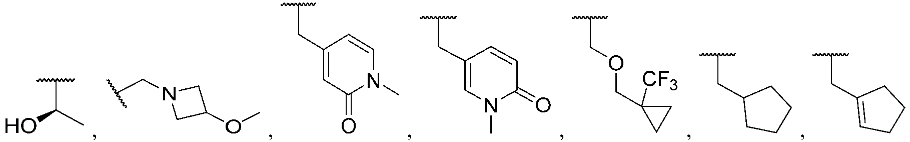 or

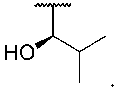

In some preferred embodiments of the present invention, R_{C} is

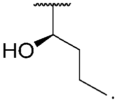

In some preferred embodiments of the present invention, R_{C} is

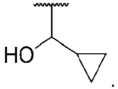

In some preferred embodiments of the present invention, in R_{D}, when L^{4a} is C₃₋₈ cycloalkylene optionally substituted with one or more L^{4a-1}, C₃₋₈ cycloalkylene is C₃₋₆ monocycloalkylene or C₅₋₈ spirocycloalkylene.

In some preferred embodiments of the present invention, in R_{D}, L^{4a} is 3- to 8-membered heterocycloalkylene optionally substituted with one or more L^{4a-1}, the 3- to 8-membered heterocycloalkylene is 3- to 6-membered heteromonocycloalkylene or 5- to 8-membered heterospirocycloalkylene.

In some preferred embodiments of the present invention, in R_{D}, L^{4a} is C₃₋₆ monocycloalkylene or 3- to 6-membered heteromonocycloalkylene. The C₃₋₆ monocycloalkylene is optionally selected from cyclopropylene, cyclobutylene, cyclopentylene and cyclohexylene. The cycloalkylene may be substituted at the same carbon atom, at adjacent carbon atoms, or at carbon atoms intervened by one or two atoms. The 3-to 6-membered heteromonocycloalkylene is optionally selected from heterocyclopropylene, heterocyclobutylene, heterocyclopentylene, and heterocyclohexylene. The heteromonocycloalkylene may be substituted at the same carbon atom, at adjacent carbon atoms, or at carbon atoms intervened by one or two atoms.

In some preferred embodiments of the present invention, in R_{D}, L^{4a} is cyclopropylene or heterocyclopropylene.

Preferably, the cyclopropylene has the following two substituted forms: 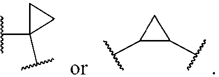 Preferably, the heterocyclopropylene has the following two substituted forms: 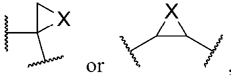 and X is O, S or NH.

In some preferred embodiments of the present invention, L^{4a} in R_{D} is cyclobutylene or heterocyclobutylene.

Preferably, the cyclobutylene has the following three substituted forms: 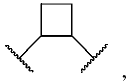 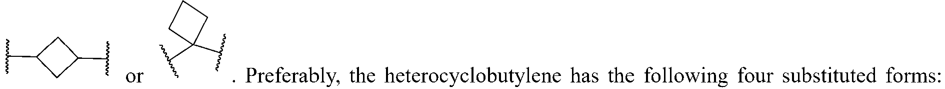 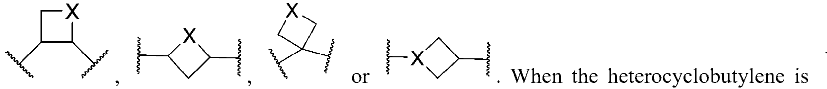 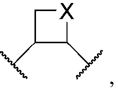 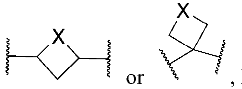 X is O, S or NH. When the heterocyclobutylene is 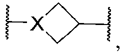 X is N.

In some preferred embodiments of the present invention, in R_{D}, L^{4a} is cyclopropylene, cyclobutylene, oxacyclobutylene, azacyclobutylene, or azacyclohexylene.

In some preferred embodiments of the present invention, in R_{D}, L^{4a} is

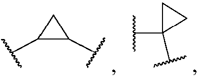

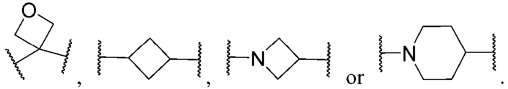

In some preferred embodiments of the present invention, in R_{D}, L^{4a} is a bond, cyclopentylene, or azacyclohexylene substituted with one or more L^{4a-1}.

In some preferred embodiments of the present invention, in R_{D}, L^{4a} is a bond, 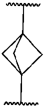 or

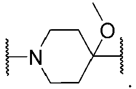

In some preferred embodiments of the present invention, in R_{D}, when R_{d1} is C₃₋₁₀cycloalkyl optionally substituted with one or more R_{d1-1}, the C₃₋₁₀cycloalkyl is C₃₋₆ monocycloalkyl or C₅₋₁₀ spirocycloalkyl.

In some preferred embodiments of the present invention, in R_{D}, when R_{d1} is 3- to 10-membered heterocycloalkyl optionally substituted with one or more R_{d1-1}, the 3- to 10-membered heterocycloalkyl is 3-to 8-membered heteromonocycloalkyl or 5- to 10-membered heterospirocycloalkyl.

In some preferred embodiments of the present invention, Formula I has a structure of Formula I-d1:

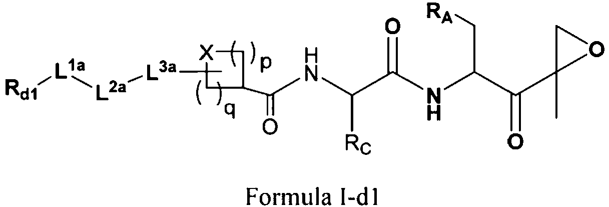

In Formula I-d1,
R_{A}, R_{C}, L^{3a}, L^{2a}, L^{1a} and R_{d1} have the same definitions as in Formula I; p and q are independently 0, 1, 2 or 3, and p and q are not both 0; and X is CH₂, NH, O or S. It can be understood by those skilled in the art that in Formula I-d1, when the substituent is attached to X, X is substituted and converted into N or CH.

In some preferred embodiments of the present invention, in Formula I-d1, X is CH₂.

In the present invention, when the carbon atom on the ring is chiral, the chirality of the carbon atom can be in R configuration or S configuration. When the substituents on the ring are suprafacial or antarafacial, they can be either suprafacial or antarafacial. For example, when L^{4a} is a meta-substituted cyclobutylene, the positions of the substituents in the present invention can be such that the substituents are located on two sides of cyclobutane respectively and indicated by 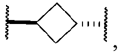 or the substituents are located on the same sides of cyclobutane and indicated by 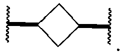 When 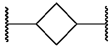 is used, it indicates that the above two substitutions are both possible.

In some preferred embodiments of the present invention, Formula I-d1 has a structure of Formula I-d1a:

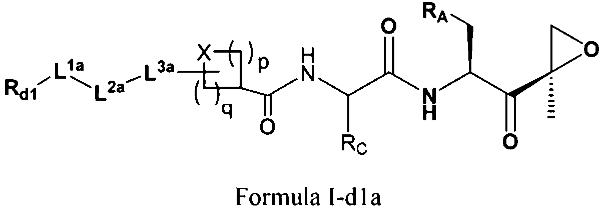

In Formula I-d1a,
R_{A}, R_{C}, L^{3a}, L^{2a}, L^{1a}, R_{d1}, p, q and X have the same definitions as in Formula I-d1.

In some preferred embodiments of the present invention, Formula I has a structure of Formula I-d1b:

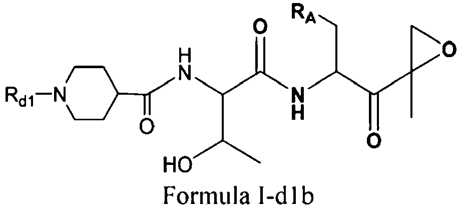

In Formula I-d1b, R_{A} and R_{d1} have the same definitions as in Formula I-d1.

In some preferred embodiments of the present invention, Formula I has a structure of Formula I-d1c:

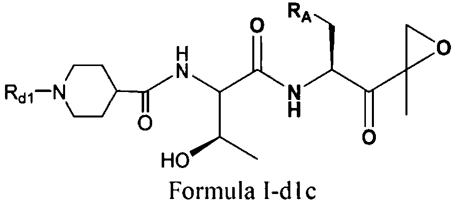

In Formula I-d1c, R_{A} and R_{d1} have the same definitions as in Formula I-d1.

In some preferred embodiments of the present invention, in R_{D}, L^{4a} is C₅₋₈ spirocycloalkylene or 5- to 8-membered heterospirocycloalkylene.

In some preferred embodiments of the present invention, in R_{D}, when L^{4a} is C₅₋₈ spirocycloalkylene, the C₅₋₈ spirocycloalkylene is a spiro-ring formed of two saturated monocyclic rings, where the two monocyclic rings may each independently contain 3, 4 or 5 carbon atoms. Preferably, the C₅₋₈ spirocycloalkylene in the present invention is spiro-pentane, spiro-hexane, spiro-heptane or spiro-octane.

In some preferred embodiments of the present invention, in R_{D}, when L^{4a} is 5- to 8-membered heterospirocycloalkylene, any one or more of the carbon atoms on the 5- to 8-membered heterospirocycloalkylene ring is/are replaced heteroatom(s).

In some preferred embodiments of the present invention, L^{4a} is C₅₋₈ spirocycloalkylene or 5- to 8-membered heterospirocycloalkylene and has a structure below: 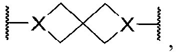 which each X is independently CH or N.

In some preferred embodiments of the present invention, when L^{4a} is C₅₋₈ spirocycloalkylene or 5-to 8-membered heterospirocycloalkylene, it has a structure of

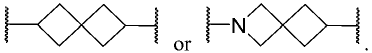

In some preferred embodiments of the present invention, Formula I has a structure of Formula I-d2:

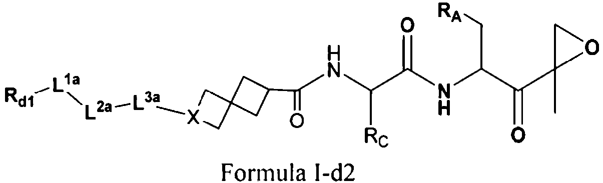

In Formula I-d2,
R_{A}, R_{C}, L^{3a}, L^{2a}, L^{1a} and R_{d1} have the same definitions as in Formula I; and X is CH or N.

In some preferred embodiments of the present invention, Formula I-d2 has a structure of Formula I-d2a:

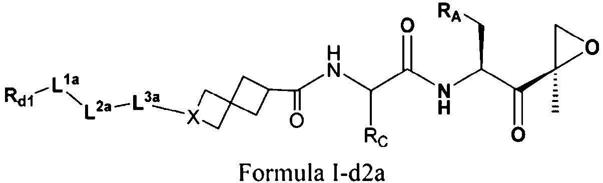

In Formula I-d2a,
R_{A}, R_{C}, L^{3a}, L^{2a}, L^{1a}, R_{d1} and X have the same definitions as in Formula I-d2.

In some preferred embodiments of the present invention, in R_{D}, L^{4a} is C₁₋₆ alkylene or C₂₋₆ alkenylene, where the C₁₋₆ alkylene or C₂₋₆ alkenylene are optionally substituted with one or more L^{4a-1}; and L^{4a-1} is hydroxyl, halogen, oxo (=O), C₁₋₆ alkylsulfonamido, or C₁₋₆ alkoxy.

In some preferred embodiments of the present invention, L^{4a} is C₁₋₆ alkylene, and preferably methylene, -CH₂CH₂-, or

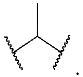

In some preferred embodiments of the present invention, L^{4a} is C₂₋₆ alkenylene, and preferably 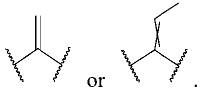 When L^{4a} is C₂₋₆ alkenylene in the present invention, The compound may be a compound having a cis-configuration, a compound having a trans-configuration, or a mixture of cis-configuration and trans-configuration. For example, when the substituents on both sides of 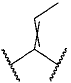 are different, the structure represents a cis-configuration, a trans-configuration, or a mixture of cis-configuration and trans-configuration.

In some preferred embodiments of the present invention, L^{4a} is C₁₋₆ alkylene or C₂₋₆ alkenylene, where the C₁₋₆ alkylene and C₂₋₆ alkenylene are optionally substituted with one or more L^{4a-1}; and L^{4a-1} is C₁₋₆ alkylsulfonamido or C₁₋₆ alkoxy.

In some preferred embodiments of the present invention, L^{4a} is optionally C₁₋₆ alkylene substituted with one or more L^{4a-1}, where the L^{4a-1} is methylsulfonamido or methoxy.

In some preferred embodiments of the present invention, Formula I has a structure of Formula I-d3:

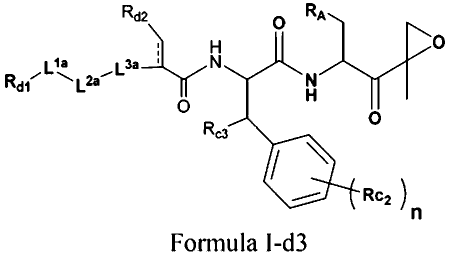

In Formula I-d3,
R_{A}, R_{C2}, L^{3a}, L^{2a}, L^{1a}, R_{d1} and n have the same definitions as in Formula I;
R_{d2} is hydrogen, hydroxyl, halogen, oxo (=O), C₁₋₆ alkylsulfonamido, or C₁₋₆ alkoxy;
Rc₃ is hydrogen, hydroxyl, C₁₋₆ alkoxy, or halogen; and
"-̅ -̅ -̅" represents a single bond or a double bond. When a double bond is indicated, it has a cis-configuration, a trans-configuration or a mixture of cis-configuration and trans-configuration.

In some preferred embodiments of the present invention, Formula I-d3 has a structure of Formula I-d3a:

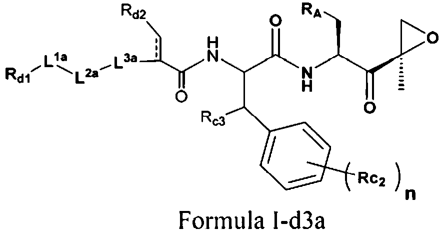

In Formula I-d3a, R_{A}, Rc₂, L^{3a}, L^{2a}, L^{1a}, R_{d1}, R_{d2}, Rc₃ and n have the same definitions as in Formula I-d3;
"-̅ -̅ -̅" represents a single bond or a double bond. When a double bond is indicated, it has a cis-configuration, a trans-configuration or a mixture of cis-configuration and trans-configuration.

In some preferred embodiments of the present invention, In Formula I, Formula I-a1, Formula I-c1, Formula I-d1, Formula I-d2, and Formula I-d3, R_{d1} is 3- to 8-membered hetero monocycloalkyl optionally substituted with one or more R_{d1-1}, the 3- to 8-membered hetero monocycloalkyl is 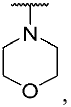 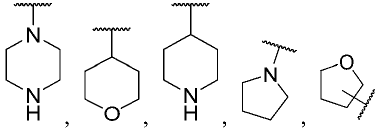 (for example 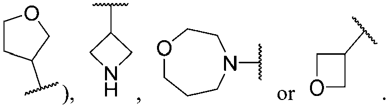 R_{d1-1} is hydroxyl, halogen, C₁₋₆ alkyl, C₁₋₆ alkoxy, C₁₋₆ haloalkyl, C₁₋₆ haloalkoxy, oxo (=O), acetyl, C₁₋₆ alkylsulfonyl, C₃₋₆ cycloalkyl, 3- to 6-membered heterocycloalkyl, 3- to 6-membered heterocycloalkylmethyl or C₃₋₆cycloalkyl; Preferably, R_{d1-1} is oxo (=O), cyclopropyl, acetyl, trifluoromethoxy, methansulfonyl, halogen, or morpholinylmethyl.

In some preferred embodiments of the present invention, in Formula I, Formula I-a1, Formula I-c1, Formula I-d1, Formula I-d2, and Formula I-d3, R_{d1} is

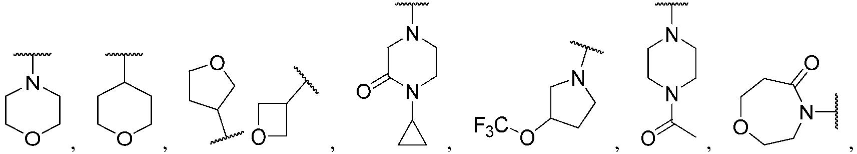

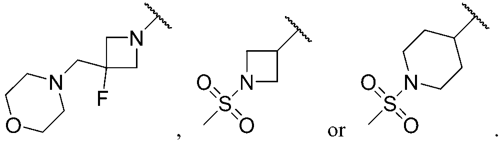

In some preferred embodiments of the present invention, in Formulas I, Formula I-a1, Formula I-c1, Formula I-d1, Formula I-d2, and Formula I-d3, R_{d1} is

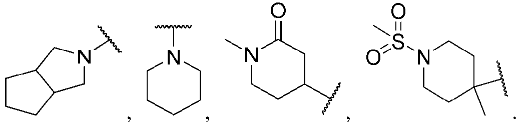

In some preferred embodiments of the present invention, in Formula I, Formula I-a1, Formula I-c1, Formula I-d1, Formula I-d2, and Formula I-d3, R_{d1} is C₅₋₁₀ spirocycloalkyl or 5- to 10-membered hetero spirocycloalkyl; and preferably, the C₅₋₁₀ spirocycloalkyl and 5- to 10-membered hetero spirocycloalkyl is

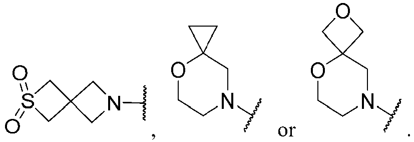

In some preferred embodiments of the present invention, in Formula I, Formula I-a1, Formula I-c1, Formula I-d1, Formula I-d2, and Formula I-d3, R_{d1} is 5- to 8-membered heteroaryl optionally substituted with one or more R_{d1-1}; and preferably, the R_{dl-1} is benzyl ( 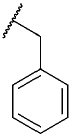 ) or morpholinylmethyl ( 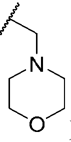 ).

In some preferred embodiments of the present invention, in Formula I, Formula I-a1, Formula I-c1, Formula I-d1, Formula I-d2, and Formula I-d3, R_{d1} is

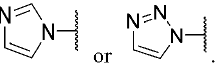

In some preferred embodiments of the present invention, in Formula I, Formula I-a1, Formula I-c1, Formula I-d1, Formula I-d2, and Formula I-d3, R_{d1} is

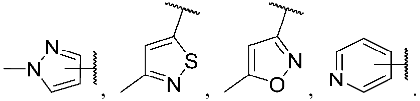

In some preferred embodiments of the present invention, in Formula I, Formula I-a1, Formula I-c1, Formula I-d1, Formula I-d2, and Formula I-d3, R_{d1} is

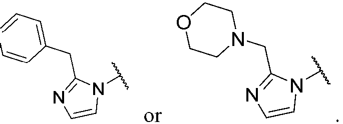

In some preferred embodiments of the present invention, in Formula I, Formula I-a1, Formula I-c1, Formula I-d1, Formula I-d2, and Formula I-d3, R_{d1} is C₁₋₆ alkylsulfonyl or C₃₋₁₀cycloalkyl.

In some preferred embodiments of the present invention, in Formula I, Formula I-a1, Formula I-c1, Formula I-d1, Formula I-d2, and Formula I-d3, R_{d1} is

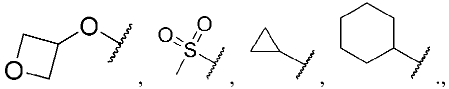

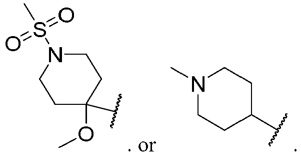

In some preferred embodiments of the present invention, in Formula I, Formula I-a1, Formula I-c1, Formula I-d1, Formula I-d2, and Formula I-d3, L^{3a}, L^{2a}, and L^{1a} are each independently a bond, -O-, - NH-, -N(CH₃)-, carbonyl (-C=O-), C₁₋₆ alkylene, sulfonyl (-SO₂-), -C(=O)NH-, or -NHC(=O)NH-.

In some preferred embodiments of the present invention, in Formula I, Formula I-a1, Formula I-c1, Formula I-d1, Formula I-d2, and Formula I-d3, L^{3a}, L^{2a}, and L^{1a} are each independently a bond, -O-, - NH-, -N(CH₃)-, carbonyl (-C=O-), methylene (-CH₂-), ethylene (-CH₂CH₂-), sulfonyl (-SO₂-), -C(=O)NH-, or -NHC(=O)NH-.

In some preferred embodiments of the present invention, in Formula I- d1, L^{3a}, L^{2a}, and L^{1a} are each independently a bond, -NH-, carbonyl (-C=O-), C₁₋₆ alkylene, or sulfonyl (-SO₂-).

In some preferred embodiments of the present invention, in Formula I-d1, L^{3a}, L^{2a}, and L^{1a} are each independently a bond, -NH-, carbonyl (-C=O-), methylene, or sulfonyl (-SO₂-).

In some preferred embodiments of the present invention, the structural unit 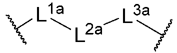 is a bond, NH-,

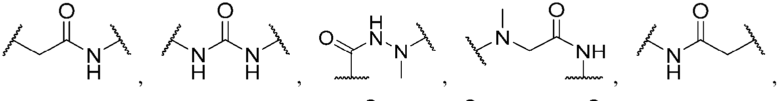

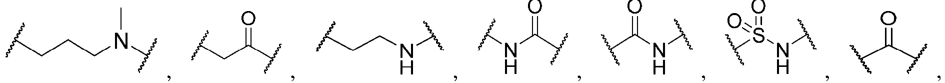

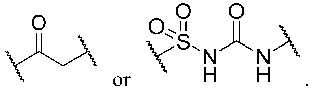

In some preferred embodiments of the present invention, the structural unit 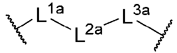 is methylene or

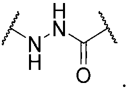

In some preferred embodiments of the present invention, Formula I or Formula I-d1 has a structure of Formula I- d4:

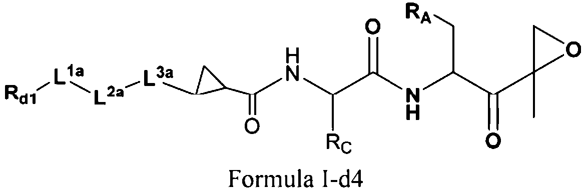

In some preferred embodiments of the present invention, the epoxy compound of Formula I is selected from any compound in Table A.

**Table A**

| | |
|---|---|
| 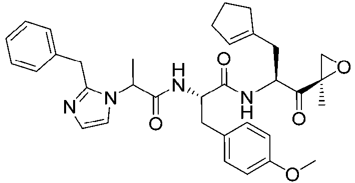 | 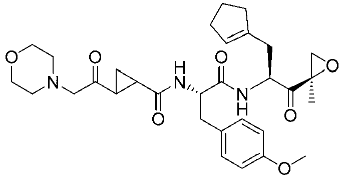 |
| A001 | A002 |
| 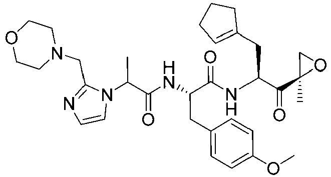 | 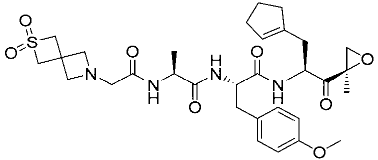 |
| A003 | A004 |
| 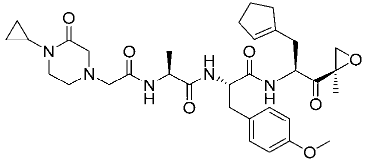 | 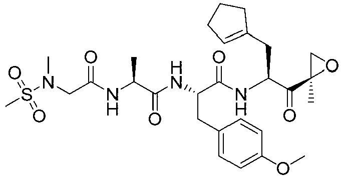 |
| A006 | A008 |
| 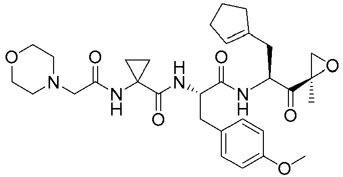 | 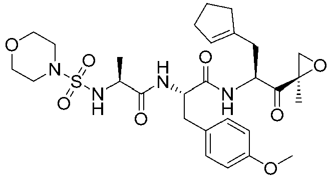 |
| A009 | A010 |
| 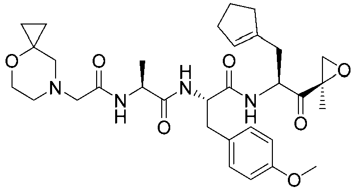 | 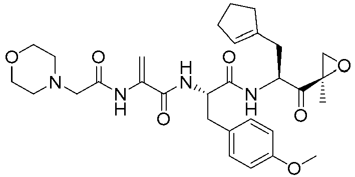 |
| A012 | A013 |
| 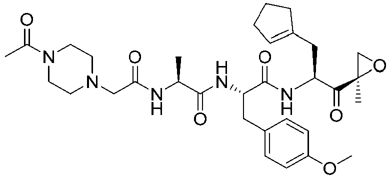 | 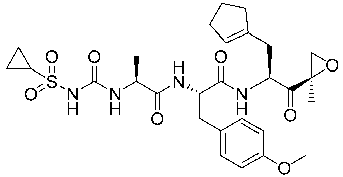 |
| A015 | A017 |
| 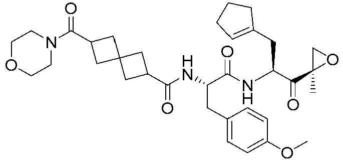 | 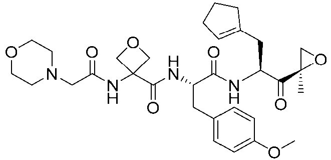 |
| A019 | A020 |
| 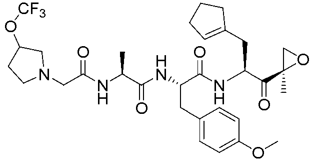 | 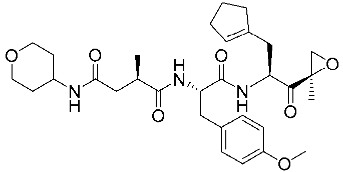 |
| A022 | A023 |
| 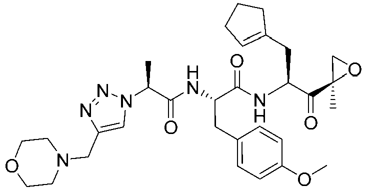 | 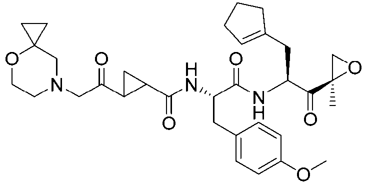 |
| A028 | A029 |
| 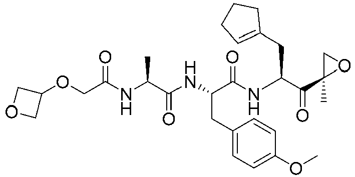 | 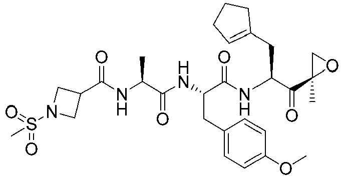 |
| A030 | A031 |
| 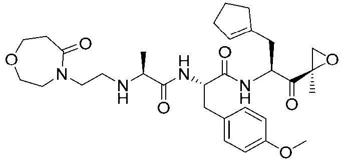 | 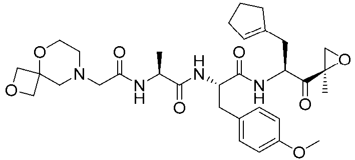 |
| A032 | A034 |
| 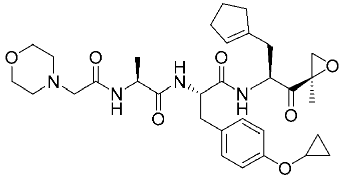 | 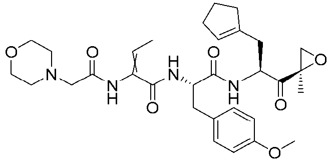 |
| A036 | A038 |
| 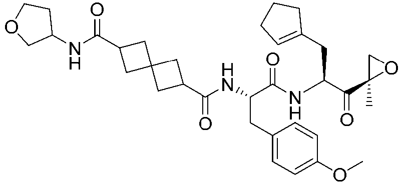 | 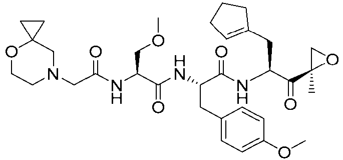 |
| A040 | A042 |
| 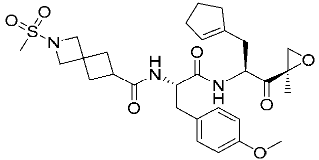 | 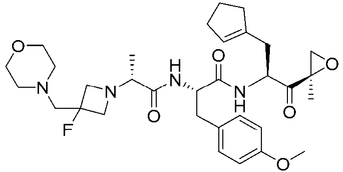 |
| A043 | A053 |
| 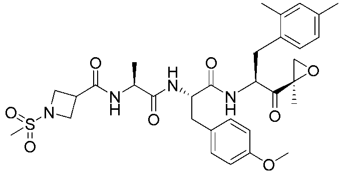 | 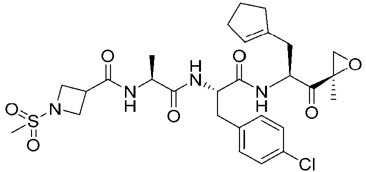 |
| A055 | A057 |
| 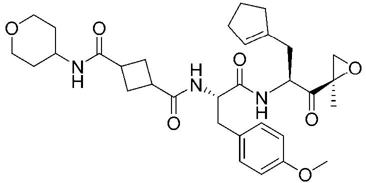 | 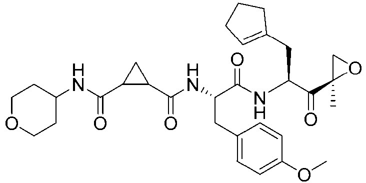 |
| A059 | A061 |
| 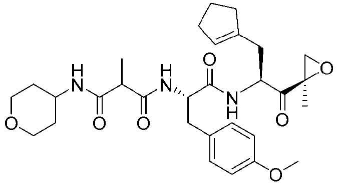 | 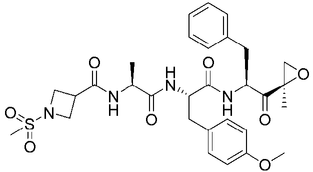 |
| A062 | A064 |
| 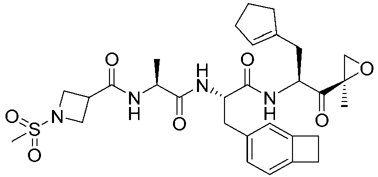 | 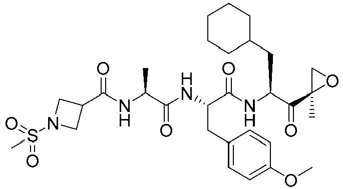 |
| A066 | A068 |
| 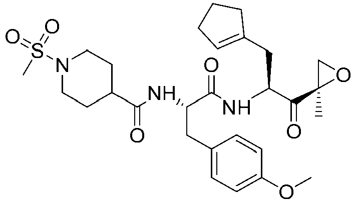 | 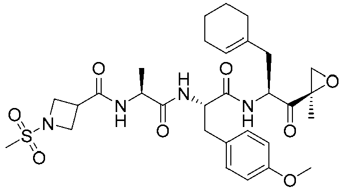 |
| A070 | A073 |
| 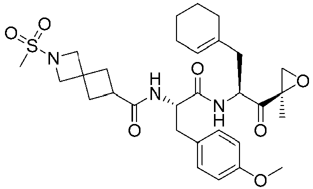 | 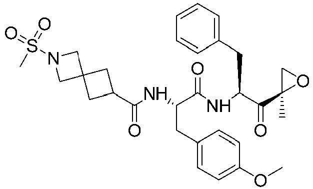 |
| A075 | A077 |
| 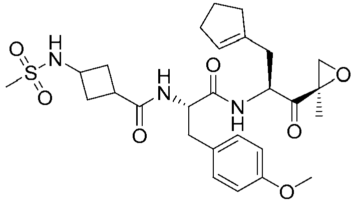 | 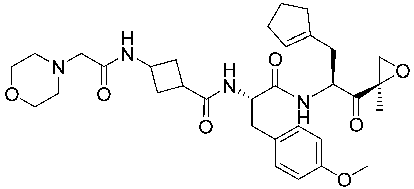 |
| A079 | A080 |
| 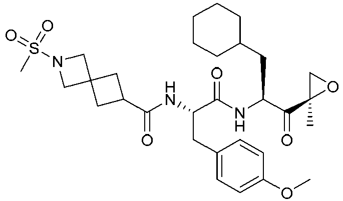 | 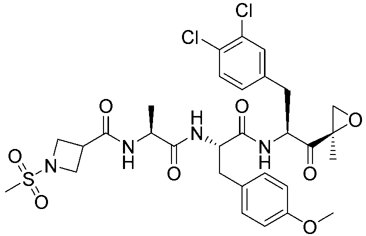 |
| A095 | A098 |
| 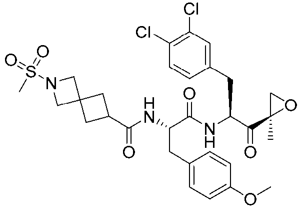 | 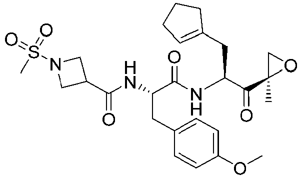 |
| A100 | A102 |
| 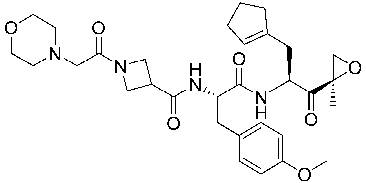 | 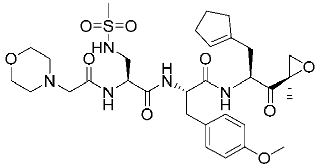 |
| A103 | A014 |
| 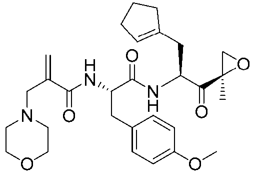 | 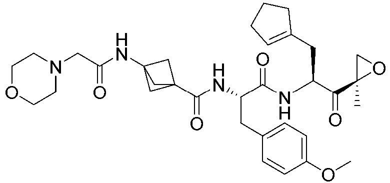 |
| A025 | A105 |
| 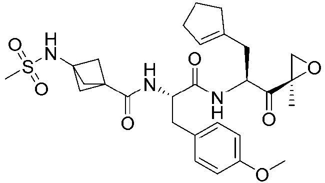 | 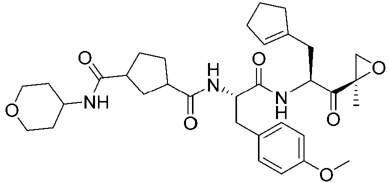 |
| A107 | A109 |
| 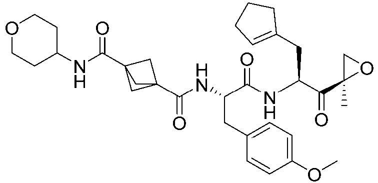 | 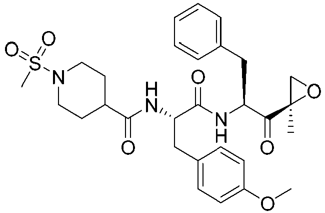 |
| A110 | A115 |
| 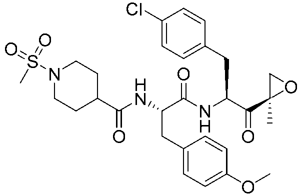 | 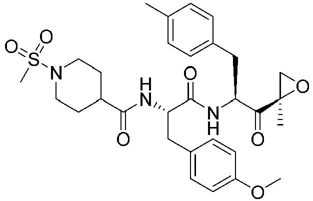 |
| A117 | A119 |
| 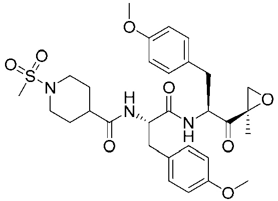 | 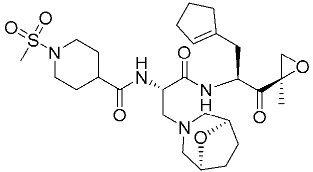 |
| A120 | A121 |
| 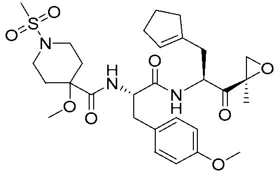 | 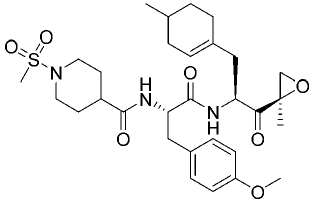 |
| A122 | A125 |
| 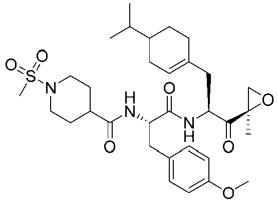 | 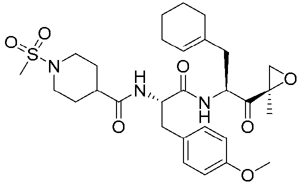 |
| A133 | A135 |
| 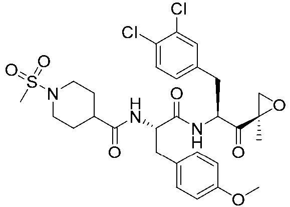 | 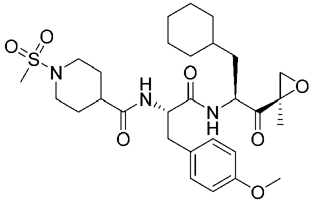 |
| A137 | A141 |
| 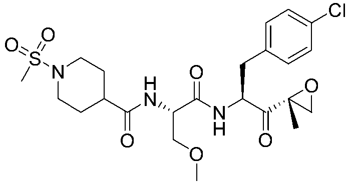 | 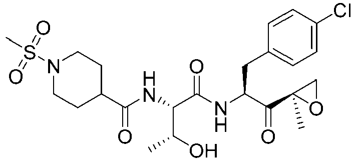 |
| A142 | A143 |
| 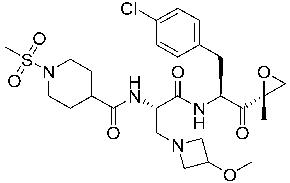 | 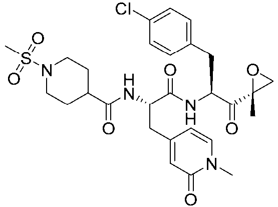 |
| A145 | A147 |
| 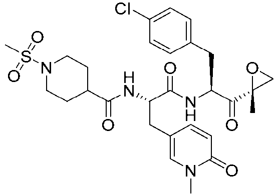 | 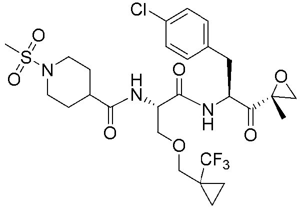 |
| A148 | A150 |
| 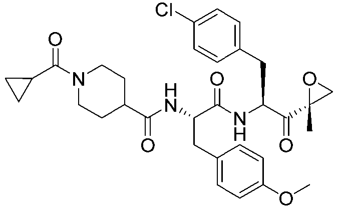 | 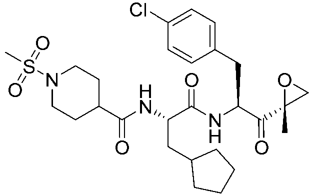 |
| A151 | A155 |
| 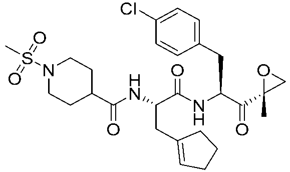 | 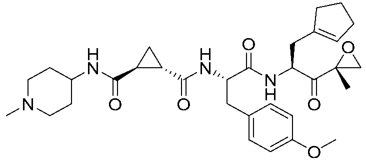 |
| A157 | A158 |
| 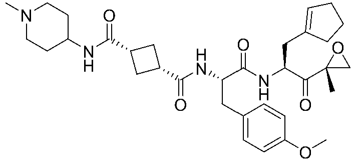 | 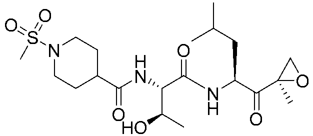 |
| A160 | A161 |
| 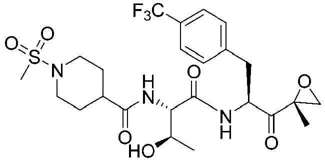 | 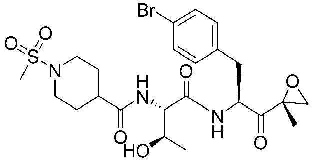 |
| A162 | A163 |
| 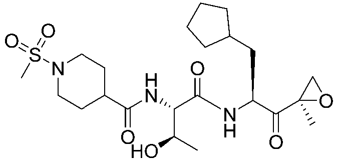 | 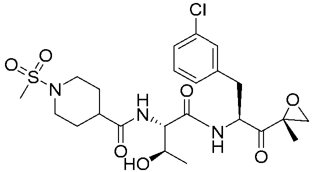 |
| A164 | A165 |
| 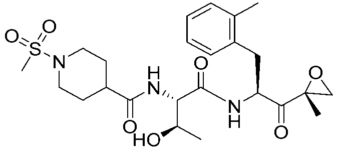 | 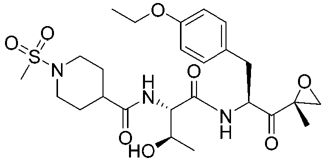 |
| A166 | A168 |
| 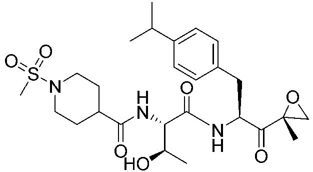 | 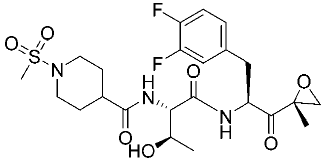 |
| A169 | A170 |
| 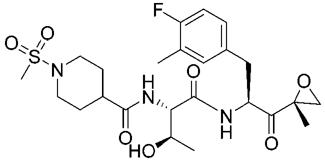 | 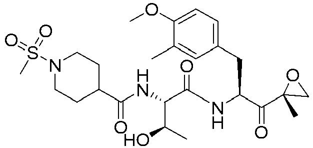 |
| A171 | A172 |
| 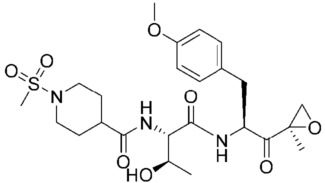 | 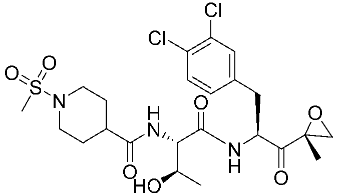 |
| A173 | A174P1 |
| 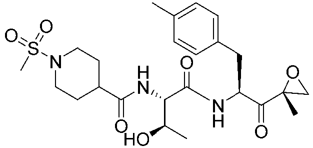 | 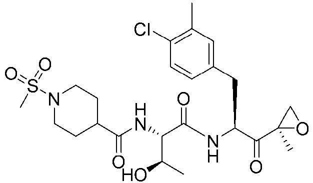 |
| A175 | A176 |
| 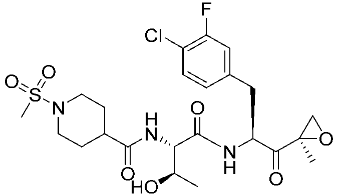 | 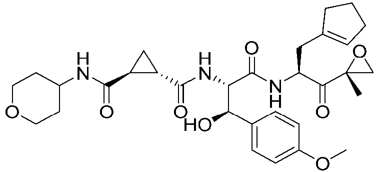 |
| A177 | A178 |
| 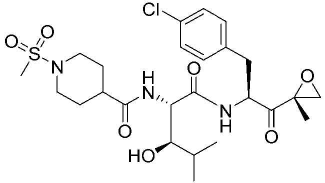 | 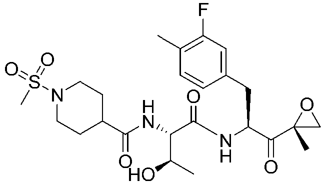 |
| A179 | A182 |
| 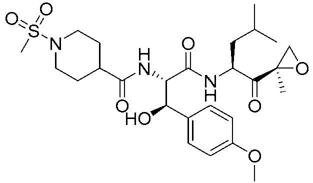 | 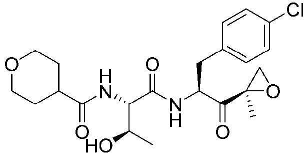 |
| A183 | A184 |
| 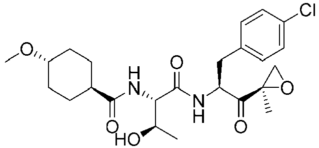 | 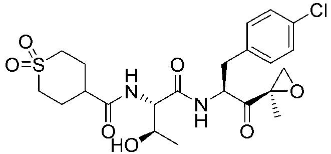 |
| A185 | A187 |
| 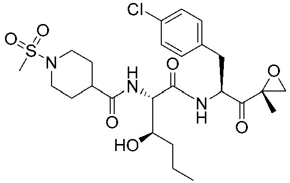 | 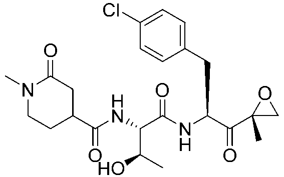 |
| A188 | A189 |
| 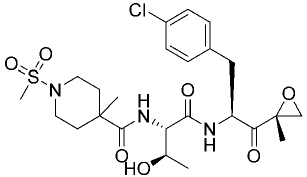 | 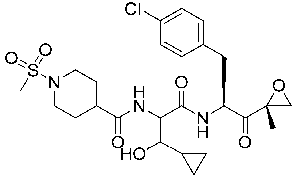 |
| A191 | A192 |
| 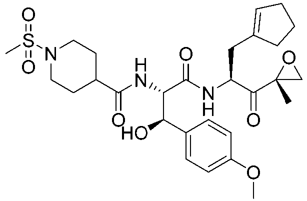 | 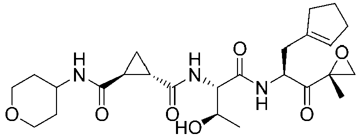 |
| A194 | A195 |
| 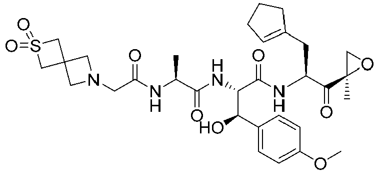 | 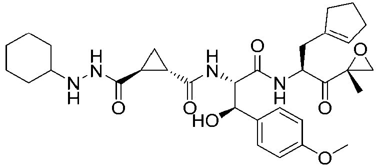 |
| A196 | A197 |
| 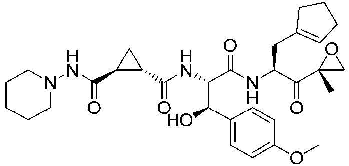 | 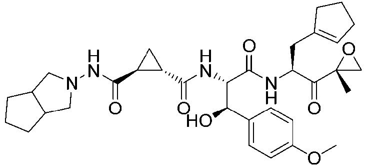 |
| A198 | A199 |
| 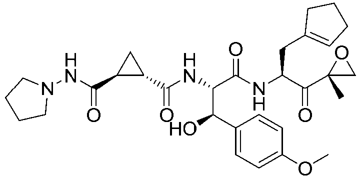 | 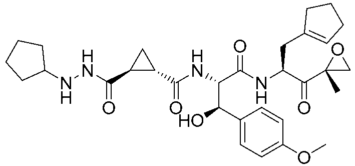 |
| A200 | A201 |
| 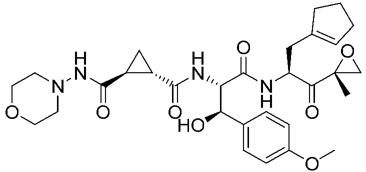 | 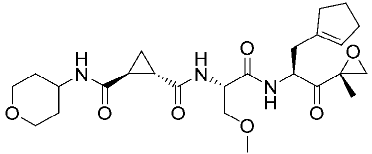 |
| A202 | A203 |
| 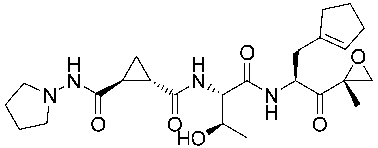 | 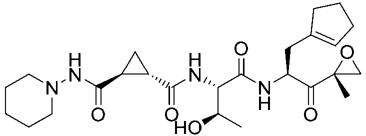 |
| A207 | A208 |
| 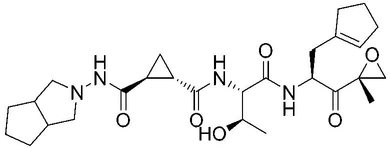 | 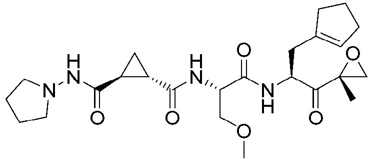 |
| A209 | A210 |
| 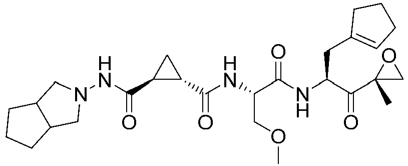 | 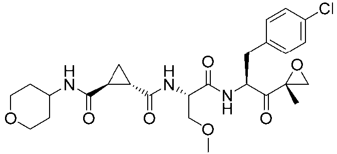 |
| A211 | A212 |
| 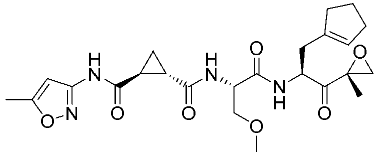 | 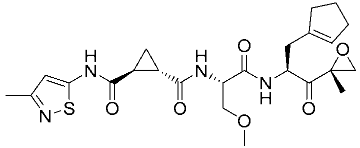 |
| A213 | A214 |
| 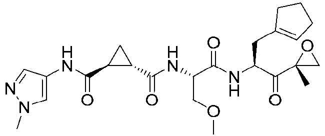 | 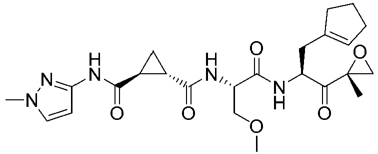 |
| A215 | A216 |
| 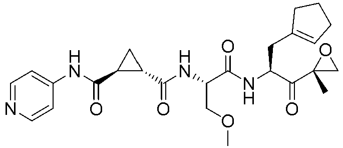 | 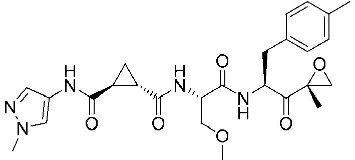 |
| A217 | A218 |
| 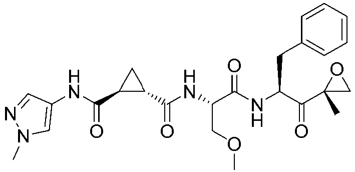 | 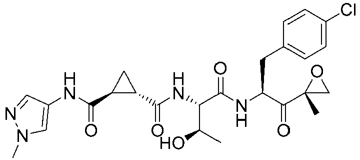 |
| A219 | A220 |
| 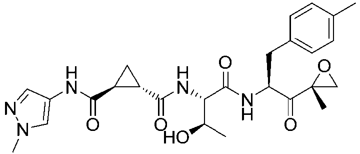 | 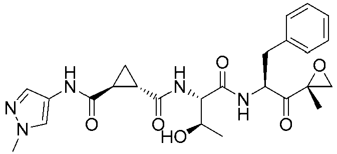 |
| A221 | A222 |

The present invention also includes a pharmaceutically acceptable salt of the epoxy compound of Formula I as described above. The pharmaceutically acceptable salt refer to a derivative of the compound of Formula I in which the parent compound is modified by converting a base moiety present thereon into a salt form, or a derivative of the compound of Formula I in which the parent compound is modified by converting an acid moiety present thereon into a salt form.

Specifically, examples of the pharmaceutically acceptable salt include, but are not limited to, salts of basic groups (such as amines) with inorganic or organic acids, or salts of acidic groups (such as carboxylic acids) with inorganic or organic bases. The pharmaceutically acceptable salt of the present invention can be synthesized with the parent compound of Formula I by reacting the compound in free base form with an appropriate acid (for example, 1-4 equivalents) in a solvent system.

The present invention further provides a pharmaceutical composition, which contains the epoxy compound of Formula I, or a pharmaceutically acceptable salt or a stereoisomer thereof, and a pharmaceutically acceptable adjuvant.

The present invention further provides use of the epoxy compound of Formula I or a pharmaceutically acceptable salt or a stereoisomer thereof, or the pharmaceutical composition as described above in the preparation of LMP7 and/or LMP2 inhibitors. In the use, the LMP7 and/or LMP2 inhibitors can be used in vivo or in vitro in mammals, mainly for experimental purposes. For example, they are used as a standard sample or a control sample for comparison purpose, or prepared into a kit according to the conventional method in the art to provide rapid detection for the LMP7 and/or LMP2 inhibiting effect.

The present invention further provides use of the epoxy compound of Formula I or a pharmaceutically acceptable salt or a stereoisomer thereof, or the pharmaceutical composition as described above in the preparation of drugs for treating autoimmune diseases. The autoimmune diseases include: psoriasis, glomerular nephritis, systemic lupus erythematosus (SLE), immune thrombocytopenic purpura (ITP), autoimmune thrombocytopenia, and diabetes mellitus, etc.

The present invention further provides use of the epoxy compound of Formula I or a pharmaceutically acceptable salt or a stereoisomer thereof, or the pharmaceutical composition as described above in the preparation of drugs for inhibiting immunoproteasome in cells. The drugs are applicable to the treatment of autoimmune diseases. The autoimmune diseases include: psoriasis, glomerular nephritis, systemic lupus erythematosus (SLE), immune thrombocytopenic purpura (ITP), autoimmune thrombocytopenia, and diabetes mellitus, etc.

The present invention further provides use of the epoxy compound of Formula I or a pharmaceutically acceptable salt or a stereoisomer thereof, or the pharmaceutical composition as described above in the preparation of drugs for treating and/or immunoproteasome (for example, β5i and β1i) related diseases. The immunoproteasome related diseases are preferably autoimmune diseases, for example, psoriasis, glomerular nephritis, systemic lupus erythematosus (SLE), immune thrombocytopenic purpura (ITP), autoimmune thrombocytopenia, and diabetes mellitus.

The compound of Formula I of the present invention can be prepared through the following Method 1 by reacting Intermediate M1 with Intermediate M2 in the presence of a condensing agent, such as EDCI, HATU, and CDI.

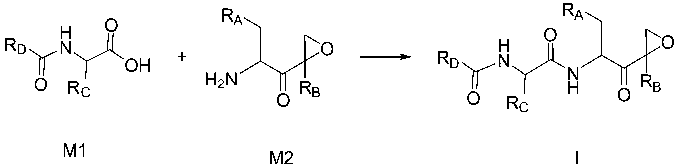

Intermediate M2 can be synthesized following a method in the prior art, for example, the method disclosed in WO2014152134.

Intermediate M1 can be prepared following a method in the prior art or by Method 2 below by hydrolyzing Intermediate M1-3, where Rₘ is alkyl, for example, methyl, ethyl, and tert-butyl, etc. The hydrolysis can be carried out under alkaline conditions, such as hydrolysis in the presence of an alkali metal hydroxide. The alkali metal hydroxide includes lithium hydroxide, sodium hydroxide, and potassium hydroxide, etc.

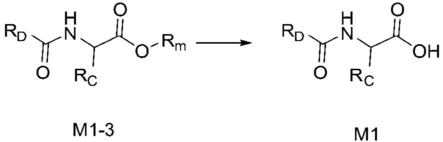

Intermediate M1-3 can be prepared following a method in the prior art or by Method 3 below by condensing Intermediate M1-1 and Intermediate M1-2 in the presence of a condensing agent such as EDCI, HATU, and CDI.

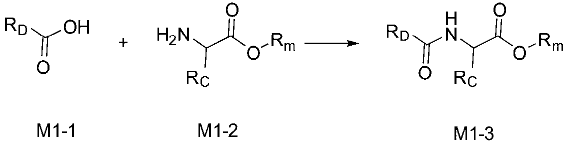

### Definitions of terms

Unless otherwise specified, the terms used in the present invention have conventional meanings in the art. Some terms are defined below.

The term "optional" or "optionally" used in the present invention refers to both substituted and unsubstituted. For example, "C₁₋₆ alkylene optionally substituted with one or more R_{C1-1}" means "C₁₋₆ alkylene" or "C₁₋₆ alkylene substituted with one or more R_{C1-1}".

Unless otherwise indicated, the absolute configuration of a stereocenter is indicated by a wedge-shaped solid-line bond ( 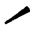 ) or a wedge-shaped dashed-line bond ( 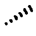 ). The relative configuration of a stereocenter is indicated by a straight solid-line bond ( 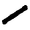 ) and a straight dashed-line bond ( 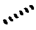 ).

When the number of a linking group is 0, for example, -(CH₂)₀-, it means that the linking group is a single bond.

The term "alkyl" used in the present invention refers to a linear or branched saturated hydrocarbon group containing 1-18 carbon atoms, such as 1-12 carbon atoms, 1-6 carbon atoms, or 1-4 carbon atoms. For example, "C₁₋₆ alkyl" is within the scope of "alkyl", and represents an alkyl group with 1-6 carbon atoms. Examples of alkyl include, but are not limited to, methyl ("Me"), ethyl ("Et"), n-propyl ("n-Pr"), isopropyl ("i-Pr"), n-butyl ("n-Bu"), isobutyl ("i-Bu"), sec-butyl ("sBu"), and t-butyl ("t-Bu").

The term "alkoxy" refers to -O-alkyl, in which the alkyl is defined as above.

"Alkenyl" refers to a substituted or unsubstituted unsaturated aliphatic group with at least one double bond and having a chain length similar to that of alkyl.

The term "cycloalkyl" as used in the present invention refers to a saturated cyclic hydrocarbon group containing 3-10 ring carbon atoms, for example 3-8 ring carbon atoms or 3-6 ring carbon atoms, which may have one or more rings, for example 1 or 2 rings. For example, "C₃₋₆ cycloalkyl" refers to a cycloalkyl group having 3-6 ring carbon atoms. Examples of cycloalkyl include, but are not limited to, cyclopropyl, cyclobutyl, cyclopentyl, cyclohexyl, and the like.

The term "heterocycloalkyl" used in the present invention refers to a group in which the ring carbon atom of cycloalkyl is replaced by one or more heteroatoms. "3- to 8-membered heterocycloalkyl" refers to a cycloalkyl group with 3-8 ring carbon atoms in which any one or more ring carbon atoms is/are replaced by heteroatom(s) or heteroatom group(s). In some embodiments, the heteroatom and heteroatom group are one or more selected from O, N, S, and -SO₂-. Examples of heterocycloalkyl include, but are not limited to, heterocyclopropyl, heterocyclobutyl, heterocyclopentyl, heterocyclohexyl, heterocycloheptyl, heterocyclooctyl, and the like.

The term "cycloalkoxy" used in the present invention refers to the group -O- cycloalkyl, where the cycloalkyl is as defined above.

The term "spirocycloalkyl" used in the present invention refers to a substituent consisting of two or three rings sharing a common atom therebetween. Each monocyclic ring has 3-5 carbon atoms, and each monocyclic ring is a saturated ring. C₅₋₁₀ spirocycloalkyl refers to a spirocycloalkyl having 5-10 carbon atoms, such as spiro[4.5]decane, and spiro[3.3]heptane.

The term "heterospirocycloalkyl" used in the present invention refers to a group in which one or more ring carbon atoms in a spirocycloalkyl group is/are replaced by heteroatom(s). 5- to 10-membered heterospirocycloalkyl means that any one or more ring carbon atoms in a spirocycloalkyl group having 5-10 carbon atoms is/are replaced by heteroatom(s) or heteroatom group(s). In some embodiments, the heteroatom and heteroatom group are one or more selected from O, N, S, and -SO₂-. Examples of heterospirocycloalkyl include, but are not limited to, 2-azaspiro[4.5]decane, 2,6- diazspiro[3.3]heptane and the like.

The terms "alkylene", "cycloalkylene", "alkenylene", "heterocycloalkylene", " spirocycloalkylene", and "heterospirocycloalkylene" used in the present invention mean that one hydrogen atom in alkyl, cycloalkyl, alkenyl, heterocycloalkyl, spirocycloalkyl, and heterospirocycloalkyl are further substituted.

The term "aryl" used in the present invention refers to a 6- to 14-membered and preferably 6- to 10-membered full carbon monocyclic or fused polycyclic group having a conjugated π electron system, and further preferably phenyl.

The term "heteroaryl" used in the present invention refers to a heteroaromatic system containing 1 to 4 heteroatoms and 5 to 14 ring atoms, where the heteroatom is one or more selected from O, S and N. Examples of heteroaryl includes, but are not limited to, imidazolyl, furyl, thienyl, thiazolyl, pyrazolyl, pyrrolyl, triazolyl, tetrazolyl, pyridinyl, pyrimidinyl and the like.

The term "halo" used in the present invention means substitution with one or more halogens, where "halogen" means fluoro, chloro, bromo and iodo.

The term "multiple" refers to 2, 3, 4, or 5.

When any variable (such as group R_{A1}) appears multiple times in the definition of a compound, their definitions are independent of each other and have no influence on each other. For example, C₆₋₁₄ aryl substituted with 3 R_{A1} means that the C₆₋₁₄ aryl is substituted with 3 R_{A1}, and the definitions of the 3 R_{A1} are independent of each other and have no influence on each other.

The "pharmaceutically acceptable salt" includes, but is not limited to, acid addition salts of the compound of Formula (I) with inorganic acids, such as hydrochloride, hydrobromide, phosphate, and sulfate, etc; and acid addition salts of the compound of Formula (I) with organic acids, such as acetate, malate, maleate, fumarate, tartrate, succinate, citrate, lactate, methanesulfonate and the like.

On the basis of not conflicting with the common knowledge in the art, the above-mentioned preferred conditions can be arbitrarily combined to obtain the preferred embodiments of the present invention.

The reagents and raw materials used in the present invention are all commercially available.

The present invention has the following positive effects. The present application provides an epoxy compound and use thereof. The epoxy compound has novel structure, and high inhibitory activity on β5i and β1i, but low inhibitory level on β5c and β1c, so the compound of the present invention has high selectivity.

### DETAILED DESCRIPTION

The following synthesis examples are used to further illustrate the concept of the present invention, and do not constitute restriction on the present invention. The compound of the present invention can also be prepared through a method in the prior art. The compound of the present invention can be separated into optically pure or racemate forms. The optically pure forms can be prepared by resolution of racemates, or by using chiral synthon or chiral reagents. The chemical structure containing one or more stereocenters in the present invention includes all possible stereoisomeric forms (e.g., diastereomers, and enantiomers) of compounds and their mixtures.

### Synthesis of Intermediates

### Synthesis of Compound A001-1

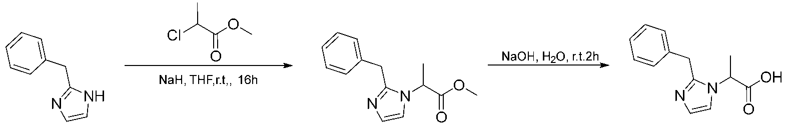

Under a nitrogen atmosphere and at room temperature, sodium hydride (91.01 mg, 3.792 mmol, 2 eq) was added to a solution of 2-benzyl-1H-imidazole (300 mg, 1.896 mmol, 1 eq) in tetrahydrofuran (2 mL), and continuously stirred at this temperature for 30 min. Methyl 2-chloropropionate (348.58 mg, 2.844 mmol, 1.5 eq) was added. After that, the system was further stirred at room temperature for 16 hrs. The reaction mixture was quenched with water at room temperature. The reaction mixture was extracted with ethyl acetate (3 x 30 mL). The organic phases were combined, back washed with saturated brine (1 x 20 mL), and dried over anhydrous sodium sulfate. After the resulting mixture was filtered, the filtrate was concentrated under reduced pressure. The residue was purified by column chromatography on silica gel to obtain methyl 2-(2-benzylimidazole-1-yl)propionate (400 mg, 86.35%), as a yellow oil.

LCMS (ESI, *m*/*z*): 245.45, [M+H]⁺.

Under a nitrogen atmosphere and at room temperature, sodium hydroxide (196.47 mg, 4.911 mmol, 3 eq) was added to a solution of methyl 2-(2-benzylimidazole-1-yl)propionate (400 mg, 1.637 mmol, 1 eq) in water (2 mL), and reacted with stirring for 2 hrs. The reaction mixture was quenched with water at room temperature. The reaction mixture was adjusted to pH 5-6 with hydrochloric acid. The reaction mixture was extracted with ethyl acetate (3 x 20 mL). The organic phases were combined, back washed with saturated brine (1 x 30 mL), and dried over anhydrous sodium sulfate. After the resulting mixture was filtered, the filtrate was concentrated under reduced pressure. to obtain 2-(2-benzylimidazole-1-yl)propionic acid (140 mg, 37.13%).

LCMS (ESI, *m*/*z*): 231.40, [M+H]⁺.

### Synthesis of Compound A002-1

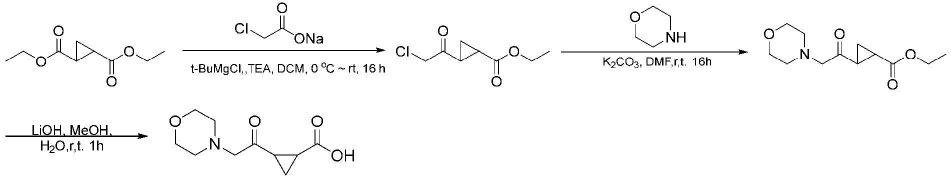

Under a nitrogen atmosphere and at room temperature, sodium chloroacetate (375.32 mg, 3.222 mmol, 1.2 eq) and triethyl amine (1.49 mL, 10.740 mmol, 4 eq) were added to a solution of diethyl cyclopropane-1,2-dicarboxylate (500 mg, 2.685 mmol, 1 eq) in dichloromethane (20 mL). The reaction solution was cooled to 0°C, and t-butylmagnesium chloride (6.5 mL, 10.74 mmol, 4 eq) was added dropwise. The reaction solution was heated to room temperature and reacted for 16 hrs. The reaction solution was quenched with a saturated ammonium chloride aqueous solution at room temperature, and extracted with ethyl acetate (3 x 40 mL). The organic phases were combined, back washed with a saturated sodium chloride solution (2 x 40 mL), and dried over anhydrous sodium sulfate. After the resulting mixture was filtered, the filtrate was concentrated under reduced pressure, to obtain ethyl 2-(2-chloroacetyl)cyclopropan-1-carboxylate (440 mg, crude product). The crude product was directly used in the next step without further purification.

LCMS: (ESI, *m*/*z*): 191.04, [M+H]⁺.

At room temperature, potassium carbonate (957.04 mg, 6.924 mmol, 3 eq) and morpholine (241.32 mg, 2.770 mmol, 1.2 eq) were added to a solution of ethyl 2-(2-chloroacetyl)cyclopropan-1-carboxylate (440 mg, 2.308 mmol, 1 eq) in *N,N-*dimethyl formamide (5 mL), and reacted at room temperature for 16 hrs. The reaction solution was quenched with water at room temperature, and concentrated under reduced pressure. The resulting crude product was purified by reverse-phase column chromatography, to obtain ethyl 2-(2-morpholinoacetyl)cyclopropan-1-carboxylate (224 mg, 40.22%), as a light yellow oil.

LCMS (ESI, *m*/*z*): 242.15, [M+H]⁺.

At room temperature, lithium hydroxide (66.70 mg, 2.784 mmol, 3 eq) was added to ethyl 2-(2-morpholinoacetyl)cyclopropan-1-carboxylate (224 mg, 0.928 mmol, 1 eq) in a mixed solvent of methanol (2 mL) and water (2 mL), and reacted at room temperature for 1 hr. The reaction solution was adjusted to pH 4-5 with 1mol/L dilute hydrochloric acid, and the mixture was concentrated under reduced pressure, to obtain 2-(2-morpholinoacetyl)cyclopropan-1-carboxylic acid (200 mg, crude product), as a white solid. The crude product was directly used in the next step without further purification.

LCMS: (ESI, *m*/*z*): 214.35, [M+H]⁺.

### Synthesis of Compound A003-1

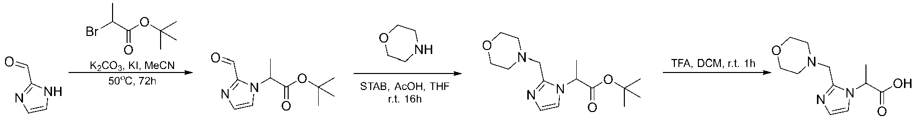

Under a nitrogen atmosphere and at room temperature, to a solution of 1H-imidazole-2-carboxaldehyde (1.0 g, 10.407 mmol, 1 eq), potassium iodide (1.73 g, 10.407 mmol, 1 eq) and potassium carbonate (2.88 g, 20.814 mmol, 2 eq) in acetonitrile (20.0 mL), t-butyl 2-bromopropionate (2.59 mL, 15.611 mmol, 1.5 eq) was added dropwise. After that, the system was heated to 50°C and further stirred for 72 hrs. The reaction solution was cooled to room temperature, diluted with water (50 mL), and extracted with ethyl acetate (3 x 50 mL). The organic phases were combined, back washed with saturated brine (1 x 30 mL), and dried over anhydrous sodium sulfate. After the resulting mixture was filtered, the filtrate was concentrated under reduced pressure, The residue was purified by column chromatography on silica gel to obtain t-butyl 2-(2-formyl-1H-imidazole-1-yl)propionate (2.0 g, 85.69%), as a light yellow liquid.

LCMS (ESI, *m*/*z*): 225.45 [M+H]⁺.

Under a nitrogen atmosphere and at room temperature, to a solution of t-butyl 2-(2-formyl-1H-imidazole-1-yl)propionate (500 mg, 2.230 mmol, 1 eq), and morpholine (485.61 mg, 5.575 mmol, 2.5 eq) in tetrahydrofuran (10.0 mL), sodium triacetoxyborohydride (567.04 mg, 2.676 mmol, 1.2 eq) and acetic acid (383.27 uL, 6.690 mmol, 3 eq) were added. After that, the system was further stirred at room temperature for 16 hrs. The reaction solution was quenched by adding a saturated sodium bicarbonate aqueous solution (50 mL) at room temperature, and extracted with dichloromethane (3 x 30 mL). The organic phases were combined, back washed with saturated brine (1 x 30 mL), and dried over anhydrous sodium sulfate. After the resulting mixture was filtered, and the filtrate was concentrated under reduced pressure. The obtained residue was purified by reverse-phase column chromatography to obtain t-butyl 2-(2-(morpholinemethyl)-1H-imidazole-1-yl)propionate (530 mg, 80.48%), as a colorless and transparent oil.

LCMS (ESI, *m*/*z*): 296.20 [M+H]⁺.

Under a nitrogen atmosphere and at room temperature, trifluoroacetic acid (1.0 mL) was added to a solution of t-butyl 2-(2-(morpholinemethyl)-1*H*-imidazole-1-yl)propionate (450 mg, 1.523 mmol, 1 eq) in dichloromethane (4.0 mL), and the mixed solution was reacted with stirring for 1 hr. The reaction solution was concentrated under reduced pressure, to obtain 2-(2-(morpholinemethyl)-1*H*-imidazole-1-yl)propionic acid (500 mg, crude product). The crude product was directly used in the next step without further purification.

LCMS (ESI, *m*/*z*): 240.15 [M+H]⁺.

### Synthesis of Compound A006-1

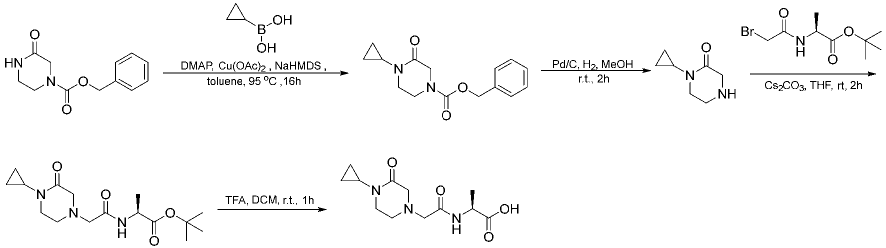

At room temperature, to a solution of benzyl 3-oxopiperazin-1-carboxylate (5.0 g, 21.344 mmol, 1 eq), 4-dimethylaminopyridine (7.82 g, 64.032 mmol, 3 eq), cupric acetate (969.03 mg, 5.336 mmol, 0.25 eq), and cyclopropylboric acid (3.67 g, 42.688 mmol, 2 eq) in toluene (100 mL), sodium bis(trimethylsilyl)amide (10.7 mL, 1 eq, 2M) was added dropwise. After that, the system was heated to 95°C and further stirred for 16 hrs. The reaction mixture was cooled to room temperature, and quenched by adding a saturated ammonium chloride aqueous solution. The obtained residue was concentrated under reduced pressure, and toluene was removed by evaporation. The residue was diluted in a saturated ammonium chloride aqueous solution (80 mL), and extracted with ethyl acetate (3 x 100 mL). The organic phases were combined, back washed with saturated brine (2 x 100 mL), and dried over anhydrous sodium sulfate. After the resulting mixture was filtered, the filtrate was concentrated under reduced pressure, The obtained residue was purified by reverse-phase column chromatography to obtain benzyl 4-cyclopropyl-3-oxopiperazin-1-carboxylate (2.1 g, 35.87%), as a yellow oil.

LCMS (ESI, *m*/*z*): 275.45 [M+H]⁺.

At room temperature, palladium on carbon (100 mg, 0.940 mmol, 0.26 eq) was added to a solution of benzyl 4-cyclopropyl-3-oxopiperazin-1-carboxylate (1 g, 3.645 mmol, 1 eq) in methanol (10.0 mL). The system was purged with nitrogen, and then evacuated. Hydrogen was introduced, and the reaction solution was reacted with stirring at room temperature for 2 hrs. After filtration, the filter cake was washed with methanol (3 x 10 mL), and the filtrate was concentrated under reduced pressure, to obtain 1-cyclopropylpiperazin-2-one (500 mg, crude product), as a light yellow oil.

LCMS (ESI, *m*/*z*): 141.15 [M+H]⁺.

Under a nitrogen atmosphere and at room temperature, to a solution of 1-cyclopropylpiperazin-2-one (300 mg, 2.140 mmol, 1 eq) and cesium carbonate (2.09 g, 6.420 mmol, 3 eq) in tetrahydrofuran (20.0 mL), (2-bromoacetyl)-L-alanine t-butyl ester (683.44 mg, 2.568 mmol, 1.2 eq) was added. After that, the system was further stirred at room temperature for 2 hrs. The reaction solution was concentrated under reduced pressure, diluted with water (30 mL), and extracted with ethyl acetate (3 x 50 mL). The organic phases were combined, back washed with saturated brine (1 x 40 mL), and dried over anhydrous sodium sulfate. After the resulting mixture was filtered, the filtrate was concentrated under reduced pressure, The obtained residue was purified by reverse-phase column chromatography to obtain (2-(4-cyclopropyl-3-oxopiperazin-1-yl)acetyl)-*L*-alanine t-butyl ester (500 mg, 71.80%), as a yellow oil.

LCMS (ESI, *m*/*z*): 326.20 [M+H]⁺.

At room temperature, to a solution of (2-(4-cyclopropyl-3-oxopiperazin-1-yl)acetyl)-*L*-alanine t-butyl ester (400 mg, 1.229 mmol, 1 eq) in dichloromethane (8.0 mL), trifluoroacetic acid (2.0 mL) was added dropwise. After that, the system was further stirred at room temperature for 1 hr. The reaction solution was concentrated under reduced pressure, to obtain (2-(4-cyclopropyl-3-oxopiperazin-1-yl)acetyl)-*L*-alanine (800 mg, crude product). The crude product was directly used in the next step without further purification.

LCMS (ESI, *m*/*z*): 270.45 [M+H]⁺.

### Synthesis of Compound A009-1

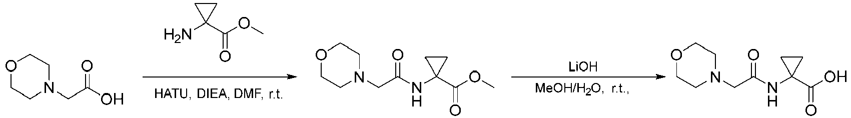

Under a nitrogen atmosphere and at room temperature, to a solution of morpholine-4-ylacetic acid (500 mg, 3.445 mmol, 1 eq) in *N,N*-dimethyl formamide (10 mL), methyl 1-aminocyclopropan-1-carboxylate (475.89 mg, 4.134 mmol, 1.2 eq), *N*,*N*,*N'*,*N'*-tetramethyl-*O*-(7-azabenzotriazol-1-yl)uronium hexafluorophosphate (2.62 g, 6.890 mmol, 2 eq), and *N,N*-diisopropylethyl amine (1.78 g, 13.780 mmol, 4 eq) were added, and the reaction solution was reacted with stirring at room temperature for 2 hrs. The reaction solution was quenched with water at room temperature, and extracted with ethyl acetate (3 x 50 mL). The organic phases were combined, back washed with saturated brine (1 x 50 mL), and dried over anhydrous sodium sulfate. After the resulting mixture was filtered, the filtrate was concentrated under reduced pressure, to obtain methyl 1-[2-(morpholine-4-yl)acetamido]cyclopropan-1-carboxylate (500 mg, crude product), as a colorless and transparent oil.

LCMS (ESI, *m*/*z*): 243.40, [M+H]⁺.

At room temperature, lithium hydroxide (148.28 mg, 6.192 mmol, 3 eq) was added to a solution of methyl 1-[2-(morpholine-4-yl)acetamido]cyclopropan-1-carboxylate (500 mg, 2.064 mmol, 1 eq) in tetrahydrofuran (4 mL) and water (2 mL), and the reaction solution was stirred at room temperature for 2 hrs. The reaction mixture was acidified to pH 6 with 1 mol/L hydrochloric acid. The mixture was concentrated under reduced pressure, to obtain 1-[2-(morpholine-4-yl)acetamido]cyclopropan-1-carboxylic acid (500 mg, crude product), as a white solid.

LCMS (ESI, *m*/*z*): 229.40, [M+H]⁺.

### Synthesis of Compound A013-1

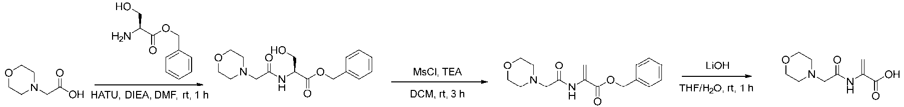

At room temperature, to a solution of morpholine-4-ylacetic acid (2 g, 13.778 mmol, 1 eq) and benzyl (2S)-2-amino-3-hydroxypropionate (3.23 g, 16.534 mmol, 1.2 eq) in *N,N*-dimethyl formamide (20 mL), *N*,*N*,*N'*,*N'*-tetramethyl-O-(7-azabenzotriazol-1-yl)uronium hexafluorophosphate (7.86 g, 20.667 mmol, 1.5 eq) and N,N-diisopropylethyl amine (7.2 mL, 41.334 mmol, 3 eq) were added batchwise. The reaction solution was continuously stirred at room temperature for 1 hr. The reaction solution was concentrated under reduced pressure. The obtained residue was purified by reverse-phase column chromatography to obtain benzyl (2S)-3-hydroxy-2-[2-(morpholine-4-yl)acetamido]propionate (4.1 g, 92.31%), as a white solid.

LCMS (ESI, *m*/*z*): 323.45, [M+H]⁺.

Under a nitrogen atmosphere and at 0°C, to a solution of benzyl (2S)-3-hydroxy-2-[2-(morpholine-4-yl)acetamido]propionate (4.1 g, 12.719 mmol, 1 eq) in dichloromethane (20 mL), triethyl amine (3.54 mL, 25.438 mmol, 2 eq) was added, and methylsulfonyl chloride (1.48 mL, 19.078 mmol, 1.5 eq) was slowly added dropwise. The reaction system was stirred at room temperature for 3 hrs. The reaction solution was quenched with water at room temperature. The mixture was concentrated under reduced pressure, and the obtained residue was purified by reverse-phase column chromatography to obtain benzyl 2-[2-(morpholine-4-yl)acetamido]prop-2-enoate (0.8 g, 21.22%), as a white solid.

LCMS (ESI, *m*/*z*): 305.10, [M+H]⁺.

At room temperature, to a solution of benzyl 2-[2-(morpholine-4-yl)acetamido]prop-2-enoate (400 mg, 1.314 mmol, 1 eq) in tetrahydrofuran/water (4.0 mL, 3/1, v/v), anhydrous lithium hydroxide (94.43 mg, 3.942 mmol, 3 eq) was added. The reaction solution was continuously stirred at room temperature for 1 hr. The reaction solution was acidified to pH 6 with 1 mol/L dilute hydrochloric acid, and the obtained residue was concentrated under reduced pressure. The obtained mixture was directly used in the next step without further purification.

LCMS (ESI, *m*/*z*): 215.10, [M+H]⁺.

### Synthesis of Compound A019-1

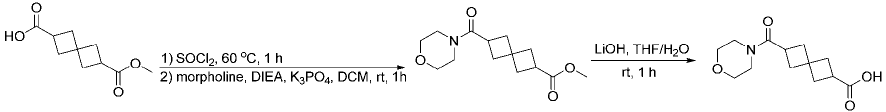

Under a nitrogen atmosphere, a solution of 6-(methoxycarbonyl)spiro[3.3]heptan-2-carboxylic acid (50 mg, 0.252 mmol, 1 equiv) in thionyl chloride (90.02 mg, 0.756 mmol, 3 equiv) was heated at 60°C for 1 hr. The reaction solution was concentrated under reduced pressure, to obtain a crude product. At room temperature, potassium phosphate (53.54 mg, 0.252 mmol, 1 equiv) was added to a solution of morpholine (65.93 mg, 0.756 mmol, 3 equiv) in dichloromethane (1 mL), and then diisopropylethylamine (32.6 mg, 0.252 mmol, 1 equiv) was added dropwise. At 0°C, a solution of the crude product in dichloromethane (1 mL) was added dropwise to the reaction system, and the reaction solution was heated to room temperature and continuously stirred for 1 hr. The reaction solution was concentrated under reduced pressure, and the obtained residue was purified by reverse-phase column chromatography to obtain methyl 6-(morpholine-4-carbonyl)spiro[3.3]heptan-2-carboxylate (50 mg, 74.15%) as a colorless and transparent liquid.

LCMS (ESI, *m*/*z*): 268.10 [M+H]⁺.

At room temperature, to a solution of methyl 6-(morpholine-4-carbonyl)spiro[3.3]heptan-2-carboxylate (50 mg, 0.187 mmol, 1 equiv) in tetrahydrofuran/water (1.2 mL, v/v, 3/1), lithium hydroxide (13.44 mg, 0.561 mmol, 3 equiv) was added. The reaction solution was stirred at room temperature for 1 hr, and neutralized to pH 6 with 1 mol/L dilute hydrochloric acid. The mixture was concentrated under reduced pressure, to obtain 6-(morpholine-4-carbonyl)spiro[3.3]heptan-2-carboxylic acid (50 mg, crude product). The crude product was directly used in the next step without further purification.

LCMS (ESI, *m*/*z*): 254.45 [M+H]⁺.

### Synthesis of Compound A020-1

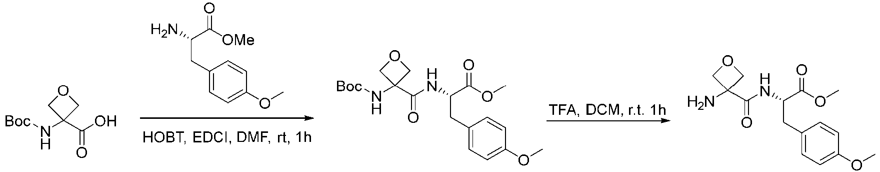

Under a nitrogen atmosphere and at room temperature, to a solution of 3-((t-butoxycarbonyl)amino)oxacyclobutan-3-carboxylic acid (500 mg, 2.302 mmol, 1 eq), 1-hydroxybenzotriazole (622.07 mg, 4.604 mmol, 2 eq), and 1-(3-dimethylaminopropyl)-3-ethylcarbodiimide hydrochloride(1.32 g, 6.906 mmol, 3 eq) in *N,N*-dimethyl formamide (15.0 mL), methyl (*S*)-2-amino-3-(4-methoxyphenyl)propionate (481.64 mg, 2.302 mmol, 1 eq) was added. After that, the system was further stirred at room temperature for 1 hr. The reaction mixture was diluted with water (50 mL), and extracted with ethyl acetate (3 x 30 mL). The organic phases were combined, back washed with saturated brine (1 x 30 mL), and dried over anhydrous sodium sulfate. After the resulting mixture was filtered, the filtrate was concentrated under reduced pressure. The obtained residue was purified by reverse-phase column chromatography to obtain methyl (*S*)-2-(3-((t-butoxycarbonyl)amino)oxacyclobutan-3-formamido)-3-(4-methoxyphenyl)propionate (900 mg, 95.73%), as a white solid.

LCMS (ESI, *m*/*z*): 409.15 [M+H]⁺.

At room temperature, to a solution of methyl (*S*)-2-(3-((t-butoxycarbonyl)amino)oxacyclobutan-3-formamido)-3-(4-methoxyphenyl)propionate (500 mg, 1.224 mmol, 1 eq) in dichloromethane (4.0 mL), trifluoroformic acid (1.0 mL) was added dropwise. After that, the system was further stirred at room temperature for 1 hr. The reaction solution was concentrated under vacuum, to obtain methyl (*S*)-2-(3-aminooxacyclobutan-3-formamido)-3-(4-methoxyphenyl)propionate (1.0 g, crude product). The crude product was directly used in the next step without further purification.

LCMS (ESI, *m*/*z*): 309.05 [M+H]⁺.

### Synthesis of Compound A028-1

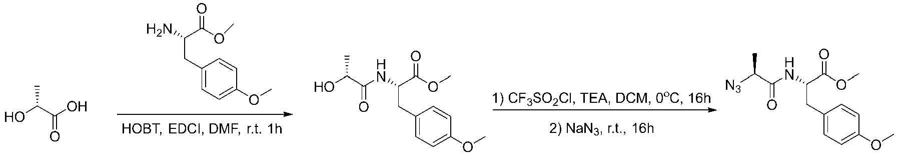

Under a nitrogen atmosphere and at room temperature, to a solution of (*R*)-2-hydroxypropionic acid (1 g, 11.101 mmol, 1 eq), 1-hydroxybenzotriazole (3.00 g, 22.202 mmol, 2 eq), and 1-(3-dimethylaminopropyl)-3-ethylcarbodiimide hydrochloride(6.38 g, 33.303 mmol, 3 eq) in N,N-dimethyl formamide (30.0 mL), methyl (*S*)-2-amino-3-(4-methoxyphenyl)propionate (2.32 g, 11.101 mmol, 1 eq) was added. After that, the system was further stirred at room temperature for 1 hr. The reaction mixture was quenched with water (60 mL) at room temperature, and extracted with ethyl acetate (3 x 50 mL). The organic phases were combined, back washed with saturated brine (1 x 50 mL), dried over anhydrous sodium sulfate, and filtered. The filtrate was concentrated under reduced pressure. The obtained residue was purified by reverse-phase column chromatography to obtain methyl (*S*)-2-((*R*)-2-hydroxypropionamido)-3-(4-methoxyphenyl)propionate (1.2 g, 38.43%), as a white solid.

LCMS (ESI, *m*/*z*): 282.40 [M+H]⁺.

Under a nitrogen atmosphere and at 0°C, to a solution of methyl (*S*)-2-((*R*)-2-hydroxypropionamido)-3-(4-methoxyphenyl)propionate (500 mg, 1.777 mmol, 1 eq) and triethyl amine (1.61 mL, 11.550 mmol, 6.5 eq) in dichloromethane (15.0 mL), trifluoromethansulfonyl chloride (1.14 mL, 10.662 mmol, 6 eq) was added dropwise. The system was heated to room temperature and stirred for 16 hrs. At 0°C, sodium azide (577.76 mg, 8.885 mmol, 5 eq) was added to the system batchwise. After that, the system was heated to room temperature and further stirred for 16 hrs. The reaction mixture was quenched with iced water (50 mL), and extracted with dichloromethane (3 x 50 mL). The organic phases were combined, back washed with saturated brine (1 x 30 mL), and dried over anhydrous sodium sulfate. After the resulting mixture was filtered, the filtrate was concentrated under reduced pressure. The obtained residue was purified by reverse-phase column chromatography to obtain methyl (*S*)-2-((*S*)-2-azidopropionamido)-3-(4-methoxyphenyl)propionate (136 mg, 24.98%), as a brown oil.

LCMS (ESI, *m*/*z*): 307.10 [M+H]⁺.

### Synthesis of Compound A036-1

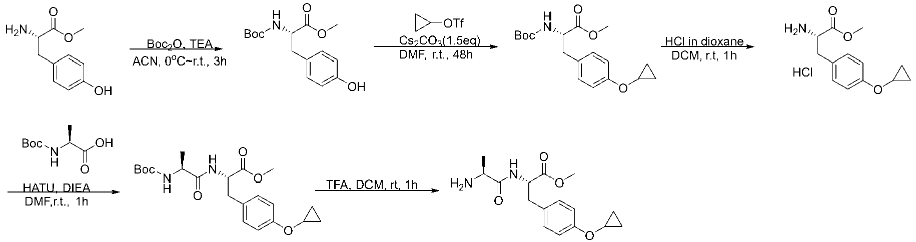

Under a nitrogen atmosphere and at room temperature, triethyl amine (5.18 g, 51.225 mmol, 1 eq) was added dropwise to a solution of tyrosine methyl ester (10 g, 51.225 mmol, 1 eq) in acetonitrile (100 mL). At 0°C, di-t-butyl dicarbonate (17.92 g, 76.838 mmol, 1.5 eq) was added dropwise. The reaction solution was heated to room temperature, reacted with stirring for 3 hrs, and quenched with water (200 mL). The reaction mixture was extracted with ethyl acetate (3 x 200 mL). The organic phases were combined, back washed with saturated brine (2 x 200 mL), and dried over anhydrous sodium sulfate. After the resulting mixture was filtered, the filtrate was concentrated under reduced pressure, The residue was purified by column chromatography on silica gel to obtain (t-butoxycarbonyl)-L-tyrosine methyl ester (10 g, 66.10%), as a white solid.

LCMS (ESI, *m*/*z*): 240.05 [M+H]⁺.

Under a nitrogen atmosphere and at 0°C, to a solution of (t-butoxycarbonyl)-L-tyrosine methyl ester (970 mg, 3.284 mmol, 1 eq) and cesium carbonate (1.07 g, 3.284 mmol, 1 eq) in N,N-dimethyl formamide(10 mL), cyclopropyl trifluoromethansulfonate (749.4 mg, 3.941 mmol, 1.2 eq) was added dropwise. The reaction solution was reacted with stirring at room temperature for 48 hrs, quenched with water (20 mL), and extracted with ethyl acetate (3 x 40 mL). The organic phases were combined, back washed with saturated brine (2 x 40 mL), and dried over anhydrous sodium sulfate. After the resulting mixture was filtered, the filtrate was concentrated under reduced pressure. The residue was purified by column chromatography on silica gel to obtain methyl (S)-2-((t-butoxycarbonyl)amino)-3-(4-cyclopropyloxyphenyl)propionate (700 mg, 63.54%), as a white solid.

LCMS (ESI, *m*/*z*): 236.05 [M+H]⁺.

At room temperature, to a solution of methyl (5)-2-((t-butoxycarbonyl)amino)-3-(4-cyclopropyloxyphenyl)propionate (700 mg, 2.087 mmol, 1 eq) in dichloromethane (3 mL), a solution of hydrogen chloride in 1,4-dioxane (3 mL, 4 M) was added dropwise. The reaction solution was reacted with stirring at room temperature for 1 hr, and concentrated under vacuum, to obtain methyl (*S*)-2-amino-3-(4-cyclopropyloxyphenyl)propionate hydrochloride(700 mg, crude product), as a white solid. The crude product was directly used in the next step without further purification.

LCMS (ESI, *m*/*z*): 236.45 [M+H]⁺.

Under a nitrogen atmosphere and at room temperature, to a solution of (2S)-2-[(t-butoxycarbonyl)amino]propionate (603.14 mg, 3.188 mmol, 1.5 eq) and *N,N,N',N'-*tetramethyl-O-(7-azabenzotriazol-1-yl)uronium hexafluorophosphate(1.62 g, 4.250 mmol, 2 eq) in *N,N*-dimethyl formamide (5 mL), *N,N*-diisopropylethyl amine (823.99 mg, 6.375 mmol, 3 eq) was added dropwise. After reaction with stirring for 5 min, methyl (*S*)-2-amino-3-(4-cyclopropyloxyphenyl)propionate (500 mg, 2.125 mmol, 1 eq) was added. The reaction solution was reacted with stirring at room temperature for 1 hr. The reaction mixture was quenched with water (20 mL), and extracted with ethyl acetate (3 x 40 mL). The organic phases were combined, back washed with saturated brine (2 x 40 mL), and dried over anhydrous sodium sulfate. After the resulting mixture was filtered, the filtrate was concentrated under reduced pressure, The residue was purified by column chromatography on silica gel to obtain methyl (*S*)-2-((*S*)-2-((t-butoxycarbonyl)amino)propionamido)-3-(4-cyclopropyloxyphenyl)propionate (600 mg, 69.46%), as a white solid.

LCMS (ESI, *m*/*z*): 351.10 [M+H]⁺.

At room temperature, to a solution of methyl (*S*)-2-((*S*)-2-((t-butoxycarbonyl)amino)propionamido)-3-(4-cyclopropyloxyphenyl)propionate (300 mg, 0.738 mmol, 1 eq) in dichloromethane (2 mL), trifluoroacetic acid (0.5 mL, 6.732 mmol, 9.12 eq) was added dropwise. The reaction solution was reacted with stirring at room temperature for 1 hr. The reaction solution was concentrated under reduced pressure, to obtain methyl (*S*)-2-((*S*)-2-aminopropionamido)-3-(4-cyclopropyloxyphenyl)propionate (300 mg, crude product), as a white solid. The crude product was directly used in the next step without further purification.

LCMS (ESI, *m*/*z*): 305.10 [M+H]⁺.

### Synthesis of Compound A038-1

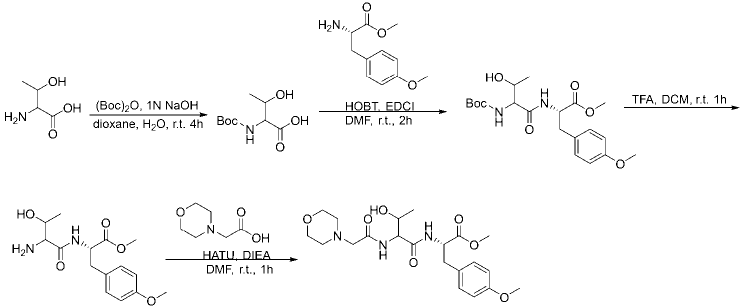

Under a nitrogen atmosphere and at room temperature, sodium hydroxide aqueous solution (10 mL, 3 eq, 1N) was added to 2-amino-3-hydroxybutyric acid (2.0 g, 16.790 mmol, 1 eq) in a mixed solvent of 1,4-dioxane/water (volume ratio 1:1, 20 mL), di-t-butyl dicarbonate (5.39 mL, 25.190 mmol, 1.50 eq) was added dropwise. After that, the system was further stirred at room temperature for 4 hrs. The reaction mixture was neutralized to pH 5-6 with dilute hydrochloric acid, and extracted with ethyl acetate (3 x 60 mL). The organic phases were combined, back washed with saturated brine (1 x 60 mL), and dried over anhydrous sodium sulfate. After the resulting mixture was filtered, the filtrate was concentrated under reduced pressure, to obtain 2-((t-butoxycarbonyl)amino)-3-hydroxybutyric acid (3.1 g, crude product), as a white solid. The crude product was directly used in the next step without further purification.

LCMS (ESI, *m*/*z*): 164.05 [M+H]⁺.

Under a nitrogen atmosphere and at room temperature, to a solution of 2-((t-butoxycarbonyl)amino)-3-hydroxylbutyric acid (2.0 g, 9.123 mmol, 1 eq), 1-hydroxybenzotriazole (2.47 g, 18.246 mmol, 2 eq), and 1-(3-dimethylaminopropyl)-3-ethylcarbodiimide hydrochloride(5.25 g, 27.369 mmol, 3 eq) in *N,N*-dimethyl formamide (20.0 mL), methyl (5)-2-amino-3-(4-methoxyphenyl)propionate (1.91 g, 9.123 mmol, 1 eq) was added. After that, the system was further stirred at room temperature for 2 hrs. The reaction mixture was quenched with water (50 mL) at room temperature, and extracted with ethyl acetate (3 x 50 mL). The organic phases were combined, back washed with saturated brine (1 x 50 mL), and dried over anhydrous sodium sulfate. After the resulting mixture was filtered, the filtrate was concentrated under reduced pressure. The obtained residue was purified by reverse-phase column chromatography to obtain methyl (2*S*)-2-(2-((t-butoxycarbonyl)amino)-3-hydroxylbutyramido)-3-(4-methoxyphenyl)propionate (2.47 g, 65.96%), as a colorless and transparent oil.

LCMS (ESI, *m*/*z*): 411.15 [M+H]⁺.

At room temperature, trifluoroacetic acid (2.0 mL) was added dropwise to a solution of methyl (2*S*)-2-(2-((t-butoxycarbonyl)amino)-3-hydroxylbutyramido)-3-(4-methoxyphenyl)propionate (700 mg, 1.705 mmol, 1 eq) in dichloromethane (8.0 mL). The reaction solution was reacted with stirring at room temperature for 1 hr. The reaction solution was concentrated under reduced pressure, to obtain methyl (2*S*)-2-(2-amino-3-hydroxylbutyramido)-3-(4-methoxyphenyl)propionate (1.0 g, crude), as a yellow oil. The crude product was directly used in the next step without further purification.

LCMS (ESI, *m*/*z*): 311.15 [M+H]⁺.

Under a nitrogen atmosphere and at room temperature, to a solution of methyl (2S)-2-(2-amino-3-hydroxylbutyramido)-3-(4-methoxyphenyl)propionate (600 mg, 1.933 mmol, 1 eq) and *N,N,N',N'-*tetramethyl-oxy-(7-azabenzotriazol-1-yl)uronium hexafluorophosphate (1.47 g, 3.866 mmol, 2 eq) in *N,N-*dimethyl formamide (15.0 mL), *N,N*-diisopropylethyl amine (1.25 g, 9.665 mmol, 5 eq) and 2-morpholinacetic acid (561.27 mg, 3.866 mmol, 2 eq) were added. After that, the system was further stirred at room temperature for 1 hr. The reaction mixture was quenched with water at room temperature, and extracted with ethyl acetate (3 x 60 mL). The organic phases were combined, back washed with saturated brine (1 x 60 mL), and dried over anhydrous sodium sulfate. After the resulting mixture was filtered, the filtrate was concentrated under reduced pressure. The obtained residue was purified by reverse-phase column chromatography under conditions including: C18 chromatographic column, mobile phase: water and acetonitrile, gradient from 0% to 50% over 10 min, and UV 220 nm. Methyl (2S)-2-(3-hydroxyl-2-(2-morpholinoacetamido)butyramido)-3-(4-methoxyphenyl)propionate (450 mg, 53.20%) as a yellow oil was obtained.

LCMS (ESI, *m*/*z*): 438.15 [M+H]⁺.

### Synthesis of Compound A040-1

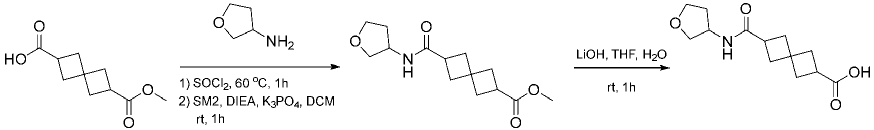

Under a nitrogen atmosphere, a solution of 6-(methoxycarbonyl)spiro[3.3]heptan-2-carboxylic acid (100 mg, 0.504 mmol, 1 eq) in thionyl chloride (2.0 mL) was reacted at 60°C with stirring for 1 hr. The reaction solution was concentrated under vacuum, and thionyl chloride was removed by evaporation. The mixture was dissolved in dichloromethane (2.0 mL) at room temperature. N,N-diisopropylethyl amine (65.2 mg, 0.504 mmol, 1 eq) and potassium phosphate (107.09 mg, 0.504 mmol, 1 eq) were added to the system. Finally, tetrahydrofuran-3-amine (87.91 mg, 1.008 mmol, 2 eq) was slowly added dropwise. After that, the system was further stirred at room temperature for 1 hr. The reaction mixture was diluted with water (40 mL), and extracted with dichloromethane (3 x 30 mL). The organic phases were combined, back washed with saturated brine (1 x 30 mL), and dried over anhydrous sodium sulfate. After the resulting mixture was filtered, the filtrate was concentrated under reduced pressure. The obtained residue was purified by reverse-phase column chromatography to obtain methyl 6-((tetrahydrofuran -3-yl)carbamyl)spiro[3.3]heptan-2-carboxylate (110 mg, 81.56%), as a yellow solid.

LCMS (ESI, *m*/*z*): 268.05 [M+H]⁺.

At room temperature, a mixed solution of methyl 6-((tetrahydrofuran -3-yl)carbamyl)spiro[3.3]heptan-2-carboxylate (100 mg, 0.374 mmol, 1 eq) and lithium hydroxide (17.92 mg, 0.748 mmol, 2 eq) in tetrahydrofuran (1.0 mL) and water (1.0 mL) was reacted with stirring for 1 hr. The reaction solution was adjusted to pH 5-6 with 1 mol/L dilute hydrochloric acid, and the mixture was concentrated under vacuum, to obtain 6-((tetrahydrofuran -3-yl)carbamyl)spiro[3.3]heptan-2-carboxylic acid (160 mg, crude product). The crude product was directly used in the next step without further purification.

LCMS (ESI, *m*/*z*): 254.05 [M+H]⁺.

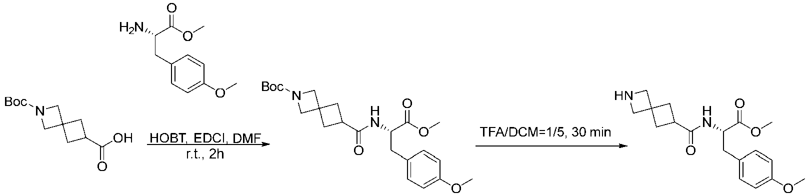

Under a nitrogen atmosphere and at room temperature, methyl (S)-2-amino-3-(4-methoxyphenyl)propionate (476.96 mg, 2.279 mmol, 1.1 eq), 1-hydroxybenzotriazole (420.02 mg, 3.108 mmol, 1.5 eq), and 1-(3-dimethylaminopropyl)-3-ethylcarbodiimide hydrochloride (1.19 g, 6.216 mmol, 3 eq) were added to a solution of 2-(t-butoxycarbonyl)-2-azaspiro[3.3]heptan-6-carboxylic acid (500 mg, 2.072 mmol, 1 eq) in N,N-dimethyl formamide (10 mL). The reaction solution was stirred at room temperature for 1 hr. The reaction solution was quenched with water at room temperature, and extracted with ethyl acetate (3 x 50 mL). The organic phases were combined, back washed with saturated brine (1 x 50 mL), and dried over anhydrous sodium sulfate. After the resulting mixture was filtered, the filtrate was concentrated under reduced pressure. The obtained residue was purified by reverse-phase column chromatography, to obtain t-butyl (S)-6-((1-methoxy-3-(4-methoxyphenyl)-1-oxoprop-2-yl)carbamyl)-2-azaspiro[3.3]heptan-2-carboxylate (500 mg, 55.79%), as a colorless oil.

LCMS (ESI, *m*/*z*): 432.90, [M+H]⁺.

At room temperature, trifluoroacetic acid (0.5 mL, 6.732 mmol, 17.13 eq) was added to a solution of t-butyl (*S*)-6-((1-methoxy-3-(4-methoxyphenyl)-1-oxoprop-2-yl)carbamyl)-2-azaspiro[3.3]heptan-2-carboxylate (170 mg, 0.393 mmol, 1 eq) in dichloromethane (2.5 mL). The reaction solution was stirred at room temperature for 30 min. The reaction solution was concentrated under reduced pressure. The crude product was directly used in the next step without further purification.

LCMS (ESI, *m*/*z*): 332.90, [M+H]⁺.

### Example 1

### Synthesis of Compound A001

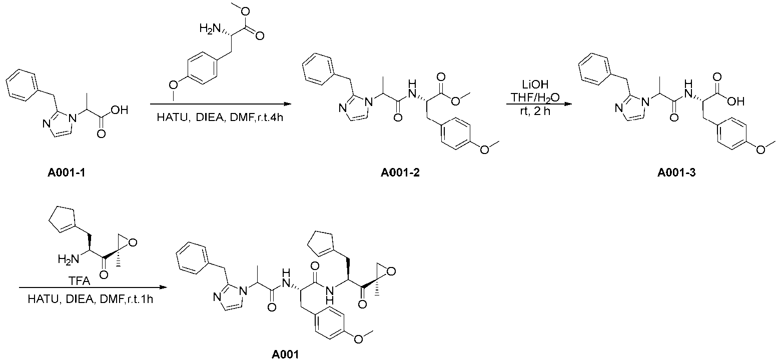

**Step 1:** Under a nitrogen atmosphere and at room temperature, to a solution of 2-(2-benzylimidazole-1-yl)propionate (120 mg, 0.521 mmol, 1 eq) and methyl (2*S*)-2-amino-3-(4-methoxyphenyl)propionate (130.85 mg, 0.625 mmol, 1.20 eq) in *N,N*-dimethyl formamide (2 mL), *N*,*N*,*N'*,*N'*-tetramethyl-O-(7-azabenzotriazol-1-yl)uronium hexafluorophosphate (396.31 mg, 1.042 mmol, 2.00 eq) and *N,N*-diisopropylethyl amine (453.87 uL, 2.605 mmol, 5 eq) were added. After that, the system was further stirred at room temperature for 4 hrs. The reaction mixture was quenched with water at room temperature. The reaction mixture was extracted with ethyl acetate (3 x 50 mL). The organic phases were combined, back washed with saturated brine (1 x 50 mL), and dried over anhydrous sodium sulfate. After the resulting mixture was filtered, the filtrate was concentrated under reduced pressure. The obtained residue was purified by reverse-phase column chromatography under conditions including: chromatographic column specification: C18; mobile phase: water and acetonitrile, gradient from 10% to 50% over 10 min, and UV 220 nm. Methyl (2S)-2-[2-(2-benzylimidazole-1-yl)propionamido]-3-(4-methoxyphenyl)propionate (150 mg, 68.29%) as an off-white solid was obtained. LCMS-A001-2: (ESI, *m*/*z):* 422.45, [M-H]⁻.

**Step 2:** At room temperature, lithium hydroxide (25.57 mg, 1.068 mmol, 3 eq) was added to a solution of methyl (2*S*)-2-[2-(2-benzylimidazole-1-yl)propionamido]-3-(4-methoxyphenyl)propionate (150 mg, 0.356 mmol, 1 eq) in tetrahydrofuran (2 mL) and water (1 mL), and reacted with stirring for 2 hrs. The reaction mixture was quenched with water at room temperature. The reaction mixture was neutralized to pH 7 with 1 mol/L dilute hydrochloric acid. The reaction mixture was extracted with ethyl acetate (3 x 20 mL). The organic phases were combined, back washed with saturated brine (1 x 50 mL), and dried over anhydrous sodium sulfate. After the resulting mixture was filtered, the filtrate was concentrated under reduced pressure, to obtain (2*S*)-2-[2-(2-benzylimidazole-l-yl)propionamido]-3-(4-methoxyphenyl)propionate (130 mg, crude product), as a white solid.

LCMS-A001-3: (ESI, *m*/*z):* 408.45, [M+H]⁺.

**Step 3:** Under a nitrogen atmosphere and at room temperature, *N,N,N',N'*-tetramethyl-oxy-(7-azabenzotriazol-1-yl)uronium hexafluorophosphate (155.53 mg, 0.408 mmol, 2 eq) and *N,N-*diisopropylethyl amine (132.16 mg, 1.021 mmol, 5 eq) were added to a solution of (2*S*)-2-[2-(2-benzylimidazole-1-yl)propionamido]-3-(4-methoxyphenyl)propionate (100 mg, 0.245 mmol, 1.2 eq) in *N,N-*dimethyl formamide (2.5 mL). Then, (2S)-2-amino-3-(cyclopent-1-en-1-yl)-1-[(2R)-2-methyloxiran-2-yl]propan-1-one trifluoroacetate (39.93 mg, 0.204 mmol, 1 eq) was added. The reaction solution was stirred at room temperature for 1 hr. The reaction mixture was quenched with water at room temperature. The reaction mixture was extracted with ethyl acetate (3 x 20 mL). The organic phases were combined, back washed with saturated brine (3 x 20 mL), and dried over anhydrous sodium sulfate. After the resulting mixture was filtered, the filtrate was concentrated under reduced pressure. The crude product was purified by high performance liquid chromatography to obtain (2S)-2-[2-(2-benzylimidazole-1-yl)propionamido]-N-[(2*S*)-3-(cyclopent-1-en-1-yl)-1-[(2*R*)-2-methyloxiran-2-yl]-1-oxoprop-2-yl]-3-(4-methoxyphenyl)propionamide (41.5 mg, 33.98%), as a white solid.

LCMS-A001: (ESI, *m*/*z):* 585.40 [M+H]⁺.

¹H NMR-A001 (400 MHz, Methanol-d4) *δ* 7.20 - 7.13 (m, 2H), 7.12 - 6.97 (m, 3H), 6.97 - 6.87 (m, 3H), 6.85 - 6.77 (m, 1H), 6.72 - 6.68 (m, 1H), 6.68 - 6.63 (m, 1H), 5.33 (d, *J* = 10.2 Hz, 1H), 4.70 - 4.60 (m, 1H), 4.55 - 4.50 (m, 1H), 4.43 - 4.36 (m, 1H), 4.02 - 3.83 (m, 2H), 3.78 - 3.70 (m, 1H), 3.64 (d, *J* = 9.5 Hz, 3H), 3.15 - 3.05 (m, 1H), 2.92 -2.85 (m, 1H), 2.84 - 2.79 (m, 1H), 2.59 -2.50 (m, 1H), 2.40 (d, *J* = 13.7 Hz, 1H), 2.15 (t, *J* = 12.0 Hz, 4H), 1.85 - 1.65 (m, 2H), 1.35 - 1.16 (m, 6H).

### Synthesis of Compound A002

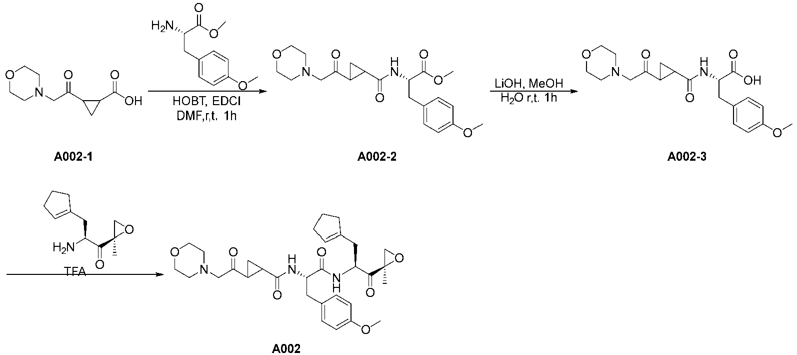

By replacing Intermediate A001-1 in Example 1 with A002-1, the compound *N*-((*S*)-1-(((*S*)-3-(cyclopent-1-en-1-yl)-1-((*R*)-2-methyloxiran-2-yl)-1-oxoprop-2-yl)amino)-3-(4-methoxyphenyl)-1-oxoprop-2-yl)-2-(2-morpholinoacetyl)cyclopropan-1-formamide (2.8 mg, 1.89%) as a white solid was obtained.

LCMS-A002: (ESI, *m*/*z):* 568.05, [M+H]⁺.

¹H NMR-A002 (400 MHz, Chloroform-d) δ 7.19 (dd, *J =* 8.6, 3.4 Hz, 2H), 6.87 - 6.81 (m, 2H), 6.42 (s, 1H), 4.59 - 4.41 (m, 2H), 3.89 (d, *J* = 41.3 Hz, 4H), 3.79 (d, *J* = 3.3 Hz, 3H), 3.53 (s, 1H), 3.31 (d, *J = 5.0* Hz, 1H), 3.02 (dd, *J =* 22.3, 16.5 Hz, 2H), 2.90 (dd, *J =* 4.9, 2.5 Hz, 2H), 2.56 (s, 1H), 2.48 (d, *J =* 14.2 Hz, 2H), 2.22 (s, 4H), 2.07 (d, *J* = 43.3 Hz, 2H),1.85 (d, *J =* 41.2 Hz, 2H) 1.58-1.79 (m, 4H)1.49 (d, *J* = 7.2 Hz, 3H), 1.38 (s, 1H).

### Synthesis of Compound A003

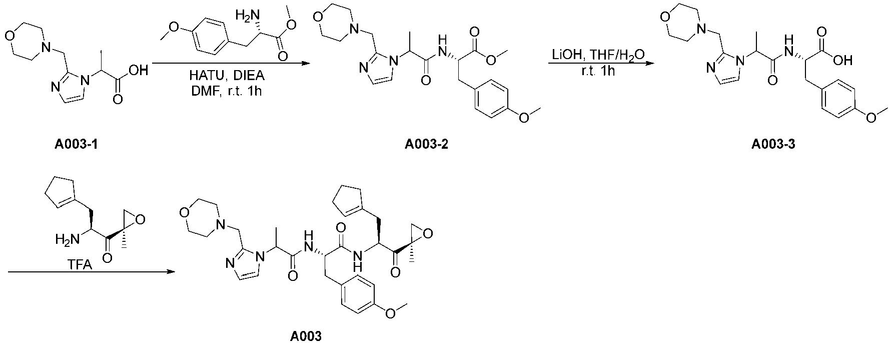

By replacing Intermediate A001-1 in Example 1 with A003-1, the compound (2*S*)-*N*-((*S*)-3-(cyclopent-1-en-1-yl)-1-((*R*)-2-methyloxiran-2-yl)-1-oxoprop-2-yl)-3-(4-methoxyphenyl)-2-(2-(2-(morpholinemethyl)-1*H*-imidazole-1-yl) propionamide (3.6 mg, 3.44%) as a white solid was obtained.

LCMS-A003: (ESI, *m*/*z):* 594.35 [M+H]⁺.

¹H NMR-A003: (400 MHz, Chloroform-d) δ 7.14 - 7.06 (m, 1H), 7.03 - 6.98 (m, 1H), 6.95 (d, *J* = 13.2 Hz, 1H), 6.81 (d, *J* = 8.6 Hz, 1H), 6.73 (d, *J* = 8.5 Hz, 1H), 6.09 (d, *J* = 120.5 Hz, 1H), 5.34 (s, 1H), 5.14 (s, 1H), 4.63 - 4.33 (m, 2H), 3.90 - 3.55 (m, 9H), 3.23 (dd, *J* = 12.1, 5.0 Hz, 1H), 3.05 - 2.93 (m, 1H), 2.93 - 2.79 (m, 2H), 2.76 - 2.63 (m, 1H), 2.51 (d, *J* = 16.3 Hz, 3H), 2.24 (d, *J* = 12.1 Hz, 3H), 2.18 (d, J = 12.7 Hz, 2H), 2.00 (s, 1H), 1.84 (s, 2H), 1.64 - 1.54 (m, 3H), 1.54 - 1.43 (m, 3H), 1.26 (s, 2H).

### Synthesis of Compound A004

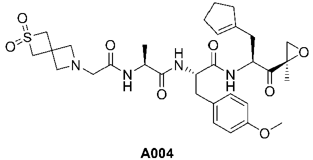

Following the synthesis method for A042, by replacing *N*-(t-butoxycarbonyl)-O-methyl-L-serine with *N*-(t-butoxycarbonyl) -L-serine and replacing 4-oxa-7-azaspiro[2.5]octane hydrochloride with 2-thia-6-azaspiro[3.3]heptance 2,2-dioxide, (*S*)-*N*-((*S*)-3-(cyclopent-1-en-1-yl)-1-((*R*)-2-methyloxiran-2-yl)-1-oxoprop-2-yl)-2-((*S*)-2-(2-(2,2-dioxo-2-thia-6-azaspiro[3.3]hept-6-yl)acetamido)-3-(4-methoxyphenyl) propionamide(13.6 mg, 9.55%) as a white solid was obtained.

LCMS-A004: (ESI, *m*/*z):* 631.25 [M+H]⁺.

¹H NMR-A004: (400 MHz, Chloroform-d) *δ* 7.20 - 7.08 (m, 3H), 6.88 - 6.78 (m, 2H), 6.57 (s, 1H), 5.94 (s, 1H), 5.30 (s, 1H), 4.58 - 4.46 (m, 2H), 4.40 (p, *J* = 7.1 Hz, 1H), 4.25 (s, 4H), 3.79 (s, 3H), 3.51 (s, 4H), 3.26 (d, *J* = 5.1 Hz, 1H), 3.22 - 3.03 (m, 2H), 2.97 (dt, *J =* 15.0, 7.4 Hz, 2H), 2.91 (dd, *J =* 6.1, 2.5 Hz, 1H), 2.50 (d, *J* = 14.4 Hz, 1H), 2.22 (d, *J* = 9.7 Hz, 3H), 2.16 (q, *J* = 8.2 Hz, 2H), 1.82 (p, *J* = 6.7 Hz, 2H), 1.50 (s, 3H), 1.35 (d, *J* = 7.0 Hz, 3H).

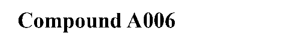

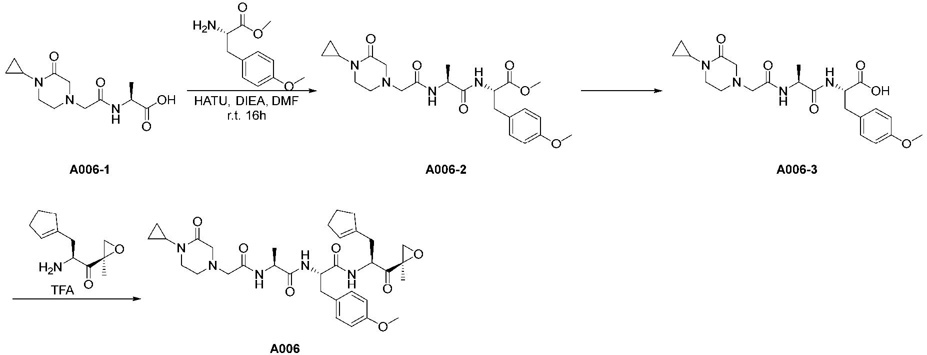

By replacing Intermediate A001-1 in Example 1 with A006-1, the compound (*S*)-*N*-((*S*)-3-(cyclopent-1-en-1-yl)-1-((*R*)-2-methyloxiran-2-yl)-1-oxoprop-2-yl)-2-((*S*)-2-(2-(4-cyclopropyl-3-oxopiperazin-1-yl)acetamido)-3-(4-methoxyphenyl)propionamide (7.3 mg, 6.37%) as a white solid was obtained.

LCMS-A006: (ESI, *m*/*z):* 624.65 [M+H]⁺.

¹H NMR-A006: (400 MHz, Chloroform-*d*) *δ* 7.38 (s, 1H), 7.21 - 7.02 (m, 2H), 6.83 (dd, *J* = 8.2, 4.9 Hz, 2H), 6.56 (d, *J* = 54.5 Hz, 1H), 6.22 - 5.96 (m, 1H), 5.43 - 5.23 (m, 1H), 4.55 (s, 2H), 4.40 (d, *J =* 21.8 Hz, 1H), 3.79 (s, 3H), 3.53 - 3.16 (m, 4H), 3.16 - 2.95 (m, 4H), 2.90 (dd, *J* = 4.9, 2.0 Hz, 1H), 2.74 (s, 3H), 2.48 (s, 1H), 2.20 (d, *J* = 28.5 Hz, 5H), 1.90 - 1.79 (m, 2H), 1.49 (d, *J* = 4.6 Hz, 3H), 1.43 - 1.14 (m, 4H), 0.84 (d, *J* = 7.1 Hz, 2H), 0.70 (s, 2H).

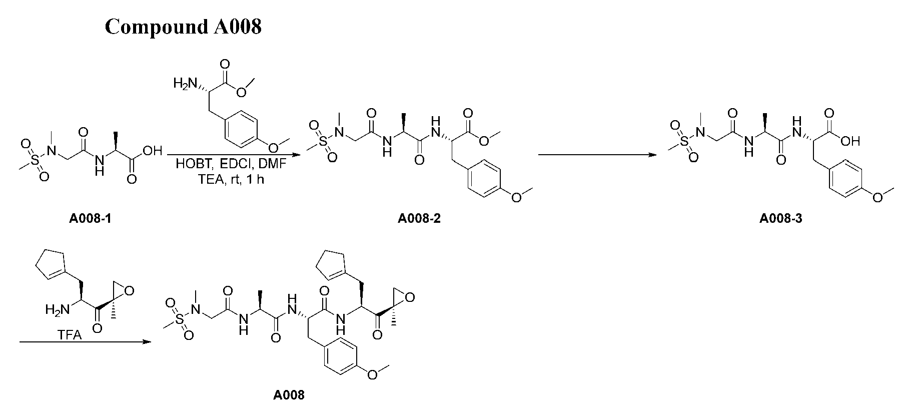

By replacing Intermediate A001-1 in Example 1 with A008-1, the compound (*S*)-*N*-((*S*)-3-(cyclopent-1-en-1-yl)-1-((*R*)-2-methyloxiran-2-yl)-1-oxoprop-2-yl)-3-(4-methoxyphenyl)-2-((*S*)-2-(2-(*N-*methylsulfonamido)acetamido)propionamide (7.6 mg, 17.67%) as a white solid was obtained.

LCMS-A008: (ESI, *m*/*z):* 593.30 [M+H]⁺.

¹H NMR-A008 (400 MHz, Chloroform-d) *δ* 7.17 (d, *J =* 8.5 Hz, 2H), 6.86 - 6.80 (m, 2H), 6.57 (dd, *J =* 18.1, 7.4 Hz, 2H), 5.98 (d, *J =* 6.9 Hz, 1H), 5.30 (s, 1H), 4.52 (q, *J =* 6.7 Hz, 2H), 4.39 (p, *J =* 7.0 Hz, 1H), 3.87 - 3.71 (m, 5H), 3.26 (d, *J =* 5.0 Hz, 1H), 2.97 (dd, *J =* 6.5, 3.1 Hz, 2H), 2.93 (d, *J =* 1.6 Hz, 6H), 2.89 (d, *J* = 5.0 Hz, 1H), 2.49 (d, *J =* 13.9 Hz, 1H), 2.24 - 2.16 (m, 5H), 1.83 (q, *J =* 7.2 Hz, 2H), 1.50 (s, 3H), 1.35 (d*, J* = 7.1 Hz, 3H).

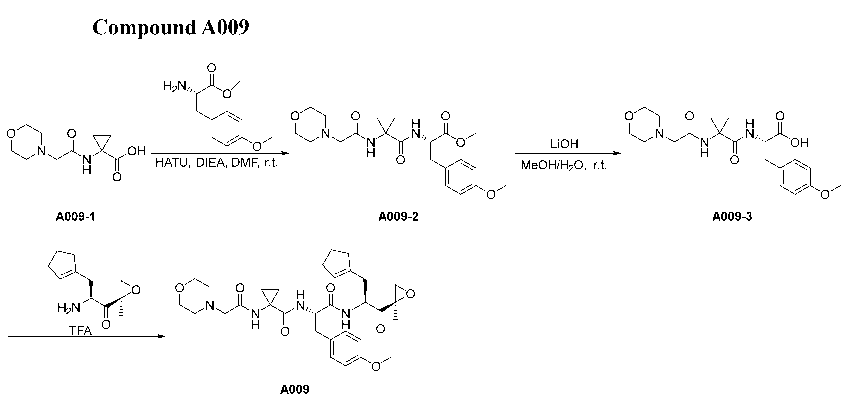

By replacing Intermediate A001-1 in Example 1 with A009-1, following a similar method, (2*S*)-*N*-[(2*S*)-3-(cyclopent-1-en-1-yl)-1-[(2*R*)-2-methyloxiran-2-yl]-1-oxoprop-2-yl]-3-(4-methoxyphenyl)-2-({1-[2-(morpholine-4-yl)acetamido]cyclopropyl}formamido) propionamide (19.54 mg, 67.64%) as a white solid was obtained.

LCMS-A009: (ESI, *m*/*z):* 583.35 [M+H]⁺.

¹H NMR-A009 (400 MHz, Methanol-d4) *δ* 7.01 - 6.87 (m, 2H), 6.76 - 6.64 (m, 2H), 5.33 (s, 1H), 4.59 - 4.52 (m, 1H), 4.48 - 4.43 (m, 1H), 3.66 (s, 3H), 3.61 - 3.52 (m, 4H), 3.08 (d, *J* = 5.2 Hz, 1H), 2.94 - 2.86 (m, 3H), 2.85 - 2.78 (m, 2H), 2.46 - 2.29 (m, 5H), 2.24 - 2.10 (m, 5H), 1.82 - 1.71 (m, 2H), 1.40 - 1.27 (m, 5H), 1.01 - 0.85 (m, 2H).

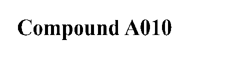

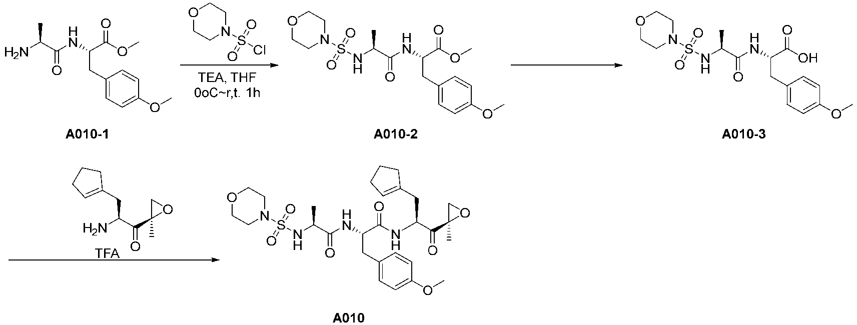

**Step 1:** Under a nitrogen atmosphere and at room temperature, to a solution of methyl (2S)-2-[(2S)-2-aminopropionamido]-3-(4-methoxyphenyl)propionate (prepared by a synthesis method similar to that for Compound A042-3, 400 mg, 1.070 mmol, 1 eq) in tetrahydrofuran (15.0 mL), triethyl amine (0.45 mL, 3.242 mmol, 3.03 eq) was added. The reaction solution was cooled to 0°C, and then morpholinesulfonyl chloride (0.15 mL, 1.209 mmol, 1.13 eq) was slowly added. The reaction solution was heated to room temperature and reacted with stirring for 1 hr. The reaction solution was quenched with water at room temperature, and extracted with ethyl acetate (3 x 30 mL). The organic phases were combined, back washed with a saturated sodium chloride solution (2 x 30 mL), and dried over anhydrous sodium sulfate. After the resulting mixture was filtered, the filtrate was concentrated under reduced pressure, The resulting crude product was purified by reverse-phase column chromatography, to obtain methyl (S)-3-(4-methoxyphenyl)-2-((S)-2-(morpholine-4-sulfonamido)propionamido)propionate (154 mg, 26.13%) as a colorless and transparent oil.

LCMS-A010-2: (ESI, *m*/*z):* 430.45, [M+H]⁺.

**Step 2:** At room temperature, lithium hydroxide (25.09 mg, 1.047 mmol, 3 eq) and hydrogen peroxide (0.95 mL, 2.792 mmol, 8 eq) were added to methyl (*S*)-3-(4-methoxyphenyl)-2-((*S*)-2-(morpholine-4-sulfonamido)propionamido)propionate (150 mg, 0.349 mmol, 1 eq) in a mixed solvent of tetrahydrofuran (37.5 mL) and water (37.5 mL). The reaction solution was reacted with stirring at room temperature for 1 hr. The reaction solution was adjusted to pH 5 with 1 mol/L dilute hydrochloric acid, and the obtained residue was concentrated under reduced pressure, to obtain (*S*)-3-(4-methoxyphenyl)-2-((*S*)-2-(morpholine-4-sulfonamido)propionamido)propionate (80 mg, crude product). The crude product was directly used in the next step without purification.

LCMS-A010-3: (ESI, *m*/*z*): 416.15, [M+H]⁺.

**Step 3**: Following a method similar to that in Step 3 of Example 1, (*S*)-*N*-((*S*)-3-(cyclopent-1-en-1-yl)-1-((*R*)-2-methyloxiran-2-yl)-1-oxoprop-2-yl)-3-(4-methoxyphenyl)-2-((*S*)-2-(morpholine-4-sulfonamido) propionamide(14.1mg, 12.29%) as a white solid was obtained.

LCMS-A010: (ESI, *m*/*z):* 593.25, [M+H]⁺.

¹H NMR-A010 (400 MHz, Chloroform-d) *δ* 7.20 - 7.12 (m, 2H), 6.90 - 6.77 (m, 2H), 6.59 (d, *J* = 7.6 Hz, 1H), 5.89 (d, *J* = 6.8 Hz, 1H), 5.28 (s, 1H), 4.81 (d, *J* = 7.6 Hz, 1H), 4.60 - 4.45 (m, 2H), 3.88 (p, *J* = 7.1 Hz, 1H), 3.79 (s, 3H), 3.69 (t, *J* = 4.7 Hz, 4H), 3.26 (d, *J* = 5.0 Hz, 1H), 3.25 - 3.09 (m, 4H), 3.03 (d, *J* = 6.0 Hz, 1H), 2.97 - 2.87 (m, 2H), 2.48 (d, *J =* 13.4 Hz, 1H), 2.21 (d, *J* = 12.6 Hz, 2H), 2.14 (d, *J =* 6.1 Hz, 2H), 1.89 - 1.74 (m, 2H), 1.53 (s, 3H), 1.38 (d, *J* = 7.1 Hz, 3H).

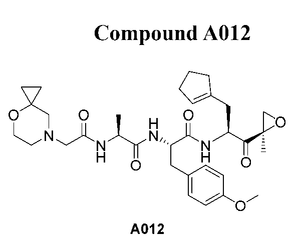

Following the synthesis method for A042, by replacing *N*-(t-butoxycarbonyl)-*O*-methyl-L-serine with *N*-(t-butoxycarbonyl) -L-serine, (2*S*)-*N*-[(2*S*)-3-(cyclopent-1-en-1-yl)-1-[(2*R*)-2-methyloxiran-2-yl]-1-oxoprop-2-yl]-3-(4-methoxyphenyl)-2-[(2*S*)-2-(2-{4-oxa-7-azaspiro[2.5]octan-7-yl}acetamido) propionamide(35.4 mg, 22.06%) as a white solid was obtained.

LCMS-A012: (ESI, *m*/*z):* 597.35 [M+H]⁺.

¹H NMR-A012 (400 MHz, Methanol-d4) *δ* 7.54 (d, *J* = 7.8 Hz, 1H), 7.20 - 7.09 (m, 2H), 6.86 - 6.77 (m, 2H), 6.67 (d, *J* = 7.5 Hz, 1H), 6.04 (d, *J* = 6.8 Hz, 1H), 5.31 (s, 1H), 4.61 - 4.48 (m, 2H), 4.45 - 4.30 (m, 1H), 3.77 (d, *J =* 8.2 Hz, 5H), 3.27 (d, *J* = 5.0 Hz, 1H), 3.05 - 2.84 (m, 5H), 2.61 - 2.54 (m, 2H), 2.50 (d, *J* = 15.2 Hz, 1H), 2.41 (s, 2H), 2.23 (d, *J* = 9.2 Hz, 3H), 2.19 - 2.11 (m, 2H), 1.89 - 1.78 (m, 2H), 1.60 (s, 3H), 1.34 (d, *J* = 7.0 Hz, 3H), 0.84 - 0.76 (m, 2H), 0.60 - 0.53 (m, 1H), 0.51 - 0.44 (m, 1H).

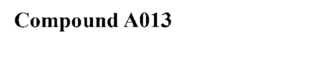

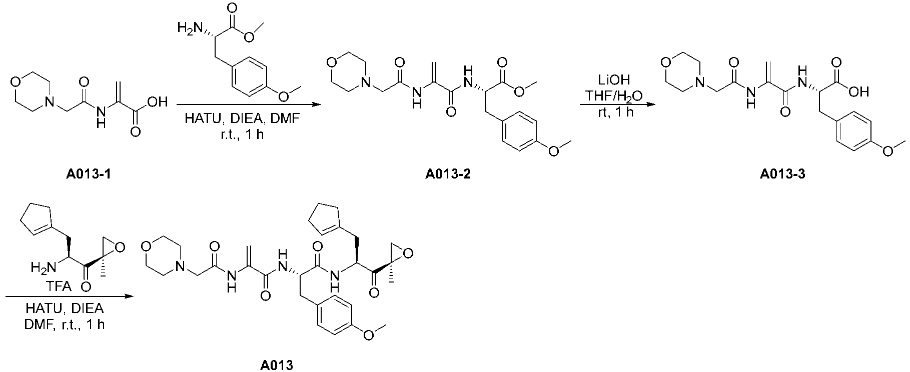

By replacing Intermediate A001-1 in Example 1 with A013-1, following a similar method, *N*-[(1*S*)-1-([(2*S*)-3-(cyclopent-1-en-1-yl)-1-[(2*R*)-2-methyloxiran-2-yl]-1-oxoprop-2-yl]carbamyl-2-(4-methoxyphenyl)ethyl]-2-[2-(morpholine-4-yl)acetamido]prop-2-enylamine (24.0 mg, 20.57%) as a white solid was obtained.

LCMS-A013: (ESI, *m*/*z):* 569.05 [M+H]⁺.

¹H NMR-A013 (400 MHz, Chloroform-d) *δ* 9.46 (s, 1H), 7.18 (d, *J* = 8.1 Hz, 2H), 6.84 (d, *J* = 8.2 Hz, 3H), 6.36 (s, 1H), 5.99 (s, 1H), 5.26 (d, *J* = 19.0 Hz, 2H), 4.67 - 4.46 (m, 2H), 3.86 (s, 4H), 3.79 (s, 3H), 3.38 (s, 2H), 3.25 (d, *J* = 5.0 Hz, 1H), 3.10 (dd, *J* = 14.0, 5.5 Hz, 1H), 3.01 - 2.94 (m, 1H), 2.90 (d, *J* = 5.0 Hz, 1H), 2.84 (s, 4H), 2.50 (d, *J* = 14.2 Hz, 1H), 2.21 *(t, J* = 4.8 Hz, 3H), 2.18 - 2.11 (m, 2H), 1.81 (dd, *J =* 12.1, 6.8 Hz, 2H), 1.51 (s, 3H).

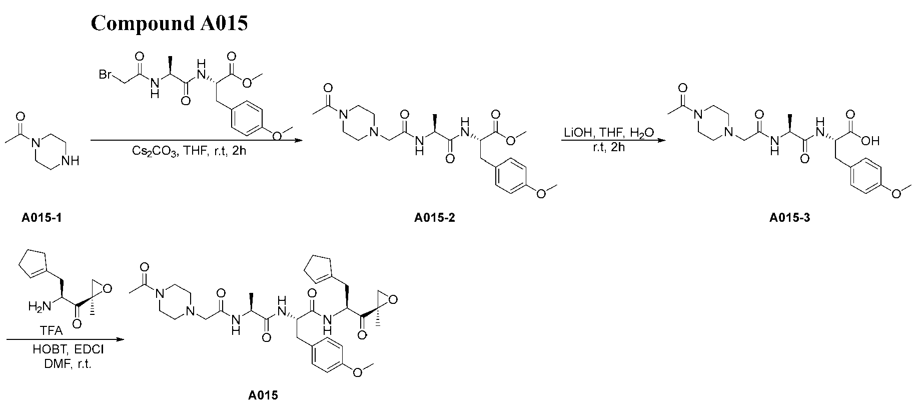

**Step 1:** At room temperature, to a solution of 1-(piperazinyl)ethanone (90 mg, 0.702 mmol, 1 eq) in tetrahydrofuran (2 mL), methyl (2*S*)-2-[(2*S*)-2-(2-bromoacetamido)propionamido]-3-(4-methoxyphenyl)propionate (prepared by a synthesis method similar to that for Compound A042-3, 338.1 mg, 0.842 mmol, 1.2 eq) and cesium carbonate (686.34 mg, 2.106 mmol, 3 eq) were added. The reaction solution was continuously stirred for 2 hrs. The reaction mixture was quenched with water at room temperature, and extracted with ethyl acetate (3 x 50 mL). The organic phases were combined, back washed with saturated brine (1 x 50 mL), and dried over anhydrous sodium sulfate. After the resulting mixture was filtered, the filtrate was concentrated under reduced pressure. The obtained residue was purified by reverse-phase column chromatography to obtain methyl (2*S*)-2-[(2*S*)-2-[2-(4-acetylpiperazin-1-yl)acetamido]propionamido]-3-(4-methoxyphenyl)propionate (80 mg, 25.40%), as a white solid.

LCMS-A015-2: (ESI, *m*/*z):* 449.25, [M+H]⁺.

**Step 2:** At room temperature, lithium hydroxide (11.21 mg, 0.468 mmol, 3 eq) was added to a solution of methyl (2*S*)-2-[(2*S*)-2-[2-(4-acetylpiperazin-1-yl)acetamido]proplonamido]-3-(4-methoxyphenyl)propionate (70 mg, 0.156 mmol, 1 eq) in tetrahydrofuran (0.6 mL) and water (0.3 mL). The reaction solution was stirred at room temperature for 2 hrs, and acidified to pH 5-6 with 1 mol/L hydrochloric acid. The reaction mixture was extracted with ethyl acetate (3 x 20 mL). The organic phases were combined, back washed with saturated brine (1 x 20 mL), and dried over anhydrous sodium sulfate. After the resulting mixture was filtered, the filtrate was concentrated under reduced pressure, to obtain (2*S*)-2-[(2*S*)-2-[2-(4-acetylpiperazin-1-yl)acetamido]propionamido]-3-(4-methoxyphenyl)propionate (50 mg, 73.73%), as a white solid.

LCMS-A015-3: (ESI, *m*/*z):* 435.20, [M+H]⁺.

**Step 3:** Following a method similar to that in Step 3 of Example 1, (*S*)-2-((*S*)-2-(2-(4-acetylpiperazin-1-yl)acetamido)-*N*-((*S*)-3-(cyclopent-1-en-1-yl)-1-((*R*)-2-methyloxiran-2-yl)-1-oxoprop-2-yl)-3-(4-methoxyphenyl)propionamide (15.5 mg, 21.91%), as a white solid was obtained.

LCMS-A015: (ESI, *m*/*z):* 612.65 [M+H]⁺.

¹H NMR-A015 (400 MHz, Methanol-d4) *δ* 7.55 (s, 1H), 7.15 (d, *J* = 8.0 Hz, 2H), 6.81 (d, *J* = 8.0 Hz, 2H), 6.73 (s, 1H), 6.10 (s, 1H), 5.32 (s, 1H), 4.64 - 4.48 (m, 2H), 4.43 (d, *J* = 9.2 Hz, 1H), 3.78 (s, 4H), 3.52 (s, 3H), 3.25 (d, *J* = 5.0 Hz, 1H), 3.22 - 3.01 (m, 2H), 3.00 - 2.93 (m, 2H), 2.90 (d, *J* = 5.0 Hz, 1H), 2.67 - 2.51 (m, 4H), 2.48 (s, 1H), 2.23 (d, *J =* 8.1 Hz, 3H), 2.17 (d, *J =* 8.1 Hz, 2H), 2.10 (s, 3H), 1.90 - 1.75 (m, 2H), 1.50 (s, 3H),1.39 - 1.30 (m, 3H).

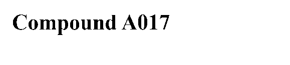

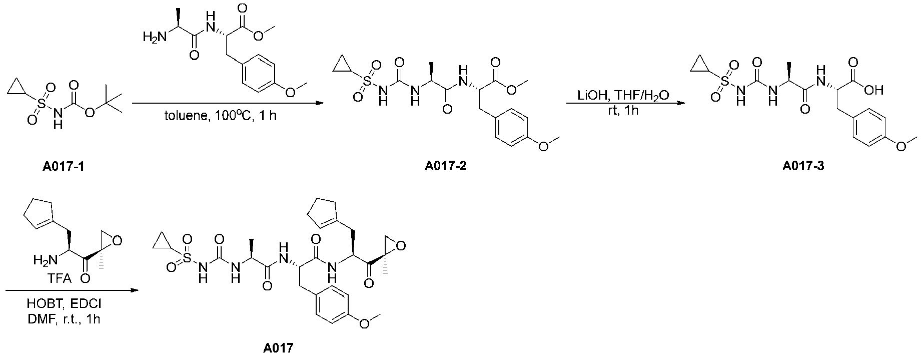

**Step 1:** Under a nitrogen atmosphere and at room temperature, methyl (2*S*)-2-[(2*S*)-2-aminopropionamido]-3-(4-methoxyphenyl)propionate (152.03 mg, 0.542 mmol, 1.2 eq) was added to a solution of t-butyl N-(cyclopropylsulfonyl)carbamate (100 mg, 0.452 mmol, 1 eq) in toluene (2 mL). The reaction solution was heated to 100°C, and stirred for 1 hr. The reaction solution was cooled to room temperature, and concentrated under reduced pressure. The obtained residue was purified by reverse-phase column chromatography to obtain methyl (2*S*)-2-[(2*S*)-2-[(cyclopropylsulfonyl carbamyl)amino]propionamido]-3-(4-methoxyphenyl)propionate (90 mg, 46.59%) as a white solid.

LCMS-A017-2: (ESI, *m*/*z):* 428.05, [M+H]⁺.

**Step 2:** At room temperature, lithium hydroxide (13.45 mg, 0.561 mmol, 3 eq) was added to a solution of methyl (2*S*)-2-[(2*S*)-2-[(cyclopropylsulfonyl carbamyl)amino]propionamido]-3-(4-methoxyphenyl)propionate (80 mg, 0.187 mmol, 1 eq) in tetrahydrofuran (0.6 mL) and water (0.3 mL). The reaction solution was stirred at room temperature for 1 hr. The reaction mixture was acidified to pH 6 with 1 mol/L hydrochloric acid and concentrated under reduced pressure, to obtain (2S)-2-[(2S)-2-[(cyclopropylsulfonyl carbamyl)amino]propionamido]-3-(4-methoxyphenyl)propionic acid (80 mg, crude product) as a white solid.

LCMS-A017-3: (ESI, *m*/*z):* 414.00, [M+H]⁺.

**Step 3:** Following the method similar to that in Step 3 of Example 1, (2*S*)-*N*-[(2*S*)-3-(cyclopent-1-en-1-yl)-1-[(2*R*)-2-methyloxiran-2-yl]-1-oxoprop-2-yl]-2-[(2*S*)-2-[(cyclopropylsulfonylcarbamyl)amino] propionamide]-3-(4-methoxyphenyl) propionamide (12.5 mg, 8.77%) as a white solid was obtained.

LCMS-A017: (ESI, *m*/*z):* 591.05 [M+H]⁺.

¹H NMR-A017 (400 MHz, Methanol-d4) *δ* 9.21 (s, 1H), 7.50 (s, 1H), 7.20 (d, *J* = 51.0 Hz, 1H), 7.06 (d, *J* = 8.2 Hz, 2H), 6.99 (s, 1H), 6.91 - 6.81 (m, 1H), 6.79 - 6.68 (m, 2H), 5.36 (s, 1H), 4.86 - 4.74 (m, 1H), 4.68 - 4.52 (m, 2H), 3.73 (s, 3H), 3.19 (d, *J* = 4.9 Hz, 1H), 3.02 - 2.96 (m, 1H), 2.96 - 2.90 (m, 1H), 2.86 (d, *J* = 4.9 Hz, 1H), 2.58 - 2.49 (m, 1H), 2.35 - 2.28 (m, 1H), 2.19 *(t, J* = 7.4 Hz, 4H), 1.84 - 1.72 (m, 2H), 1.50 (s, 3H), 1.40 - 1.24 (m, 5H), 1.18 - 1.04 (m, 2H).

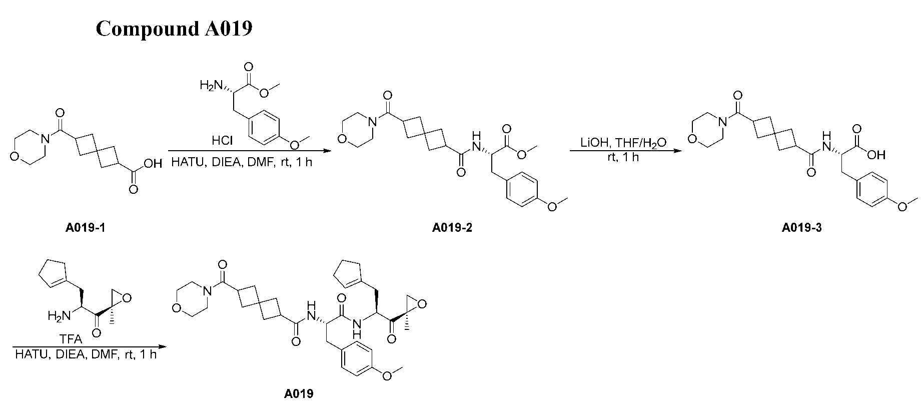

By replacing Intermediate A001-1 in Example 1 with A019-1, following a similar method, (2*S*)-*N*-[(2*S*)-3-(cyclopent-1-en-1-yl)-1-[(2*R*)-2-methyloxiran-2-yl]-1-oxoprop-2-yl]-3-(4-methoxyphenyl)-2-{[6-(morpholine-4-carbonyl)spiro[3.3]hept-2-yl]formamide (16.0 mg, 22.58%) as an orange solid was obtained.

LCMS-A019: (ESI, *m*/*z*): 608.15[M+H]⁺.

¹H NMR-A019 (400 MHz, Chloroform-*d*, *ppm*) *δ* 7.21 -7.10 (m, 2H), 6.87 - 6.78 (m, 2H), 5.92 (t, *J* = 7.4 Hz, 1H), 5.83 (t, *J* = 6.1 Hz, 1H), 5.27 (s, 1H), 4.59 - 4.44 (m, 2H), 3.79 (d, *J* = 2.9 Hz, 3H), 3.68 - 3.53 (m, 6H), 3.33 (t, *J* = 4.8 Hz, 2H), 3.28 - 3.21 (m, 1H), 3.13 - 2.96 (m, 2H), 2.94 - 2.76 (m, 3H), 2.53 - 2.38 (m, 2H), 2.31 - 2.18 (m, 6H), 2.17 - 2.05 (m, 6H), 1.88 - 1.74 (m, 2H), 1.50 (s, 3H).

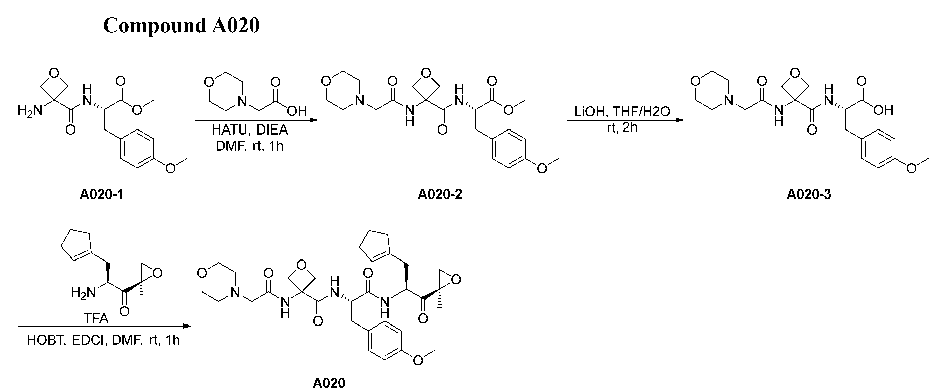

**Step 1:** Under a nitrogen atmosphere and at room temperature, to a solution of methyl (*S*)-2-(3-aminooxacyclobutan-3-formamido)-3-(4-methoxyphenyl)propionate (600 mg, 1.946 mmol, 1 eq), *N*,*N*,*N'*,*N'*-tetramethyl-*O*-(7-azabenzotriazol-1-yl)uronium hexafluorophosphate (1.48 g, 3.892 mmol, 2 eq), and *N*,*N*-diisopropylethyl amine (1.26 g, 9.730 mmol, 5 eq) in *N*,*N*-dimethyl formamide (20.0 mL), 2-morpholinacetic acid (310.72 mg, 2.141 mmol, 1.1 eq) was added batchwise. After that, the system was further stirred at room temperature for 1 hr. The reaction mixture was quenched with water (60 mL) at room temperature, and extracted with ethyl acetate (3 x 50 mL). The organic phases were combined, back washed with saturated brine (1 x 30 mL), and dried over anhydrous sodium sulfate. After the resulting mixture was filtered, the filtrate was concentrated under reduced pressure. The obtained residue was purified by reverse-phase column chromatography to obtain methyl (*S*)-3-(4-methoxyphenyl)-2-(3-(2-morpholinoacetamido)oxacyclobutan-3-formamido)propionate (300 mg, 35.40%) as a colorless and transparent oil.

LCMS-A020-2: (ESI, *m*/*z*): 436.10 [M+H]⁺.

**Step 2:** At room temperature, the mixed solution of methyl (*S*)-3-(4-methoxyphenyl)-2-(3-(2-morpholinoacetamido)oxacyclobutan-3-formamido)propionate (260 mg, 0.597 mmol, 1 eq) and lithium hydroxide (28.6 mg, 1.194 mmol, 2 eq) in tetrahydrofuran (3.0 mL) and water (3.0 mL) was reacted with stirring for 2 hrs. The reaction solution was adjusted to pH 5-6 with 1 mol/L dilute hydrochloric acid, and concentrated under vacuum, to obtain (*S*)-3-(4-methoxyphenyl)-2-(3-(2-morpholinoacetamido)oxacyclobutan-3-formamido)propionate (300 mg, crude product). The crude product was directly used in the next step without further purification.

LCMS-A020-3: (ESI, *m*/*z):* 422.15 [M+H]⁺.

**Step 3:** Following a method similar to that in Step 3 of Example 1, N-((*S*)-1-(((*S*)-3-(cyclopent-1-en-1-yl)-1-((*R*)-2-methyloxiran-2-yl)-1-oxoprop-2-yl)amino)-3-(4-methoxyphenyl)-1-oxoprop-2-yl)-3-(2-morpholinoacetamido)oxacyclobutan-3-formamide (30.3 mg, 21.18%) as a light yellow solid was prepared.

LCMS-A020: (ESI, *m*/*z):* 599.20 [M+H]⁺.

¹H NMR-A020: (400 MHz, Chloroform-d) *δ* 7.95 (s, 1H), 7.33 (s, 1H), 7.20 - 7.12 (m, 2H), 6.87 - 6.77 (m, 2H), 6.27 (dd, *J =* 108.5, 6.9 Hz, 1H), 5.42 - 5.30 (m, 1H), 4.81 (s, 4H), 4.57 (p, *J* = 7.7 Hz, 2H), 3.77 (d, *J = 3.0* Hz, 7H), 3.37 - 3.24 (m, 1H), 3.17 - 2.91 (m, 4H), 2.89 (dd, *J* = 5.0, 1.3 Hz, 1H), 2.50 (d, *J* = 16.8 Hz, 5H), 2.32 - 2.10 (m, 5H), 1.83 (dt, *J* = 13.8, 7.0 Hz, 2H), 1.49 (d, *J* = 4.4 Hz, 3H).

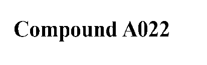

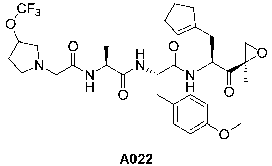

Following the synthesis method for A042, by replacing *N*-(t-butoxycarbonyl)-*O*-methyl-L-serine with *N*-(t-butoxycarbonyl) -L-serine and replacing 4-oxa-7-azaspiro[2.5]octane hydrochloride with 3-(trifluoromethoxy)pyrrolidine, (2*S*)-*N*-[(2*S*)-3-(cyclopent-1-en-1-yl)-1-[(2*R*)-2-methyloxiran-2-yl]-1-oxoprop-2-yl]-3-(4-methoxyphenyl)-2-[(2*S*)-2-(2-[3-(trifluoromethoxy)pyrrolidin-1-yl]acetamido propionamide (40.7 mg, 29.35%) as a white solid was obtained.

LCMS-A022: (ESI, *m*/*z):* 639.20 [M+H]⁺.

¹H NMR-A022 (400 MHz, DMSO-*d*₆) *δ* 8.31 - 8.18 (m, 1H), 8.03 - 7.93 (m, 1H), 7.70 (d, *J* = 7.7 Hz, 1H), 7.10 (d, *J* = 8.5 Hz, 2H), 6.82 - 6.69 (m, 2H), 5.39 (s, 1H), 5.02 - 4.82 (m, 1H), 4.55 - 4.40 (m, 2H), 4.27 (h, *J* = 6.6 Hz, 1H), 3.70 (s, 3H), 3.17 (d, *J* = 5.3 Hz, 1H), 3.09 - 3.03 (m, 2H), 2.98 (d, *J* = 5.3 Hz, 1H), 2.90 - 2.73 (m, 4H), 2.69 - 2.61 (m, 1H), 2.45 - 2.37 (m, 2H), 2.27 - 2.16 (m, 6H), 1.87 (s, 1H), 1.83 - 1.73 (m, 2H), 1.37 (s, 3H), 1.18 - 1.10 (m, 3H).

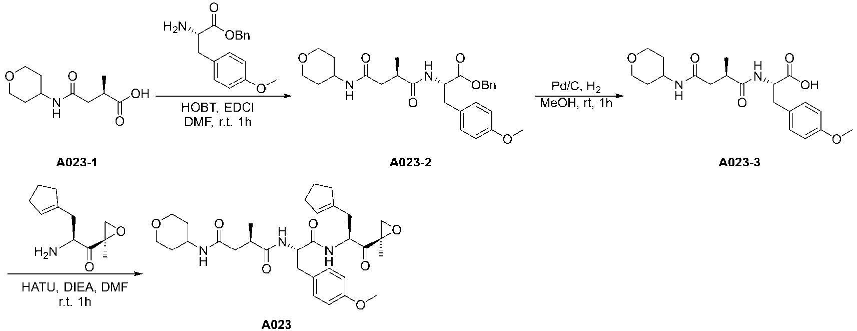

By replacing Intermediate A001-1 in Example 1 with A023-1, following a similar method, (*R*)*-N*¹-((*S*)-1-(((*S*)-3-(cyclopent-1-en-1-yl)-1-((*R*)-2-methyloxiran-2-yl)-1-oxoprop-2-yl)amino)-3-(4-methoxyphenyl)-1-oxoprop-2-yl)-2-methyl-*N*⁴-(tetrahydro-2*H*-pyran-4-yl)succinamide (12.3 mg, 16.88%) as a white solid was obtained.

LCMS-A023: (ESI, *m*/*z):* 570.15 [M+H]⁺.

¹H NMR-A023: (400 MHz, Chloroform-d) *δ* 7.17 (d, *J* = 8.3 Hz, 2H), 6.91 - 6.77 (m, 2H), 6.47 (d, *J* = 7.6 Hz, 1H), 6.08 (d, *J* = 6.9 Hz, 1H), 5.66 (d, *J* = 8.0 Hz, 1H), 5.30 (d, *J* = 17.6 Hz, 1H), 4.52 (dt, *J* = 14.2, 7.9 Hz, 2H), 3.93 (d, *J* = 9.0 Hz, 3H), 3.78 (d, *J* = 3.0 Hz, 3H), 3.53 - 3.39 (m, 2H), 3.27 (d, *J =* 5.0 Hz, 1H), 3.03 (dd, *J* = 14.0, 6.0 Hz, 1H), 2.99 - 2.85 (m, 2H), 2.82 - 2.67 (m, 1H), 2.44 (dq, *J* = 14.7, 7.2, 6.2 Hz, 2H), 2.33 - 2.04 (m, 6H), 1.95 - 1.74 (m, 4H), 1.49 (s, 3H), 1.42 (td, *J* = 12.3, 11.6, 4.5 Hz, 2H), 1.16 (d, *J* = 7.0 Hz, 3H).

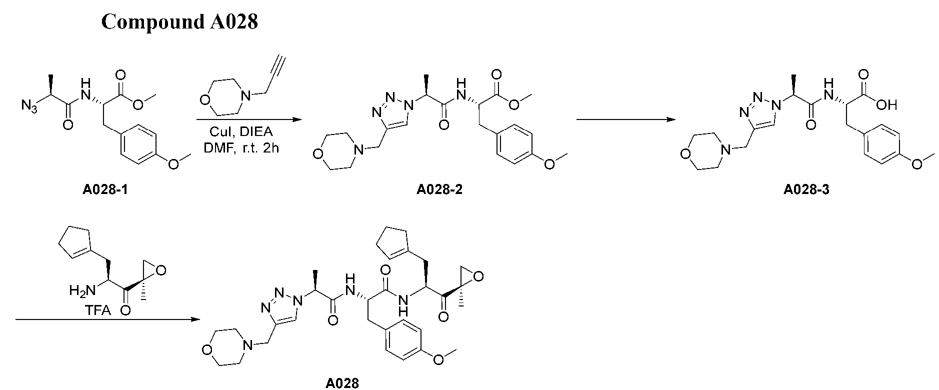

**Step 1:** Under a nitrogen atmosphere and at room temperature, to a solution of methyl (*S*)-2-((*S*)-2-azidopropionamido)-3-(4-methoxyphenyl)propionate (136 mg, 0.444 mmol, 1 eq) and cuprous iodide (8.46 mg, 0.044 mmol, 0.1 eq) in *N*,*N*-dimethyl formamide (2.0 mL), 4-(prop-2-yn-1-yl)morpholine(66.69 mg, 0.533 mmol, 1.20 eq) and *N*,*N*-diisopropylethyl amine (114.77 mg, 0.888 mmol, 2.00 eq) were added dropwise. After that, the system was further stirred at room temperature for 2 hrs. The reaction mixture was quenched with water (30 mL), and extracted with ethyl acetate (3 x 30 mL). The organic phases were combined, back washed with saturated brine (1 x 30 mL), and dried over anhydrous sodium sulfate. After the resulting mixture was filtered, the filtrate was concentrated under reduced pressure. The obtained residue was purified by reverse-phase column chromatography to obtain methyl (*S*)-3-(4-methoxyphenyl)-2-((*S*)-2-(4-(morpholinemethyl)-1*H*-1,2,3-triazol-1-yl)propionamido)propionate (95 mg, 49.59%), as a yellow oil.

LCMS-A028-2: (ESI, *m*/*z):* 431.90 [M+H]⁺.

**Step 2:** At room temperature, to a mixed solution of methyl (*S*)-3-(4-methoxyphenyl)-2-((*S*)-2-(4-(morpholinemethyl)-1*H*-1,2,3-triazol-1-yl)propionamido)propionate (80 mg, 0.185 mmol, 1 eq) and lithium hydroxide (8.88 mg, 0.370 mmol, 2 eq) in tetrahydrofuran (1.0 mL) and water (1.0 mL), hydrogen peroxide (19 uL, 0.244 mmol, 8 eq, 30%) was added dropwise. After that, the system was further stirred at room temperature for 1 hr. The reaction solution was adjusted to pH 5-6 with 1 mol/L dilute hydrochloric acid, and the mixture was concentrated under vacuum, to obtain (*S*)-3-(4-methoxyphenyl)-2-((*S*)-2-(4-(morpholinemethyl)-1*H*-1,2,3-triazol-1-yl)propionamido)propionate (120 mg, crude product). The crude product was directly used in the next step without further purification.

LCMS-A028-3: (ESI, *m*/*z):* 418.15 [M+H]⁺.

Step 3: Using a method similar to that in Step 3 of Example 1, the compound (*S*)-*N*-((*S*)-3-(cyclopent-1-en-1-yl)-1-((*R*)-2-methyloxiran-2-yl)-1-oxoprop-2-yl)-3-(4-methoxyphenyl)-2-((*S*)-2-(4-(morpholinemethyl)-1*H*-1,2,3-triazol-1-yl) propionamide(25.9 mg, 18.16%) as a white solid was obtained.

LCMS-A028: (ESI, *m*/*z):* 595.25 [M+H]⁺.

¹H NMR-A028: (400 MHz, Chloroform-*d*) *δ* 7.78 - 7.54 (m, 1H), 7.14 - 7.02 (m, 2H), 6.88 - 6.74 (m, 2H), 6.55 (dd, *J* = 15.8, 7.3 Hz, 1H), 5.79 (d, *J* = 6.9 Hz, 1H), 5.33 - 5.15 (m, 2H), 4.57 - 4.41 (m, 2H), 3.79 (d, *J* = 1.4 Hz, 3H), 3.73 (s, 5H), 3.23 (t, *J* = 4.6 Hz, 1H), 3.03 - 2.83 (m, 3H), 2.56 (s, 3H), 2.48 (d, *J* = 14.7 Hz, 1H), 2.21 (t, *J* = 7.8 Hz, 2H), 2.18 - 2.06 (m, 3H), 1.78 (td, *J =* 12.4, 8.0 Hz, 5H), 1.49 (s, 3H).

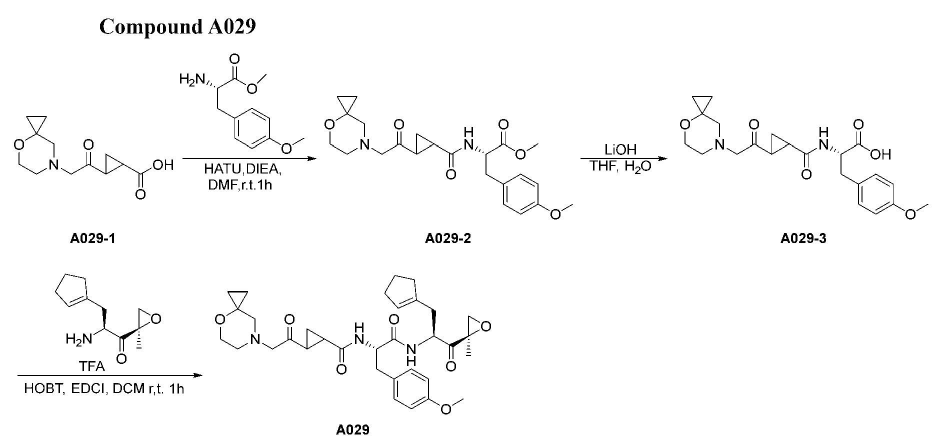

By replacing Intermediate A001-1 in Example 1 with A029-1, following a similar method, (2*S*)-*N*-[(2*S*)-3-(cyclopent-1-en-1-yl)-1-[(2*R*)-2-methyloxiran-2-yl]-1-oxoprop-2-yl]-3-(4-methoxyphenyl)-2-{[2-(2-{4-oxa-7-azaspiro[2.5]octan-7-yl}acetyl)cyclopropyl]formamide (3.3 mg, 2.25%) as a white solid was obtained.

LCMS-A029: (ESI, *m*/*z):* 594.65 [M+H]⁺.

¹H NMR-A029 (400 MHz, Chloroform-*d*) *δ* 7.40 (s, 1H), 7.23 - 7.08 (m, 2H), 6.82 (d, *J* = 7.3 Hz, 2H), 5.51 - 5.20 (m, 1H), 5.08 - 4.82 (m, 1H), 4.54 (s, 1H), 3.96 (d, *J* = 78.5 Hz, 4H), 3.79 (d, *J* = 6.0 Hz, 3H), 3.62 - 3.08 (m, 5H), 3.06 - 2.82 (m, 3H), 2.75 (s, 4H), 2.30 - 2.13 (m, 4H), 1.83 (d, *J* = 8.6 Hz, 2H), 1.47 (d, *J* = 14.7 Hz, 3H), 1.05 (d, *J* = 16.2 Hz, 2H), 0.85 (s, 2H).

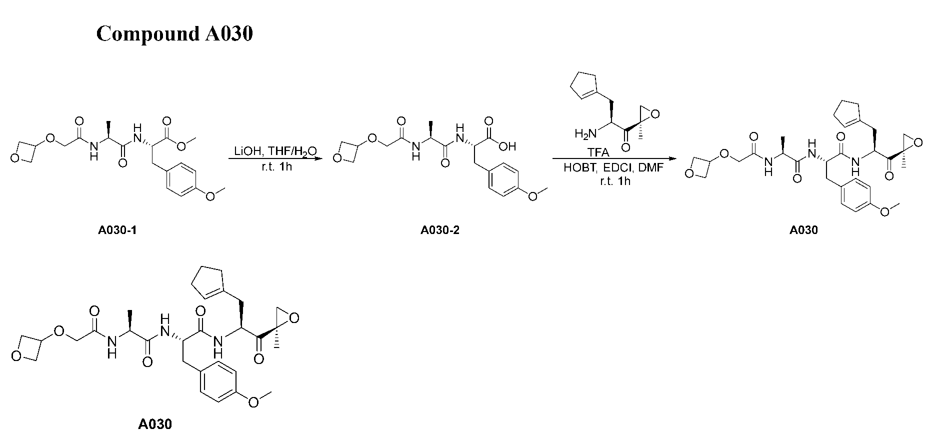

Following the synthesis method for A042, by replacing *N*-(t-butoxycarbonyl)-*O*-methyl-L-serine with *N*-(t-butoxycarbonyl) -L-serine and replacing 4-oxa-7-azaspiro[2.5]octane hydrochloride with 3-oxacyclobutanol, the compound (*S*)-*N*-((*S*)-3-(cyclopent-1-en-1-yl)-1-((*R*)-2-methyloxiran-2-yl)-1-oxoprop-2-yl)-3-(4-methoxyphenyl)-2-((*S*)-2-(2-(oxacyclobutan-3-yloxy)acetamido) propionamide(7.8 mg, 8.87%) as a white solid was obtained.

LCMS-A030: (ESI, *m*/*z):* 558.15 [M+H]⁺.

¹H NMR-A030 (400 MHz, Chloroform-*d*) *δ* 7.22 - 7.12 (m, 1H), 7.11 - 7.04 (m, 1H), 6.99 - 6.87 (m, 1H), 6.87 - 6.77 (m, 2H), 6.70 - 6.47 (m, 1H), 6.18 (dd, *J* = 143.5, 6.9 Hz, 1H), 5.44 - 5.28 (m, 1H), 4.85 - 4.29 (m, 7H), 3.98 - 3.70 (m, 5H), 3.42 - 3.21 (m, 1H), 3.11 - 2.86 (m, 3H), 2.50 (d, J = 13.6 Hz, 1H), 2.35 - 2.20 (m, 3H), 2.15 (d, *J* = 8.5 Hz, 2H), 1.84 (dt, *J* = 12.5, 4.7 Hz, 2H), 1.48 (s, 1H), 1.44 - 1.21 (m, 6H).

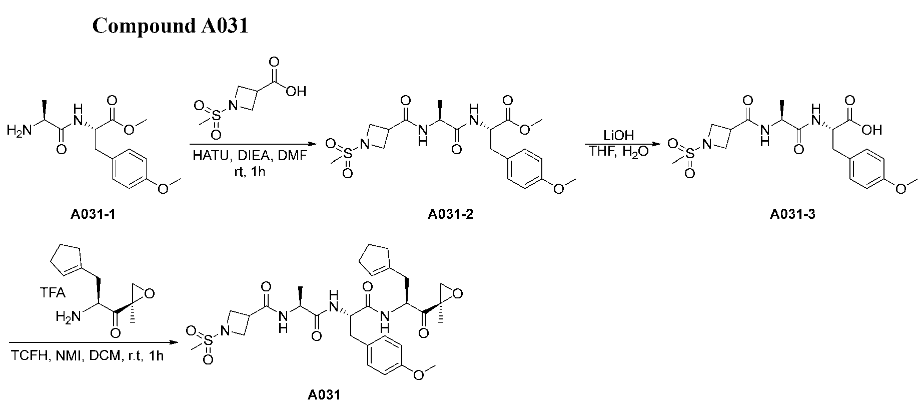

**Step 1:** Under a nitrogen atmosphere and at room temperature, to a solution of methyl (2S)-2-[(2*S*)-2-aminopropionamido]-3-(4-methoxyphenyl)propionate (210 mg, 0.749 mmol, 1 eq) in *N*,*N*-dimethyl formamide (4 mL), 1-methylsulfonylazetidin-3-carboxylic acid (161.08 mg, 0.899 mmol, 1.2 eq), 1-hydroxybenzotriazole (202.45 mg, 1.498 mmol, 2 eq) and 1-(3-dimethylaminopropyl)-3-ethylcarbodiimide hydrochloride (574.44 mg, 2.996 mmol, 4 eq) were added. The reaction solution was stirred at room temperature for 1 hr. The reaction mixture was quenched with water at room temperature, and extracted with ethyl acetate (3 x 20 mL). The organic phases were combined, back washed with a saturated sodium chloride solution (2 x 20 mL), and dried over anhydrous sodium sulfate. After the resulting mixture was filtered, the filtrate was concentrated under reduced pressure, The resulting crude product was purified by reverse-phase column chromatography, to obtain methyl (2*S*)-2-[(2*S*)-2-[(1-methansulfonyl azetidin-3-yl)formamido]propionamido]-3-(4-methoxyphenyl)propionate (150 mg, 45.35%) as a white solid was obtained.

LCMS-A031-2: (ESI, *m*/*z):* 441.80, [M+H]⁺.

**Step 2:** At room temperature, lithium hydroxide (24.41 mg, 1.020 mmol, 3 eq) was added to a solution of methyl (2S*)*-2-[(2*S*)-2-[(1-methylsulfonyl azetidin-3-yl)formamido]propionamido]-3-(4-methoxyphenyl)propionate (150 mg, 0.340 mmol, 1 eq) in tetrahydrofuran (1 mL) and water (0.5 mL). The reaction solution was stirred at room temperature for 1 hr. The reaction mixture was acidified to pH 5-6 with hydrochloric acid, and extracted with ethyl acetate (3 x 20 mL). The organic phases were combined, back washed with saturated brine (1 x 30 mL), and dried over anhydrous sodium sulfate. After the resulting mixture was filtered, the filtrate was concentrated under reduced pressure, to obtain crude (2*S*)-2-[(2*S*)-2-[(1-methylsulfonyl azetidin-3-yl)formamido]propionamido]-3-(4-methoxyphenyl)propionic acid (150 mg, crude product). as a white solid was obtained.

LCMS-A031-3: (ESI, *m*/*z):* 428.00, [M+H]⁺.

**Step 3:** Using a method similar to that in Step 3 of Example 1, the compound (2*S*)-*N*-[(2*S*)-3-(cyclopent-1-en-1-yl)-1-[(2*R*)-2-methyloxiran-2-yl]-1-oxoprop-2-yl]-2-[(2*S*)-2-[(1-methansulfonylazetidin-3-yl)formamido] propionamide]-3-(4-methoxyphenyl) propionamide(4.0 mg, 2.69%) as a white solid was obtained.

LCMS-A031: (ESI, *m*/*z):* 605.60 [M+H]⁺.

¹H NMR-A031 (400 MHz, Chloroform-d) *δ* 7.16 (d, *J =* 8.3 Hz, 2H), 6.86 - 6.79 (m, 2H), 6.52 (d, *J* = 7.4 Hz, 1H), 6.23 (d, *J* = 7.2 Hz, 1H), 5.83 (d, *J* = 6.9 Hz, 1H), 5.26 (s, 1H), 4.57 - 4.47 (m, 2H), 4.42 (t, *J* = 7.0 Hz, 1H), 4.14 - 4.02 (m, 4H), 3.79 (s, 3H), 3.23 (t, *J* = 6.9 Hz, 2H), 3.07 - 2.99 (m, 1H), 2.94 (s, 3H), 2.92 - 2.84 (m, 2H), 2.49 (d, *J =* 14.4 Hz, 1H), 2.21 (d, *J* = 9.8 Hz, 2H), 2.18 - 2.05 (m, 3H), 1.90 - 1.76 (m, 2H), 1.50 (s, 3H), 1.37 (d, *J* = 7.0 Hz, 3H).

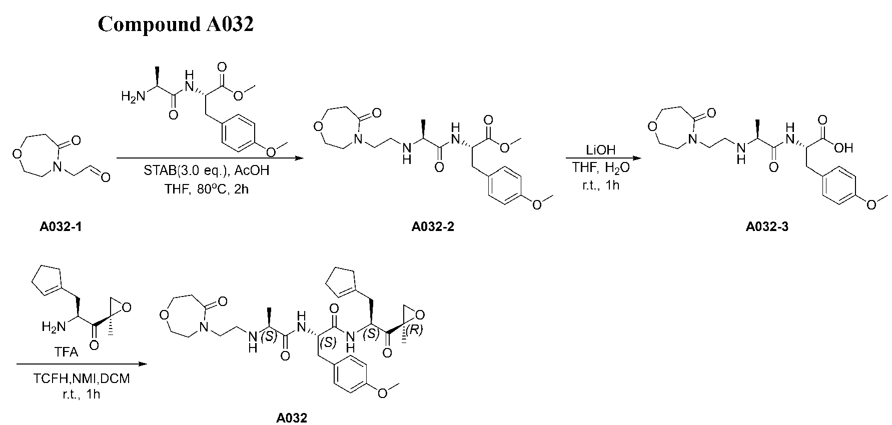

**Step 1:** Under a nitrogen atmosphere and at room temperature, to a solution of 2-(5-oxo-1,4-oxaaza-4-yl)acetaldehyde (400 mg, 2.545 mmol, 1 eq) and methyl (*S*)-2-((*S*)-2-aminopropionamido)-3-(4-methoxyphenyl)propionate (713.43 mg, 2.545 mmol, 1 eq) in tetrahydrofuran (20.0 mL), sodium triacetoxyborohydride (1.62 g, 7.635 mmol, 3 eq) and acetic acid (458.5 mg, 7.635 mmol, 3 eq) were added batchwise. After that, the system was heated to 80°C and further stirred for 16 hrs. The reaction mixture was diluted with water (60 mL), and extracted with ethyl acetate (3 x 50 mL). The organic phases were combined, back washed with saturated brine (1 x 60 mL), and dried over anhydrous sodium sulfate. After the resulting mixture was filtered, the filtrate was concentrated under reduced pressure. The crude product was purified by high performance liquid chromatography to obtain methyl (*S*)-3-(4-methoxyphenyl)-2-((*S*)-2-((2-(5-oxo-1,4-oxaaza-4-yl)ethyl)amino)propionamido)propionate (150 mg, 13.98%) as a white solid was obtained.

LCMS-A032-2: (ESI, *m*/*z):* 422.50 [M+H]⁺.

**Step 2:** At room temperature, a mixed solution of methyl (*S*)-3-(4-methoxyphenyl)-2-((*S*)-2-((2-(5-oxo-1,4-oxaaza-4-yl)ethyl)amino)propionamido)propionate (90 mg, 0.214 mmol, 1 eq) and lithium hydroxide (10.23 mg, 0.428 mmol, 2 eq) in tetrahydrofuran (0.5 mL) and water (0.5 mL) was reacted with stirring for 1 hr. The reaction solution was adjusted to pH 5-6 with 1mol/L dilute hydrochloric acid, and concentrated under vacuum, to obtain (*S*)-3-(4-methoxyphenyl)-2-((*S*)-2-((2-(5-oxo-1,4-oxaaza-4-yl)ethyl)amino)propionamido)propionic acid (100 mg, crude product). The crude product was directly used in the next step without further purification.

LCMS-A032-3: (ESI, *m*/*z):* 408.15 [M+H]⁺.

**Step 3:** Using a method similar to that in Step 3 of Example 1, the compound (*S*)-*N*-((*S*)-3-(cyclopent-1-en-1-yl)-1-((*R*)-2-methyloxiran-2-yl)-1-oxoprop-2-yl)-3-(4-methoxyphenyl)-2-((*S*)-2-((2-(5-oxo-1,4-oxaaza-4-yl)ethyl)amino)propionamide formate (6.7 mg, 8.41%) as a white solid was obtained.

LCMS-A032: (ESI, *m*/*z):* 584.95 [M+H]⁺.

¹H NMR-A032: (400 MHz, Chloroform-d) *δ* 8.24 (s, 1H), 7.91 (d, *J* = 8.3 Hz, 1H), 7.16 (d, J = 8.1 Hz, 2H), 6.82 (d, *J* = 8.0 Hz, 2H), 6.37 (d, *J* = 7.0 Hz, 1H), 5.36 (s, 1H), 4.57 (dq, *J* = 8.1, 4.7 Hz, 2H), 3.77 (d, *J* = 5.4 Hz, 5H), 3.75 - 3.65 (m, 2H), 3.65 - 3.34 (m, 6H), 3.26 (d, *J* = 5.0 Hz, 1H), 2.99 (td, *J =* 13.7, 7.1 Hz, 2H), 2.89 (d, *J* = 5.0 Hz, 1H), 2.77 (dd, *J* = 6.5, 3.2 Hz, 3H), 2.61 (s, 1H), 2.51 (d, *J* = 14.8 Hz, 1H), 2.31 - 2.10 (m, 5H), 1.83 (p, *J* = 7.5 Hz, 2H), 1.49 (s, 3H), 1.26 (d, *J* = 4.6 Hz, 3H).

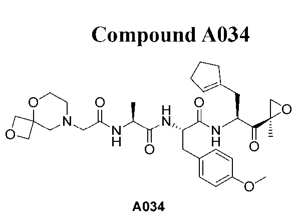

Following the synthesis method for A042, by replacing *N*-(t-butoxycarbonyl)-*O*-methyl-L-serine with N-(t-butoxycarbonyl) -L-serine and replacing 4-oxa-7-azaspiro[2.5]octane hydrochloride with 2,5-dioxa-8-azaspiro[3.5]nonane, the compound (*S*)-2-((*S*)-2-(2-(2, 5-dioxa-8-azaspiro[3.5]non-8-ylacetamido)-*N*-((*S*)-3-(cyclopent-1-en-1-yl)-1-((*R*)-2-methyloxiran-2-yl)-1-oxoprop-2-yl)-3-(4-methoxyphenyl)propionamide (1.13 mg, 15.97%) as a white solid was obtained.

LCMS-A034: (ESI, *m*/*z):* 613.20, [M+H]⁺.

¹H NMR-A034: (400 MHz, Chloroform-*d*) *δ* 7.40 (s, 1H), 7.19 (d, *J* = 24.7 Hz, 2H), 6.95 - 6.68 (m, 2H), 6.58 (s, 1H), 5.98 (s, 1H), 5.31 (s, 1H), 4.95 - 4.05 (m, 7H), 3.80 (d, *J =* 12.4 Hz, 3H), 3.68 (s, 2H), 3.27 (s, 1H), 2.97 (s, 5H), 2.72 (d, *J =* 26.3 Hz, 2H), 2.59 - 2.32 (m, 3H), 2.20 (d, *J =* 30.2 Hz, 5H), 1.83 (s, 2H), 1.56 (s, 2H), 1.36 (s, 4H).

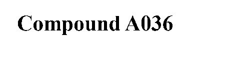

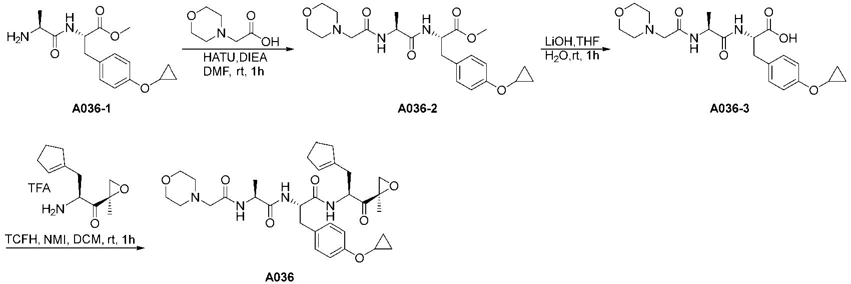

**Step 1:** Under a nitrogen atmosphere and at room temperature, to a solution of morpholin-4-ylacetic acid (106.61 mg, 0.735 mmol, 1.5 eq) and *N,N,N',N*'-tetramethyl-O-(7-azabenzotriazol-1-yl)uronium hexafluorophosphate (372.34 mg, 0.980 mmol, 2 eq) in *N*,*N*-dimethyl formamide (3 mL), *N,N-*diisopropylethyl amine (189.84 mg, 1.470 mmol, 3 eq) was added dropwise. After reaction with stirring for 5 min, methyl (S)-2-((S)-2-aminopropionamido)-3-(4-cyclopropyloxyphenyl)propionate (150 mg, 0.490 mmol, 1 eq) was added. The reaction solution was reacted with stirring at room temperature for 1 hr. The reaction mixture was diluted with water (15 mL), and extracted with ethyl acetate (3 x 15 mL). The organic phases were combined, back washed with saturated brine (1 x 15 mL), and dried over anhydrous sodium sulfate. After the resulting mixture was filtered, the filtrate was concentrated under reduced pressure. The obtained residue was purified by reverse-phase column chromatography to obtain methyl (S)-3-(4-cyclopropyloxyphenyl)-2-((*S*)-2-(2-morpholinoacetamido)propionamido)propionate (150 mg, 70.67%) as a white solid.

LCMS-A036-2: (ESI, *m*/*z):* 434.55 [M+H]⁺.

**Step 2:** At room temperature, to a solution of methyl (*S*)-3-(4-cyclopropyloxyphenyl)-2-((*S*)-2-(2-morpholinoacetamido)propionamido)propionate (140 mg, 0.323 mmol, 1 eq) in tetrahydrofuran/water (v/v=1/1, 2 mL), lithium hydroxide (15.47 mg, 0.646 mmol, 2 eq) was added. The reaction solution was reacted with stirring at room temperature for 1 hr. The reaction solution was acidified to pH 5-6 with 1 mol/L hydrochloric acid. The mixture was concentrated under reduced pressure, to obtain (*S*)-3-(4-cyclopropyloxyphenyl)-2-((S)-2-(2-morpholinoacetamido)propionamido)propionate (150 mg, crude product), as a white solid. The crude product was directly used in the next step without further purification.

LCMS-A036-3: (ESI, *m*/*z):* 418.30 [M+H]⁺.

**Step 3:** Using a method similar to that in Step 3 of Example 1, the compound (*S*)-*N*-((*S*)-3-(cyclopent-1-en-1-yl)-1-((*R*)-2-methyloxiran-2-yl)-1-oxoprop-2-yl)-3-(4-cyclopropyloxyphenyl)-2-((*S*)-2-(2-morpholinoacetamido)propionamide (9.4 mg, 11.23%) as a white solid was obtained.

LCMS-A036: (ESI, *m*/*z):* 597.00 [M+H]⁺.

¹H NMR-A036: (400 MHz, DMSO-*d*₆) *δ* 8.27 (d, *J* = 7.3 Hz, 1H), 8.04 (d, *J* = 8.4 Hz, 1H), 7.74 (d, *J* = 7.8 Hz, 1H), 7.16 - 7.04 (m, 2H), 6.94 - 6.83 (m, 2H), 5.39 (s, 1H), 4.54 - 4.39 (m, 2H), 4.35 - 4.23 (m, 1H), 3.79 - 3.68 (m, 1H), 3.55 (t, *J* = 4.6 Hz, 4H), 3.18 (d, *J* = 5.3 Hz, 1H), 2.98 (d, *J* = 5.2 Hz, 1H), 2.94 - 2.82 (m, 3H), 2.67 *(d, J* = 1.6 Hz, 1H), 2.39 - 2.32 (m, 5H), 2.27 - 2.17 (m, 5H), 1.84 - 1.74 (m, 2H), 1.38 (s, 3H), 1.15 (d, *J* = 7.0 Hz, 3H), 0.80 - 0.71 (m, 2H), 0.64 - 0.56 (m, 2H).

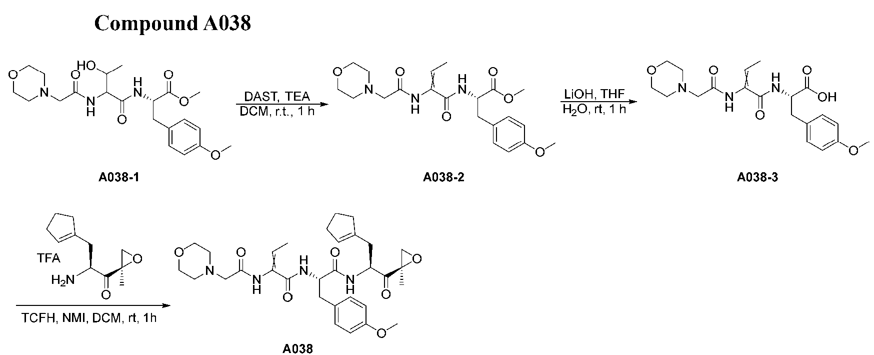

**Step 1:** Under a nitrogen atmosphere and at room temperature, to a solution of methyl (2*S*)-2-(3-hydroxyl-2-[2-(morpholine-4-yl)acetamido]butyramido-3-(4-methoxyphenyl)propionate (200 mg, 0.457 mmol, 1 eq) in dichloromethane (3 mL), triethyl amine (138.78 mg, 1.371 mmol, 3 eq) was added dropwise. The reaction solution was cooled to 0°C, and diethylaminosulfur trifluoride (110.53 mg, 0.685 mmol, 1.5 eq) was then added dropwise. The reaction solution was heated to room temperature, and reacted with stirring for 1 hr. The reaction mixture was quenched with iced water (20 mL) at 0°C, and extracted with ethyl acetate (3 x 20 mL). The organic phases were combined, back washed with saturated brine (2 x 20 mL), and dried over anhydrous sodium sulfate. After the resulting mixture was filtered, the filtrate was concentrated under reduced pressure. The obtained residue was purified by reverse-phase column chromatography to obtain methyl (*S*)-3-(4-methoxyphenyl)-2-(2-(2-morpholinoacetamido)but-2-enamido)propionate (50 mg, 26.07%) as a light yellow oil.

LCMS-A038-2: (ESI, *m*/*z):* 420.15 [M+H]⁺.

**Step 2:** At room temperature, to a solution of methyl (*S*)-3-(4-methoxyphenyl)-2-(2-(2-morpholinoacetamido)but-2-enamido)propionate (50 mg, 0.119 mmol, 1 eq) in tetrahydrofuran/water (v/v=1/1, 2 mL), lithium hydroxide (5.71 mg, 0.238 mmol, 2 eq) was added. The reaction solution was reacted with stirring at room temperature for 1 hr. The reaction mixture was concentrated under reduced pressure, to obtain (*S*)-3-(4-methoxyphenyl)-2-(2-(2-morpholinoacetamido)but-2-enamido)propionic acid (50 mg, crude product). The crude product was directly used in the next step without further purification.

LCMS-A038-3: (ESI, *m*/*z):* 404.30 [M+H]⁻.

**Step 3:** Using a method similar to that in Step 3 of Example 1, the compound *N*-((*S*)-1-(((*S*)-3-(cyclopent-1-en-1-yl)-1-((*R*)-2-methyloxiran-2-yl)-1-oxoprop-2-yl)amino)-3-(4-methoxyphenyl)-1-oxoprop-2-yl)-2-(2-morpholinoacetamido)but-2-enamide (12.3 mg, 17.12%) as a white solid was obtained.

LCMS-A038: (ESI, *m*/*z):* 583.20 [M+H]⁺.

¹H NMR-A038: (400 MHz, DMSO-*d*₆) *δ* 8.93 (s, 1H), 8.33 - 8.13 (m, 1H), 7.81 - 7.65 (m, 1H), 7.22 - 7.02 (m, 2H), 6.86 - 6.70 (m, 2H), 6.29 - 6.13 (m, 1H), 5.39 (s, 1H), 4.57 - 4.28 (m, 2H), 3.70 (d, *J* = 0.8 Hz, 3H), 3.58 (t, *J* = 4.7 Hz, 4H), 3.23 - 3.18 (m, 1H), 3.05 - 2.94 (m, 3H), 2.93 - 2.85 (m, 1H), 2.81 - 2.72 (m, 1H), 2.46 (d, *J* = 5.4 Hz, 6H), 2.21 (s, 4H), 1.84 - 1.72 (m, 2H), 1.60 (d, *J* = 6.9 Hz, 3H), 1.39 (d, *J* = 5.8 Hz, 3H).

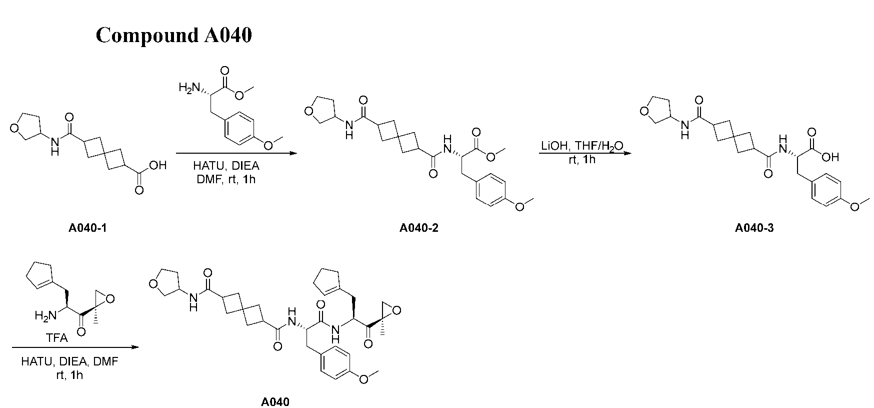

Using a method similar to that in Example 1, by replacing Intermediate A001-1 with Intermediate A040-1, *N*²-((*S*)-1-(((*S*)-3-(cyclopent-1-en-1-yl)-1-((*R*)-2-methyloxiran-2-yl)-1-oxoprop-2-yl)amino)-3-(4-methoxyphenyl)-1-oxoprop-2-yl)-*N*⁶-(tetrahydrofuran -3-yl)spiro[3.3]heptan-2,6-diformamide (3.0 mg, 3.50%) as a white solid was obtained.

LCMS-A040: (ESI, *m*/*z):* 608.20 [M+H]⁺.

¹H NMR-A040: (400 MHz, Chloroform-*d*) *δ* 7.21 - 7.10 (m, 2H), 6.84 (t, *J* = 8.7 Hz, 2H), 6.53 - 5.64 (m, 2H), 5.48 (s, 1H), 5.35 - 5.12 (m, 2H), 4.52 (d, *J* = 16.4 Hz, 3H), 3.91 (q, *J* = 7.7 Hz, 1H), 3.85 - 3.70 (m, 5H), 3.62 (d, *J* = 9.6 Hz, 1H), 3.28 - 2.67 (m, 7H), 2.52 - 2.01 (m, 14H), 1.79 (dd, *J* = 15.8, 8.3 Hz, 2H), 1.50 (s, 3H).

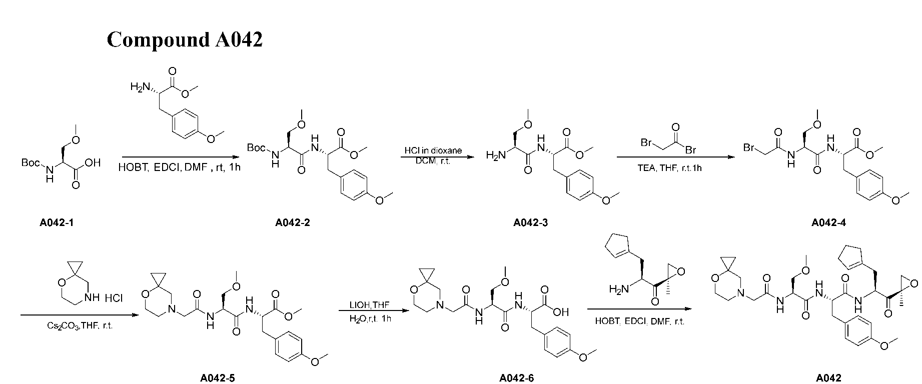

**Step 1:** Under a nitrogen atmosphere and at room temperature, 1-hydroxybenzotriazole (462.23 mg, 3.422 mmol, 1.5 eq), 1-(3-dimethylaminopropyl)-3-ethylcarbodiimide hydrochloride (1.3 g, 6.806 mmol, 3 eq) and methyl (S)-2-amino-3-(4-methoxyphenyl)propionate (575 .12 mg, 6.806 mmol, 1.2 eq) were added to a solution of *N*-(t-butoxycarbonyl)-*O*-methyl-L-serine (500 mg, 2.283 mmol, 1 eq) in *N*,*N*-dimethyl formamide (2 mL), and stirred at room temperature for 1 hr. The reaction mixture was quenched with water at room temperature, and extracted with ethyl acetate (3 x 50 mL). The organic phases were combined, back washed with a saturated sodium chloride solution (1 x 50 mL), and dried over anhydrous sodium sulfate. After the resulting mixture was filtered, the filtrate was concentrated under reduced pressure. The obtained residue was purified by reverse-phase column chromatography to obtain methyl (S)-2-((S)-2-((t-butoxycarbonyl)amino)-3-methoxypropionamido)-3-(4-methoxyphenyl)propionate (400 mg, 42.73%) as a white solid.

LCMS-A042-2 (ESI, m/z): 410.90, [M+H]⁺.

**Step 2:** At room temperature, 4 mol/L hydrochloric acid solution in dioxane (5.00 mL) was added to a solution of methyl (*S*)-2-((*S*)-2-((t-butoxycarbonyl)amino)-3-methoxypropionamido)-3-(4-methoxyphenyl)propionate (300 mg, 0.720 mmol, 1 eq) in dichloromethane (5.00 mL). The reaction solution was stirred at room temperature for 1 hr. The reaction solution was concentrated under reduced pressure, to obtain the crude product methyl (*S*)-2-((*S*)-2-amino-3-methoxypropionamido)-3-(4-methoxyphenyl)propionate (200 mg, crude product). The crude product was directly used in the next step without further purification.

LCMS-A042-3 (ESI, *m*/*z*): 311.45, [M+H]⁺.

**Step 3:** Under a nitrogen atmosphere and at room temperature, triethyl amine (268.73 uL, 1.932 mmol, 3 eq) was added to a solution of methyl (*S*)-2-((*S*)-2-amino-3-methoxypropionamido)-3-(4-methoxyphenyl)propionate (200 mg, 0.644 mmol, 1 eq) in tetrahydrofuran (20.00 mL), and bromoacetyl bromide (67.16 uL, 0.773 mmol, 1.2 eq) was added dropwise at 0°C. The reaction solution was heated to room temperature, and stirred for 1 hr. The reaction solution was cooled to room temperature, and extracted with ethyl acetate (3 x 20 mL). The organic phases were combined, back washed with saturated brine (1 x 20 mL), and dried over anhydrous sodium sulfate. After filtration, the filtrate was concentrated under reduced pressure. The crude product was directly used in the next step without further purification.

LCMS-A042-4 (ESI, *m*/*z*): 432.80, [M+H]⁺.

**Step 4:** Under a nitrogen atmosphere and at room temperature, 4-oxa-7-azaspiro[2.5]octane hydrochloride (79.10 mg, 0.529 mmol, 1.2 eq) and cesium carbonate (430.61 mg, 1.323 mmol, 3 eq) were added to a solution of methyl (*S*)-2-((*S*)-2-(2-bromoacetamido)-3-methoxypropionamido)-3-(4-methoxyphenyl)propionate (190 mg, 0.437 mmol, 1 eq) in tetrahydrofuran (38.00 mL). The reaction solution was stirred at room temperature for 1 hr. The reaction mixture was quenched with water at room temperature, and extracted with ethyl acetate (3 x 50 mL). The organic phases were combined, back washed with saturated brine (1 x 50 mL), and dried over anhydrous sodium sulfate. After the resulting mixture was filtered, The filtrate was concentrated under reduced pressure. The obtained residue was purified by reverse-phase column chromatography under conditions including: chromatographic column specification: mobile phase: water and acetonitrile, gradient from 10% to 90% over 30 min, and UV 220 nm. Methyl (*S*)-2-((*S*)-2-(2-(4-oxa-7-azaspiro[2.5]octan-7-yl)acetamido)-3-methoxypropionamido)-3-(4-methoxyphenyl)propionate (140 mg, 68.56%) as a white solid was obtained.

LCMS-A042-5 (ESI, m/z): 464.50, [M+H]⁺.

**Steps 5 and 6:** Using a method similar to that in Example 1, by replacing Intermediate A001-2 with Intermediate A042-5, 2-(2-(4-oxa-7-azaspiro[2.5]octan-7-yl)acetyl)-*N*-((*S*)-1-(((*S*)-3-(cyclopent-1-en-1-yl)-1-((R)-2-methyloxiran-2-yl)-1-oxoprop-2-yl)amino)-3-(4-methoxyphenyl)-1-oxoprop-2-yl)cyclopropan-1-formamide (2 mg, 1.95%) as a white solid was obtained.

LCMS-A042 (ESI, *m*/*z):* 627.65 [M+H]⁺.

¹H NMR-A042 (400 MHz, Chloroform-d) *δ* 7.96 (s, 1H), 7.26 (s, 1H), 7.14 (d, J = 7.6 Hz, 2H), 6.82 (d, J = 8.2 Hz, 2H), 6.13 (d, J = 29.8 Hz, 1H), 5.30 (s, 1H), 4.62 - 4.41 (m, 3H), 3.90 - 3.65 (m, 6H), 3.55 - 3.20 (m, 6H), 3.15 - 3.01 (m, 2H), 2.97 - 2.86 (m, 3H), 2.71 (s, 2H), 2.60 - 2.44 (m, 3H), 2.24 - 2.13 (m, 4H), 1.84 - 1.78 (m, 2H), 1.45 (s, 3H), 0.82 (s, 2H), 0.61 (d, J = 25.1 Hz, 2H).

Following a similar method, the compounds below were further prepared.

| | |
|---|---|
| 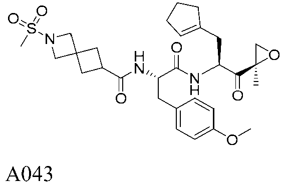 | LCMS-A043 (ESI, *m*/*z):* 574.25 [M+H]⁺. ¹H NMR-A043 (400 MHz, Methanol-*d*₄) *δ* 7.17 (d, *J* = 8.3 Hz, 2H), 6.83 (d, *J* = *8.2* Hz, 2H), 6.00 (d, *J =* 7.4 Hz, 1H), 5.79 (d, *J =* 6.6 Hz, 1H), 5.27 (s, 1H), 4.57 - 4.45 (m, 2H), 3.89 (s, 2H), 3.86 (d, *J* = 2.5 Hz, 2H), 3.79 (s, 3H), 3.24 (d, *J =* 5.0 Hz, 1H), 3.04 - 2.95 (m, 1H), 2.93 - 2.88 (m, 2H), 2.82 (s, 4H), 2.53 - 2.41 (m, 2H), 2.38 - 2.29 (m, 3H), 2.21 (d, *J* = 10.4 Hz, 2H), 2.17 - 2.07 (m, 3H), 1.85 - 1.78 (m, 2H), 1.50 (s, 3H). |
| 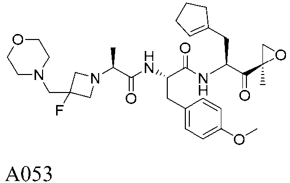 | LCMS-A053 (ESI, *m*/*z*): 601.33 [M+H]⁺. ¹H NMR-A053: (400 MHz, Methanol-*d*₄) *δ* 8.30 (s, 1H), 7.14 - 7.10 (m, 2H), 6.84 - 6.81 (m, 2H), 5.46 (s, 1H), 4.66 (dd, *J* = 9.1, 4.6 Hz, 1H), 4.57 (dd, *J* = 9.4, 5.3 Hz, 1H), 3.76 (s, 3H), 3.68 (t, *J* = 4.7 Hz, 4H), 3.44 (dd, *J* = 14.9, 8.8 Hz, 1H), 3.22 (p, *J* = 5.5 Hz, 3H), 3.11 - 3.04 (m, 2H), 2.94 (t, *J* = 6.4 Hz, 2H), 2.83 - 2.77 (m, 1H), 2.77 (s, 1H), 2.70 (s, 1H), 2.53 (dt, *J* = 11.5, 5.7 Hz, 5H), 2.32 - 2.25 (m, 5H), 1.90 - 1.83 (m, 2H), 1.45 (s, 3H), 1.08 (d, *J* = 6.8 Hz, 3H). |
| 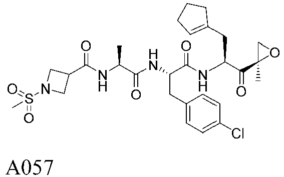 | LCMS-A057 (ESI, *m*/*z*): 609.21 [M+H]⁺. ¹H NMR-A057 (400 MHz, Chloroform-*d*) *δ* 7.28 (d, *J* = 1.7 Hz, 1H), 7.19 (d, *J* = 8.4 Hz, 3H), 6.54 (d, *J* = 7.5 Hz, 1H), 6.16 (d, *J* = 7.1 Hz, 1H), 5.80 (d, *J* = 6.7 Hz, 1H), 5.30 (s, 1H), 4.59 - 4.52 (m, 1H), 4.52 - 4.47 (m, 1H), 4.45 - 4.36 (m, 1H), 4.18 - 4.16 (m, 1H), 4.15 - 4.09 (m, 2H), 4.07 - 4.03 (m, 1H), 3.26 - 3.19 (m, 2H), 3.08 - 3.01 (m, 1H), 2.97 (d, *J* = 7.7 Hz, 1H), 2.94 (s, 3H), 2.92 (d, *J =* 5.0 Hz, 1H), 2.51 (d, *J* = 14.5 Hz, 1H), 2.23 (d, *J* = 6.0 Hz, 2H), 2.19 (d, *J* = 5.5 Hz, 1H), 2.15 (t, *J* = 7.0 Hz, 2H), 1.89 - 1.77 (m, 2H), 1.51 (s, 3H), 1.36 (d, *J* = 7.0 Hz, 3H). |
| 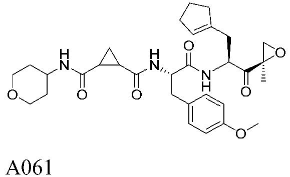 | LCMS-A061 (ESI, *m*/*z):* 568.29 [M+H]⁺. ¹H NMR-A061 (400 MHz, Chloroform-*d*) *δ* :7.20 - 7.10 (m, 2H), 6.89 - 6.77 (m, 2H), 6.40 - 6.26 (m, 1H), 5.98 - 5.85 (m, 1H), 5.82 - 5.60 (m, 1H), 5.27 (s, 1H), 4.65 - 4.45 (m, 2H), 3.96 (d, *J* = 12.2 Hz, 3H), 3.79 (s, 3H), 3.53 - 3.37 (m, 2H), 3.30 - 3.00 (m, 2H), 2.97 - 2.84 (m, 2H), 2.48 (d, *J* = 14.2 Hz, 1H), 2.25 - 2.18 (m, 2H), 2.18 - 2.09 (m, 2H), 1.97 - 1.78 (m, 6H), 1.54 - 1.47 (m, 6H), 1.35 - 1.26 (m, 2H). |
| 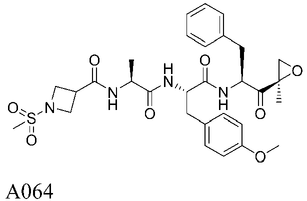 | LCMS-A064 (ESI, *m*/*z):* 615.24 [M+H]⁺. ¹H NMR-A064 (400 MHz, DMSO-*d*₆) *δ* 8.41 (d, *J* = 7.4 Hz, 1H), 8.15 (d, *J* = 7.5 Hz, 1H), 7.85 (d, *J* = 8.3 Hz, 1H), 7.33 - 7.27 (m, 2H), 7.26 - 7.17 (m, 3H), 7.07 (d, *J =* 8.5 Hz, 2H), 6.77 (d, *J =* 8.5 Hz, 2H), 4.57 (d, *J* = 5.4 Hz, 1H), 4.42 (q, *J =* 4.0 Hz, 1H), 4.24 (t, *J =* 7.2 Hz, 1H), 3.93 - 3.81 (m, 4H), 3.70 (s, 3H), 3.17 (d, *J* = 5.3 Hz, 1H), 2.98 (d, *J* = 5.2 Hz, 1H), 2.95 (s, 3H), 2.92 (d, *J* = 5.2 Hz, 1H), 2.87 (d, *J* = 4.5 Hz, 1H), 2.72 (d, *J* = 9.1 Hz, 2H), 1.35 (s, 3H), 1.09 (d, *J* = 7.0 Hz, 3H). |
| 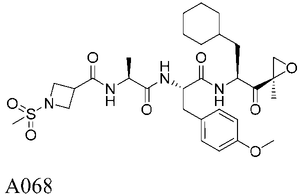 | LCMS-A068 (ESI, *m*/*z):* 621.29 [M+H]⁺. ¹H NMR-A068 (400 MHz, Chloroform-*d*) *δ* 7.14 (d, *J* = 8.5 Hz, 2H), 6.88 - 6.77 (m, 2H), 6.47 (d, *J* = 7.4 Hz, 1H), 6.20 (d, *J* = 7.1 Hz, 1H), 5.89 (d, *J* = 7.7 Hz, 1H), 5.88 - 5.12 (m, 1H), 4.61 - 4.47 (m, 2H), 4.42 (q, *J* = 6.9 Hz, 1H), 4.17 - 3.98 (m, 4H), 3.79 (s, 3H), 3.30 - 3.15 (m, 2H), 3.05 (dd, *J* = 14.1, 6.0 Hz, 1H), 2.98 - 2.93 (m, 4H), 2.93 - 2.83 (m, 1H), 1.75 (d, *J* = 14.1 Hz, 1H), 1.71 - 1.61 (m, 4H), 1.51 (s, 3H), 1.38 (d, *J* = 6.9 Hz, 3H), 1.25 - 1.03 (m, 5H), 0.99 - 0.77 (m, 2H). |
| 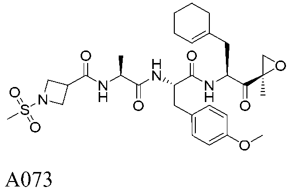 | LCMS-A073 (ESI, *m*/*z*): 619.27 [M+H]⁺. ¹H NMR-NMR-A073 (400 MHz, Chloroform-*d*): *δ* 7.23 - 7.12 (m, 2H), 6.89 - 6.77 (m, 2H), 6.56 (d, *J* = 7.3 Hz, 1H), 6.22 (d, *J* = 7.0 Hz, 1H), 5.66 (d, *J* = 6.3 Hz, 1H), 5.19 (s, 1H), 4.57 - 4.35 (m, 3H), 4.16 - 3.99 (m, 4H), 3.79 (s, 3H), 3.30 - 3.17 (m, 2H), 3.12 - 3.00 (m, 1H), 2.95 (s, 3H), 2.92 - 2.83 (m, 2H), 2.37 (d, *J* = 13.6 Hz, 1H), 1.89 (s, 2H), 1.86 - 1.77 (m, 3H), 1.56- 1.52 (m, 3H), 1.50 (s, 3H), 1.47 - 1.41 (m, 1H), 1.38 (d, *J* = 7.0 Hz, 3H). |
| 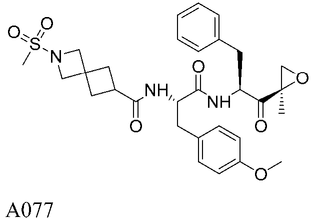 | LCMS-A077 (ESI, *m*/*z*): 584.24 [M+H]⁺. ¹H NMR-A077: (400 MHz, Chloroform-*d*) *δ* 7.25 - 7.19 (m, 3H), 7.15 - 7.08 (m, 2H), 6.96 (dd, *J* = 6.9, 2.5 Hz, 2H), 6.86 - 6.79 (m, 2H), 5.89 (t, *J =* 8.4 Hz, 2H), 4.71 - 4.61 (m, 1H), 4.47 (q, *J* = 7.1 Hz, 1H), 3.89 (s, 2H), 3.85 (d, *J* = 2.7 Hz, 2H), 3.80 (s, 3H), 3.23 (d, *J* = 4.9 Hz, 1H), 3.07 (dd, *J =* 13.9, 4.9 Hz, 1H), 2.98 (dd, *J* = 13.9, 5.9 Hz, 1H), 2.92 (d, *J* = 4.9 Hz, 1H), 2.86 (d, *J* = 7.9 Hz, 1H), 2.82 (s, 3H), 2.74 (q*, J =* 7.9 Hz, 1H), 2.61 (dd, *J =* 13.9, 8.3 Hz, 1H), 2.38 (dd, *J* = 12.0, 7.2 Hz, 1H), 2.32 (d, *J* = 8.1 Hz, 3H), 1.50 (s, 3H). |
| 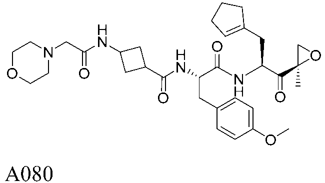 | LCMS-A080 (ESI, *m*/*z*): 597.32 [M+H]⁺. ¹H NMR-A080: (400 MHz, Chloroform-d) *δ* 7.42-7.21 (m, *J* = 8.9 Hz, 1H), 7.20 - 7.13 (m, 2H), 6.92 - 6.76 (m, 2H), 6.14 (dd, *J* = 47.0, 7.5 Hz, 1H), 5.86 (t, *J* = 6.0 Hz, 1H), 5.28 (s, 1H), 4.61 - 4.32 (m, 3H), 3.79 (s, 3H), 3.76 - 3.56 (m, 4H), 3.25 (d, *J* = 5.0 Hz, 1H), 3.13 - 2.98 (m, 1H), 2.97 (s, 2H), 2.93 - 2.85 (m, 2H), 2.75 - 2.61 (m, 1H), 2.63 - 2.32 (m, 7H), 2.30 - 2.18 (m, 3H), 2.18 - 2.01 (m, 4H), 1.90 - 1.75 (m, 2H), 1.50 (s, 3H). |
| 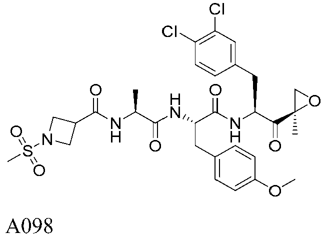 | LCMS-A098 (ESI, *m*/*z):* 683.16 [M+H]⁺. ¹H NMR-A098: (400 MHz, Chloroform-d) δ 7.32 (d, *J =* 8.2 Hz, 3H), 7.09 (d, *J =* 7.9 Hz, 2H), 6.84 (dd, *J =* 19.5, 8.3 Hz, 2H), 6.34 (d, *J =* 7.3 Hz, 1H), 6.17 (d, *J* = 7.0 Hz, 1H), 6.05 (d, *J =* 7.5 Hz, 1H), 4.65 (td, *J =* 7.7, 4.8 Hz, 1H), 4.45 (q, *J =* 7.6 Hz, 1H), 4.36 (dt, *J =* 14.2, 7.1 Hz, 1H), 4.27 - 3.87 (m, 4H), 3.80 (s, 3H), 3.22 (t, *J* = 6.3 Hz, 2H), 3.14 - 2.96 (m, 2H), 3.07 - 2.73 (m, 5H), 2.58 (dd, *J =* 14.1, 8.4 Hz, 1H), 1.51 (d, *J =* 11.3 Hz, 3H), 1.31 (d, *J* = 7.0 Hz, 3H). |
| 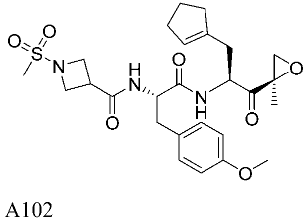 | LCMS-A102 (ESI, *m*/*z*): 534.22 [M+H]⁺. ¹H NMR-A102: (400 MHz, Chloroform-*d*) *δ* 7.22 - 7.17 (m, 2H), 6.88 - 6.79 (m, 2H), 6.26 (d, *J* = 7.3 Hz, 1H), 5.68 (d, *J* = 6.5 Hz, 1H), 5.26 (s, 1H), 4.60-4.52 (m, 1H), 4.51-4.44 (m, 1H), 4.10 - 4.03 (m, 3H), 3.99-3.94 (m, 1H), 3.80 (s, 3H), 3.25 - 3.16 (m, 2H), 3.06- 3.00 (m, 1H), 2.91 (d, *J* = 5.4 Hz, 5H), 2.50 (d, *J* = 13.7 Hz, 1H), 2.22 (s, 2H), 2.17- 2.09 (m, 3H), 1.88- 1.76 (m, 2H), 1.51 (s, 3H). |
| 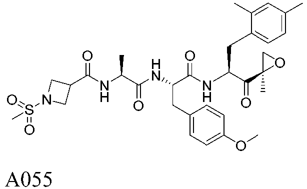 | LCMS-A055 (ESI, *m*/*z):* 643.27 [M+H]⁺. ¹H NMR-A055 (400 MHz, Chloroform-d) *δ*: 7.18 (d, *J* = 8.5 Hz, 2H), 6.94 (d, *J* = 9.8 Hz, 1H), 6.88 - 6.81 (m, 3H), 6.57 (d, *J* = 7.6 Hz, 1H), 6.45 (d, *J* = 7.1 Hz, 1H), 6.18 (d, *J* = 7.0 Hz, 1H), 5.71 (d, *J* = 6.8 Hz, 1H), 4.69 - 4.58 (m, 1H), 4.45 - 4.31 (m, 2H), 4.15 - 4.00 (m, 5H), 3.81 (s, 3H), 3.27 (d, *J* = 5.0 Hz, 1H), 3.25 - 3.18 (m, 1H), 3.07 - 2.97 (m, 2H), 2.93 (d, *J* = 6.9 Hz, 3H), 2.86 - 2.77 (m, 1H), 2.45 - 2.31 (m, 1H), 2.26 (d, *J* = 2.1 Hz, 6H), 1.46 (s, 3H), 1.31 (d, *J* = 7.0 Hz, 3H). |
| 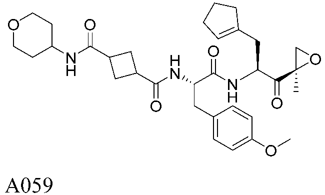 | LCMS-A059 (ESI, *m*/*z*): 582.31 [M+H]⁺. ¹H NMR-A059: (400 MHz, Chloroform-d) *δ* 7.19 (d, *J* = 8.2 Hz, 2H), 6.83 (d, *J* = 8.1 Hz, 2H), 6.71 (s, 1H), 6.44 - 6.01 (m, 1H), 6.01 - 5.67 (m, 1H), 5.30 (s, 1H), 4.61 - 4.48 (m, 2H), 4.05 - 3.94 (m, 3H), 3.78 (s, 3H), 3.48 (t, *J* = 11.5 Hz, 2H), 3.26 (d, *J* = 5.0 Hz, 1H), 3.08 - 2.85 (m, 5H), 2.57 - 2.42 (m, 3H), 2.41 - 2.30 (m, 2H), 2.29 - 2.09 (m, 5H), 1.93 - 1.86 (m, 2H), 1.86 - 1.76 (m, 2H), 1.58 - 1.52 (m, 2H), 1.50 (s, 3H). |
| 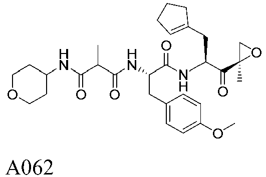 | LCMS-A062 (ESI, *m*/*z*): 556.29 [M+H]⁺. ¹H NMR-A062: (400 MHz, Chloroform-d) *δ* 7.21 - 7.11 (m, 2H), 6.96 - 6.75 (m, 3H), 6.54 - 6.37 (m, 1H), 5.95 (d, *J* = 6.8 Hz, 1H), 5.29 (s, 1H), 4.58 - 4.47 (m, 2H), 3.93 (d, *J* = 10.1 Hz, 3H), 3.78 (s, 3H), 3.46 (dd, *J* = 12.7, 10.3 Hz, 2H), 3.27 (t, *J* = 4.3 Hz, 1H), 3.08 - 2.84 (m, 4H), 2.49 (d, *J* = 14.4 Hz, 1H), 2.29 - 2.19 (m, 3H), 2.18 - 2.09 (m, 2H), 1.92 - 1.75 (m, 4H), 1.52 - 1.47 (m, 4H), 1.47 - 1.44 (m, 1H), 1.44 - 1.31 (m, 3H). |
| 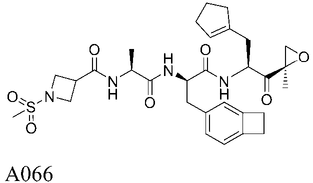 | LCMS-A066 (ESI, *m*/*z):* 601.26 [M+H]⁺. ¹H NMR-A066 (400 MHz, Chloroform-*d*) *δ* 7.12 - 7.02 (m, 1H), 6.99 - 6.94 (m, 2H), 6.50 (d, *J =* 7.2 Hz, 1H), 6.23 (d, *J* = 7.1 Hz, 1H), 5.76 (d, *J* = 6.7 Hz, 1H), 5.22 (s, 1H), 4.57 - 4.46 (m, 2H), 4.42 (t, *J* = 6.9 Hz, 1H), 4.15 - 4.09 (m, 2H), 4.09 - 4.04 (m, 2H), 3.28 - 3.19 (m, 2H), 3.14 (s, 4H), 3.12 - 3.05 (m, 1H), 2.94 (s, 3H), 2.91 (d, *J =* 5.0 Hz, 2H), 2.87 (d, *J* = 8.1 Hz, 1H), 2.92 - 2.88 (m, 2H), 2.13 (t, *J* = 7.3 Hz, 3H), 1.86 - 1.78 (m, 2H), 1.50 (s, 3H), 1.38 (d, *J =* 7.0 Hz, 3H). |
| 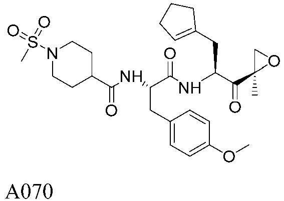 | LCMS-A070 (ESI, *m*/*z*): 562.25 [M+H]⁺. ¹H NMR-A070: (400 MHz, Chloroform-*d*) *δ* 7.21 - 7.14 (m, 2H), 6.88 - 6.79 (m, 2H), 6.15 (d, *J* = 7.3 Hz, 1H), 5.75 (d, *J* = 6.5 Hz, 1H), 5.53 - 5.04 (m, 2H), 4.55 (q, *J* = 7.2 Hz, 1H), 4.52 - 4.43 (m, 1H), 3.79 (s, 3H), 3.78 - 3.64 (m, 2H), 3.25 (d, *J* = 5.0 Hz, 1H), 3.03 (dd, *J* = 13.9, 5.8 Hz, 1H), 2.95 - 2.87 (m, 2H), 2.83 - 2.72 (m, 5H), 2.50 (d, *J* = 14.3 Hz, 1H), 2.28 - 2.20 (m, 2H), 2.20 - 2.17 (m, 1H), 2.16 - 2.10 (m, 2H), 1.94 - 1.69 (m, 6H), 1.51 (s, 3H). |
| 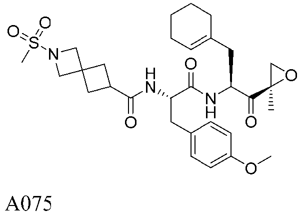 | LCMS-A075 (ESI, *m*/*z*): 588.27 [M+H]⁺. ¹H NMR-A075: (400 MHz, DMSO-*d*₆) *δ* 8.19 (d, *J* = 7.3 Hz, 1H), 7.84 (d, *J* = 8.7 Hz, 1H), 7.10 (d, *J* = 8.5 Hz, 2H), 6.79 (d, *J* = 8.6 Hz, 2H), 5.39 (s, 1H), 4.54 - 4.42 (m, 2H), 3.82 (s, 2H), 3.69 (d, *J* = 9.2 Hz, 3H), 3.65 (s, 1H), 3.63 (s, 1H), 3.21 (d, *J* = 5.3 Hz, 1H), 2.97 (d, *J =* 5.3 Hz, 1H), 2.92 (s, 3H), 2.87 - 2.79 (m, 2H), 2.56 (s, 1H), 2.22 (d, *J* = 14.8 Hz, 2H), 2.20 - 2.15 (m, 2H), 2.03 - 1.93 (m, 3H), 1.92 (s, 2H), 1.56 (d, *J* = 6.7 Hz, 2H), 1.51 - 1.42 (m, 2H), 1.37 (s, 3H). |
| 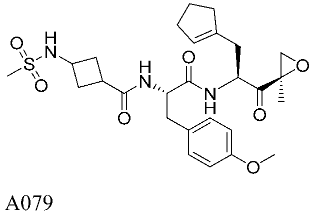 | LCMS-A079 (ESI, *m*/*z)*: 548.25 [M+H]⁺. ¹H NMR-A079 (400 MHz, Chloroform-*d*) *δ* 7.15 (dd, *J* = 20.3, 8.2 Hz, 1H), 7.07 (t, *J* = 7.9 Hz, 1H), 6.89 - 6.75 (m, 2H), 6.47 - 5.82 (m, 2H), 5.67 - 5.20 (m, 1H), 4.88 - 4.32 (m, 3H), 4.28 - 3.83 (m, 1H), 3.78 (dd, *J* = 6.1, 4.2 Hz, 3H), 3.34 - 3.20 (m, 1H), 3.12 - 2.77 (m, 6H), 2.76 - 2.38 (m, 4H), 2.19 (d, *J* = 27.7 Hz, 7H), 1.82 (q, *J* = 8.4, 7.7 Hz, 2H), 1.49 (dd, *J* = 9.4, 2.0 Hz, 3H). |
| 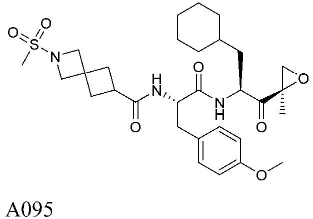 | LCMS-A095 (ESI, *m*/*z*): 590.28 [M+H]⁺. ¹H NMR-A095: (400 MHz, Chloroform-*d*) *δ* 7.14 (d, *J* = 8.3 Hz, 2H), 6.87 - 6.79 (m, 2H), 6.02 - 5.82 (m, 2H), 4.56 (q, *J* = 7.1 Hz, 1H), 4.53 - 4.45 (m, 1H), 3.90 (s, 2H), 3.86 (d, *J =* 2.7 Hz, 2H), 3.79 (s, 3H), 3.21 (d, *J* = 5.0 Hz, 1H), 3.07 - 2.91 (m, 2H), 2.89 (d, *J* = 4.8 Hz, 1H), 2.83 (s, 4H), 2.45 (dd, *J* = 12.2, 7.1 Hz, 1H), 2.40 - 2.28 (m, 3H), 1.80 1.72 (m, 2H), 1.71 - 1.60 (m, 4H), 1.51 (s, 3H), 1.31 - 1.04 (m, 5H), 0.90 (q, *J* = 11.5, 11.0 Hz, 2H). |
| 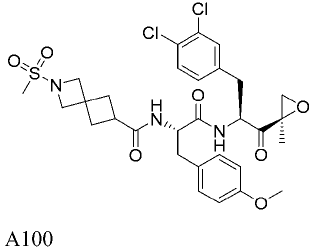 | LCMS-A100 (ESI, *m*/*z*): 652.16 [M+H]⁺. ¹H NMR-A100 (400 MHz, Chloroform-d) *δ* 7.38 - 7.29 (m, 1H), 7.14 - 7.01 (m, 3H), 6.88 - 6.76 (m, 3H), 6.07 (d, *J* = 7.4 Hz, 1H), 5.78 (d, *J* = 7.4 Hz, 1H), 4.65 (td, *J* = 7.9, 4.6 Hz, 1H), 4.48 (q, *J* = 7.2 Hz, 1H), 3.89 (s, 2H), 3.85 (d, *J* = 4.0 Hz, 2H), 3.80 (s, 3H), 3.18 (d, *J* = 4.8 Hz, 1H), 3.07 - 2.92 (m, 3H), 2.88 - 2.78 (m, 4H), 2.77 - 2.67 (m, 1H), 2.57 (dd, *J* = 14.0, 8.3 Hz, 1H), 2.43 - 2.24 (m, 4H), 1.50 (s, 3H). |
| 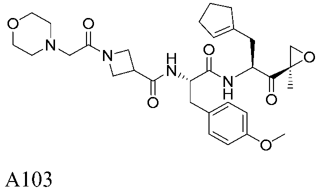 | LCMS-A103 (ESI, *m*/*z):* 583.31 [M+H]⁺. ¹H NMR-A103 (400 MHz, Chloroform-d) *δ* 7.19 (dd, *J* = 11.7, 8.3 Hz, 2H), 6.91 - 6.77 (m, 2H), 6.24 (dd, *J* = 23.4, 7.4 Hz, 1H), 5.71 (dd, *J =* 16.1, 6.5 Hz, 1H), 5.26 (d, *J* = 8.0 Hz, 1H), 4.66 - 4.53 (m, 1H), 4.45 (ddd, *J =* 15.0, 9.2, 5.2 Hz, 1H), 4.34 (p, *J* = 8.9 Hz, 2H), 4.17 - 4.02 (m, 2H), 3.80 (s, 3H), 3.72 (t, *J* = 4.6 Hz, 4H), 3.23 (q, *J* = 8.0, 5.9 Hz, 2H), 3.11 - 2.96 (m, 3H), 2.90 (dd, *J* = 11.4, 4.9 Hz, 2H), 2.62 - 2.33 (m, 5H), 2.21 (d, *J* = 9.7 Hz, 2H), 2.14 (q, *J* = 8.9, 8.0 Hz, 3H), 1.88 - 1.75 (m, 2H). |
| 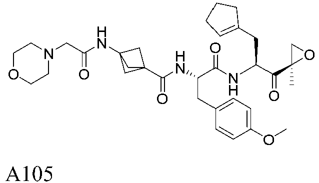 | LCMS-A105: (ESI, *m*/*z*): 609.20 [M+H]⁺. ¹H NMR-A105 (400 MHz, Chloroform-d) *δ* 7.52 (s, 1H), 7.21 - 7.11 (m, 2H), 6.89 - 6.78 (m, 2H), 6.15 (d, *J* = 7.5 Hz, 1H), 5.86 (d, *J* = 6.4 Hz, 1H), 5.27 (s, 1H), 4.60 - 4.50 (m, 1H), 4.50 - 4.43 (m, 1H), 3.79 (s, 7H), 3.27 (d, *J* = 5.0 Hz, 1H), 3.11 - 2.92 (m, 3H), 2.92 - 2.86 (m, 2H), 2.80 - 2.35 (m, 5H), 2.31 (s, 6H), 2.21 (d, *J* = 9.7 Hz, 2H), 2.18 - 2.09 (m, 3H), 1.88 - 1.75 (m, 2H), 1.51 (s, 3H). |
| 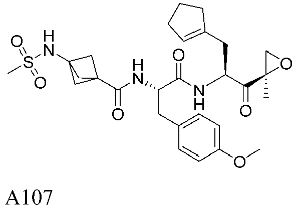 | LCMS-A107: (ESI, *m*/*z*):560.50 [M+H]⁺. ¹H NMR-A107: (400 MHz, Chloroform-*d*) *δ* 7.17 (d, *J* = 8.4 Hz, 2H), 6.84 (d, *J* = 8.5 Hz, 2H), 6.20 (d, *J* = 7.4 Hz, 1H), 5.75 (d, *J* = 6.4 Hz, 1H), 5.26 (s, 1H), 4.92 (s, 1H), 4.57 - 4.42 (m, 2H), 3.80 (s, 3H), 3.25 (d, *J* = 5.0 Hz, 1H), 3.03 (dd, *J* = 13.9, 5.3 Hz, 1H), 2.99 (s, 3H), 2.93 - 2.85 (m, 2H), 2.56 - 2.45 (m, 1H), 2.28 (s, 6H), 2.26 - 2.18 (m, 2H), 2.18 - 2.07 (m, 3H), 1.91 - 1.74 (m, 2H), 1.51 (s, 3H). |
| 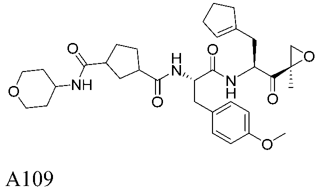 | LCMS-A109: (ESI, *m*/*z*):596.15, [M+H]⁺. ¹H NMR-A109: (400 MHz, Chloroform-d) *δ* 7.18 (dd, *J* = 8.7, 2.6 Hz, 1H), 7.10 (d, *J =* 8.4 Hz, 1H), 6.92 - 6.67 (m, 4H), 6.09 (dd, *J* = 23.8, 6.7 Hz, 1H), 5.37 - 5.31 (m, 1H), 4.63 - 4.51 (m, 2H), 4.06 - 3.91 (m, 3H), 3.78 (*q, J* = 1.2 Hz, 3H), 3.50 (t, *J* = 11.6 Hz, 2H), 3.34 - 3.27 (m, 1H), 3.07 - 2.94 (m, 2H), 2.90 (dd, *J* = 5.2, 2.2 Hz, 1H), 2.71 (d, *J* = 7.1 Hz, 2H), 2.50 (d, *J* = 14.8 Hz, 1H), 2.24 (s, 3H), 2.17 (d, *J* = 7.0 Hz, 2H), 2.06 - 1.98 (m, 2H), 1.98 - 1.67 (m, 8H), 1.52 (s, 2H), 1.49 (d, *J* = 6.2 Hz, 3H). |
| 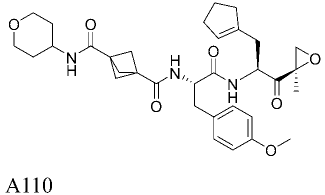 | LCMS-A110: (ESI, *m*/*z*):594.10, [M+H]⁺. ¹H NMR-A110: (400 MHz, Chloroform-d) *δ* 7.17 (d, *J* = 8.5 Hz, 2H), 6.84 (d, *J* = 8.6 Hz, 2H), 6.16 (d, *J* = 7.5 Hz, 1H), 5.76 (d, *J* = 6.4 Hz, 2H), 5.31 (d, *J* = 8.0 Hz, 1H), 5.26 (s, 1H), 4.57 - 4.50 (m, 1H), 4.49 - 4.44 (m, 1H), 3.97 (d, *J* = 3.7 Hz, 1H), 3.95 (s, 1H), 3.79 (s, 3H), 3.51 - 3.43 (m, 2H), 3.26 (d, *J* = 5.0 Hz, 1H), 3.04 (dd, *J* = 13.8, 5.5 Hz, 1H), 2.93 - 2.86 (m, 2H), 2.49 (d, *J* = 14.5 Hz, 1H), 2.18 (s, 6H), 2.13 (t*, J =* 8.1 Hz, 3H), 1.90 (s, 1H), 1.86 (s, 1H), 1.81 (q, *J* = 7.1 Hz, 2H), 1.52 (s, 4H), 1.48 (d, *J* = 4.6 Hz, 1H), 1.44 (d, *J* = 4.5 Hz, 1H). |
| 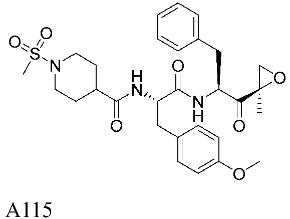 | LCMS-A115: (ESI, *m*/*z*):572.10 [M+H]⁺. ¹H NMR-A115: (400 MHz, Chloroform-*d*) δ 7.26 - 7.19 (m, 3H), 7.14 (d, *J* = 8.3 Hz, 2H), 7.00 - 6.91 (m, 2H), 6.89 - 6.79 (m, 2H), 6.00 (d, *J* = 7.3 Hz, 1H), 5.89 (d, *J* = 7.1 Hz, 1H), 4.67 (td, *J* = 7.6, 4.8 Hz, 1H), 4.49 (q, *J* = 7.1 Hz, 1H), 3.80 (s, 3H), 3.68 (t, *J* = 12.6 Hz, 2H), 3.24 (d, *J* = 5.0 Hz, 1H), 3.08 (dd, *J* = 14.0, 4.9 Hz, 1H), 3.01 (dd, *J =* 14.0, 6.0 Hz, 1H), 2.93 (d, *J* = 4.9 Hz, 1H), 2.87 (dd, *J* = 13.9, 7.7 Hz, 1H), 2.81 2.71 (m, 5H), 2.61 (dd, *J* = 13.9, 8.2 Hz, 1H), 2.20 - 2.09 (m, 1H), 1.87 - 1.77 (m, 2H), 1.72 (td, *J* = 10.3, 5.1 Hz, 2H), 1.51 (s, 3H). |
| 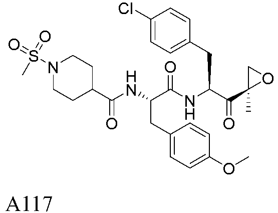 | LCMS-A117: (ESI, *m*/*z*):606.45, [M+H]⁺. ¹H NMR-A117 (400 MHz, Chloroform-d) *δ* 7.22 - 7.16 (m, 2H), 7.11 (d, *J* = 8.5 Hz, 2H), 6.92 (d, *J* = 8.1 Hz, 2H), 6.83 (d, *J* = 8.3 Hz, 2H), 5.96 (t, *J* = 8.5 Hz, 2H), 4.67 (td, *J =* 8.0, 4.8 Hz, 1H), 4.49 (q, *J* = 7.2 Hz, 1H), 3.80 (s, 3H), 3.74 - 3.62 (m, 2H), 3.21 (d, *J* = 4.9 Hz, 1H), 3.01 (ddd, *J* = 41.9, 13.3, 4.8 Hz, 3H), 2.86 (dd, *J =* 14.0, 7.7 Hz, 1H), 2.81 - 2.68 (m, 5H), 2.58 (dd, *J* = 14.1, 8.3 Hz, 1H), 2.13 (td, *J* = 10.8, 10.3, 5.0 Hz, 1H), 1.79 (dd, *J* = 8.8, 4.1 Hz, 2H), 1.73 - 1.63 (m, 2H), 1.50 (s, 3H). |
| 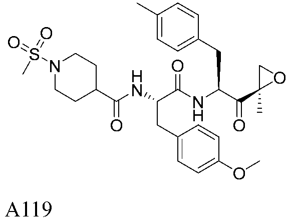 | LCMS-A119: (ESI, *m*/*z):* 585.90 [M+H]⁺. ¹H NMR-A119 (400 MHz, Chloroform-*d*) *δ* 7.14 (d, *J* = 8.2 Hz, 2H), 7.03 (d, *J* = 7.6 Hz, 2H), 6.83 (dd, *J* = 8.1, 4.9 Hz, 4H), 6.04 (d, *J* = 7.4 Hz, 1H), 5.87 (d, *J* = 6.8 Hz, 1H), 4.66 - 4.61 (m, 1H), 4.53 - 4.46 (m, 1H), 3.80 (s, 3H), 3.72 - 3.63 (m, 2H), 3.24 (d, *J* = 5.0 Hz, 1H), 3.07 - 2.97 (m, 2H), 2.93 (d, *J* = 4.9 Hz, 1H), 2.87 (dd, *J* = 14.0, 7.8 Hz, 1H), 2.76 (s, 5H), 2.56 (dd, *J* = 14.0, 8.2 Hz, 1H), 2.30 (s, 3H), 2.20 - 2.12 (m, 1H), 1.86 - 1.78 (m, 2H), 1.76 - 1.68 (m, 2H), 1.57 (d, *J* = 6.6 Hz, 3H). |
| 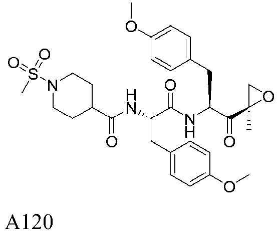 | LCMS-A120: (ESI, *m*/*z*): 602.10 [M+H]⁺. ¹H NMR-A120 (400 MHz, Chloroform-*d*) *δ* 7.17 - 7.10 (m, 2H), 6.91 - 6.81 (m, 4H), 6.79 - 6.71 (m, 2H), 6.03 - 5.83 (m, 2H), 4.65 - 4.47 (m, 2H), 3.79 (d, *J* = 10.3 Hz, 6H), 3.68 (t, *J* = 10.7 Hz, 2H), 3.23 (d, *J =* 5.0 Hz, 1H), 3.06 - 2.96 (m, 2H), 2.93 (d, *J* = 5.0 Hz, 1H), 2.91 - 2.82 (m, 1H), 2.81 - 2.68 (m, 5H), 2.61 - 2.51 (m, 1H), 2.20 - 2.10 (m, 1H), 1.87 - 1.78 (m, 2H), 1.78 - 1.64 (m, 2H), 1.51 (s, 3H). |
| 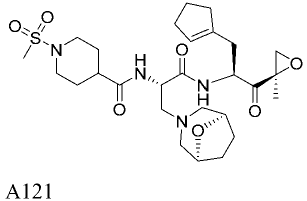 | LCMS-A121: (ESI, *m*/*z*):568.05, [M+H]⁺. ¹H NMR-A121: (400 MHz, Chloroform-d) *δ* 8.46 - 8.17 (m, 1H), 6.49 (s, 1H), 5.46 (s, 1H), 4.75 (s, 1H), 4.53 (s, 1H), 4.33 (d, *J* = 26.4 Hz, 2H), 3.78 (d, *J* = 12.0 Hz, 2H), 3.46 (d, *J* = 79.8 Hz, 2H), 3.06 (d, *J* = 11.9 Hz, 1H), 2.95 - 2.89 (m, 1H), 2.79 (d, *J* = 2.5 Hz, 5H), 2.59 (s, 3H), 2.48 (d, *J* = 11.8 Hz, 1H), 2.33 (d, *J* = 42.0 Hz, 7H), 2.08 - 1.93 (m, 4H), 1.92 - 1.80 (m, 4H), 1.51 (s, 3H). |
| 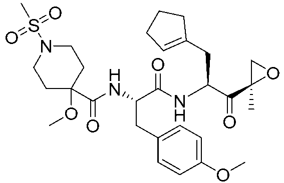 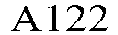 | LCMS-A122: (ESI, *m*/*z*):592.10 [M+H]⁺. ¹H NMR-A122: (400 MHz, Chloroform-d) *δ* 7.22 - 7.10 (m, 2H), 6.93 - 6.77 (m, 3H), 5.95 (d, *J* = 6.7 Hz, 1H), 5.31 (s, 1H), 4.61 - 4.46 (m, 2H), 3.78 (s, 3H), 3.65 - 3.47 (m, 2H), 3.30 - 3.23 (m, 1H), 3.05 (s, 3H), 3.03 - 2.95 (m, 3H), 2.92 (dd, *J* = 11.6, 3.9 Hz, 2H), 2.78 (s, 3H), 2.51 (d, *J* = 14.5 Hz, 1H), 2.34 - 2.19 (m, 3H), 2.19 - 2.11 (m, 2H), 2.11 - 1.94 (m, 2H), 1.92 - 1.78 (m, 3H), 1.77 - 1.68 (m, 1H), 1.51 (s, 3H). |
| 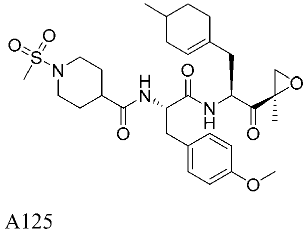 | LCMS-A125: (ESI, *m*/*z*):589.95, [M+H]⁺. ¹H NMR-A125 (400 MHz, Chloroform-*d*) *δ* 7.06 (d, *J* = 8.2 Hz, 2H), 6.82 (dd, *J* = 8.5, 1.6 Hz, 2H), 6.05 (dd, *J* = 11.4, 6.9 Hz, 1H), 5.94 (d, *J* = 7.3 Hz, 1H), 5.34 (s, 1H), 4.60 (dt*, J =* 11.3, 5.8 Hz, 1H), 4.51 (q, *J* = 7.7, 7.1 Hz, 1H), 3.78 (s, 3H), 3.74 - 3.61 (m, 2H), 3.31 (t, *J* = 4.6 Hz, 1H), 2.99 (dd, *J =* 14.0, 7.4 Hz, 1H), 2.91 (dd, *J =* 12.3, 5.8 Hz, 2H), 2.78 (s, 5H), 2.40 - 2.30 (m, 1H), 2.24 - 2.13 (m, 1H), 2.08 - 1.72 (m, 8H), 1.48 (s, 5H), 1.19 (dd, *J* = 22.1, 10.4 Hz, 1H), 0.93 (dd, *J* = 6.3, 3.2 Hz, 3H). |
| 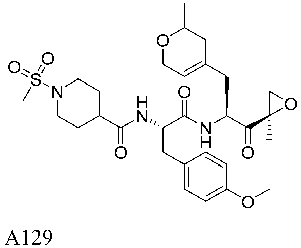 | LCMS-A129: (ESI, *m*/*z*): 592.15 [M+H]⁺. ¹H NMR-A129 (400 MHz, Chloroform-*d*) *δ* 7.23 - 7.12 (m, 2H), 6.84 (d, *J* = 7.5 Hz, 2H), 6.18 (s, 1H), 5.89 - 5.64 (m, 1H), 5.22 (d, *J* = 50.7 Hz, 1H), 4.50 (s, 2H), 4.16 - 3.89 (m, 2H), 3.79 (d, *J* = 1.6 Hz, 3H), 3.77 - 3.64 (m, 2H), 3.57 - 3.43 (m, 1H), 3.29 - 3.18 (m, 1H), 3.11 - 2.99 (m, 1H), 2.96 - 2.83 (m, 2H), 2.78 (s, 5H), 2.43 (d, *J* = 15.6 Hz, 1H), 2.17 (d, *J* = 34.3 Hz, 2H), 1.96 - 1.84 (m, 4H), 1.81 (s, 2H), 1.51 (d, *J* = 4.0 Hz, 3H), 1.24 - 1.09 (m, 3H). |
| 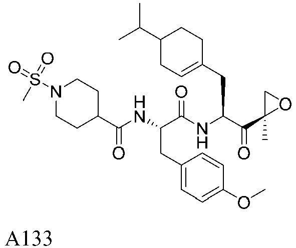 | LCMS-A133: (ESI, *m*/*z*): 618.25 [M+H]⁺. ¹H NMR-A133 (400 MHz, Chloroform-d) *δ* 7.21 (d, *J* = 8.1 Hz, 2H), 6.85 (d, *J* = 8.1 Hz, 2H), 6.23 (d, *J* = 7.1 Hz, 1H), 5.64 (t, *J* = 5.8 Hz, 1H), 5.18 (t, *J* = 6.1 Hz, 1H), 4.58 - 4.50 (m, 1H), 4.47 - 4.38 (m, 1H), 3.81 - 3.68 (m, 5H), 3.27 (d, *J =* 5.1 Hz, 1H), 3.04 (dd, *J =* 14.0, 5.5 Hz, 1H), 2.93 - 2.84 (m, 2H), 2.77 (s, 5H), 2.38 (d, *J* = 13.6 Hz, 1H), 2.21 (tt*, J =* 10.8, 4.0 Hz, 1H), 1.97 - 1.70 (m, 10H), 1.51 (s, 3H), 1.46 - 1.40 (m, 1H), 1.22 - 1.05 (m, 2H), 0.86 (dd, *J* = 6.7, 4.2 Hz, 6H). |
| 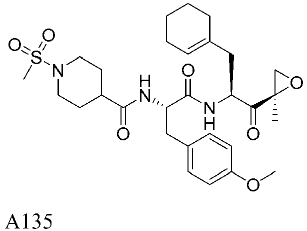 | LCMS-A135: (ESI, *m*/*z*): 576.05 [M+H]⁺. ¹H NMR-A135 (400 MHz, Chloroform-d) *δ* 7.23 - 7.18 (m, 2H), 6.93 - 6.80 (m, 2H), 6.22 (d, *J* = 7.1 Hz, 1H), 5.63 (d, *J* = 6.1 Hz, 1H), 5.19 (s, 1H), 4.62 - 4.49 (m, 1H), 4.49 - 4.39 (m, 1H), 3.79 (s, 3H), 3.77 - 3.64 (m, 2H), 3.27 (d, *J* = 5.1 Hz, 1H), 3.09 - 3.01 (m, 1H), 2.91 (d, *J* = 5.0 Hz, 1H), 2.89 - 2.83 (m, 1H), 2.81 (t, *J* = 3.0 Hz, 1H), 2.78 (s, 3H), 2.75 (t, *J =* 3.1 Hz, 1H), 2.37 (d, *J =* 13.6 Hz, 1H), 2.27 - 2.14 (m, 1H), 1.87 (d, *J* = 19.6 Hz, 6H), 1.83 - 1.77 (m, 2H), 1.77 - 1.70 (m, 1H), 1.55 (s, 2H), 1.51 (s, 3H), 1.51 - 1.38 (m, 2H). |
| 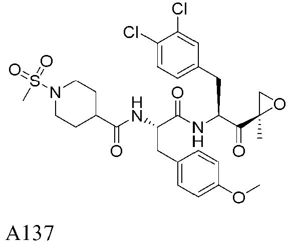 | LCMS-A137: (ESI, *m*/*z*):639.80 [M+H]⁺. ¹H NMR-A137 (400 MHz, Chloroform-*d*) δ 7.31 (d, *J* = 8.2 Hz, 1H), 7.11 (d, *J* = 8.3 Hz, 2H), 7.07 (d, *J* = 2.1 Hz, 1H), 6.89 - 6.80 (m, 3H), 6.07 (d, *J* = 7.4 Hz, 1H), 5.92 *(d, J* = 7.3 Hz, 1H), 4.70 - 6.61 (m, 1H), 4.55 - 4.45 (m, 1H), 3.80 (s, 3H), 3.76 - 3.63 (m, 2H), 3.20 (d, *J* = 4.9 Hz, 1H), 3.10 - 2.98 (m, 2H), 2.95 (d, *J* = 4.8 Hz, 1H), 2.87 (dd, *J =* 14.0, 7.7 Hz, 1H), 2.77 (s, 5H), 2.57 (dd, *J =* 14.1, 8.4 Hz, 1H), 2.20 - 2.11 (m, 1H), 1.87 - 1.76 (m, 2H), 1.75 - 1.64 (m, 2H), 1.51 (s, 3H). |
| 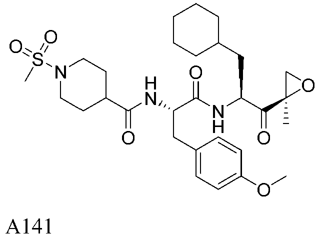 | LCMS-A141: (ESI, *m*/*z*): 578.00 [M+H]⁺. ¹H NMR-A141 (400 MHz, Chloroform-d) *δ* 7.22 - 7.11 (m, 2H), 6.93 - 6.80 (m, 2H), 6.11 (d, *J* = 7.3 Hz, 1H), 5.85 (d, *J* = 7.6 Hz, 1H), 4.58 (q, *J* = 7.0 Hz, 1H), 4.54 - 4.44 (m, 1H), 3.79 (s, 5H), 3.22 (d, *J* = 5.0 Hz, 1H), 3.06 - 2.93 (m, 2H), 2.90 (d, *J* = 5.0 Hz, 1H), 2.78 (s, 5H), 2.26 - 2.15 (m, 1H), 1.97 - 1.59 (m, 9H), 1.53 (s, 1H), 1.52 (s, 3H), 1.24 - 1.04 (m, 5H), 0.89 (p, *J* = 10.8 Hz, 2H). |
| 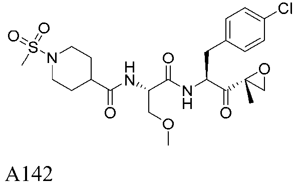 | LCMS-A142: (ESI, *m*/*z):* 530.20 [M+H]⁺. ¹H NMR-A142: (400 MHz, Methanol-*d*₄) *δ* 7.35 - 7.19 (m, 4H), 4.82 - 4.71 (m, 1H), 4.34 - 4.26 (m, 1H), 3.74 (d, *J* = 11.5 Hz, 2H), 3.60 - 3.47 (m, 1H), 3.47 - 3.36 (m, 1H), 3.32 - 3.23 (m, 2H), 3.17 - 3.09 (m, 1H), 2.98 (dt, *J =* 5.0, 2.6 Hz, 1H), 2.84 (s, 3H), 2.82 - 2.65 (m, 3H), 2.45 - 2.30 (m, 1H), 1.85 (s, 2H), 1.74 (dd, *J =* 13.7, 9.7 Hz, 2H), 1.52 - 1.45 (m, 3H), 1.29 (dd, *J =* 7.2, 2.1 Hz, 1H), 1.16 (d, *J* = 7.1 Hz, 1H). |
| 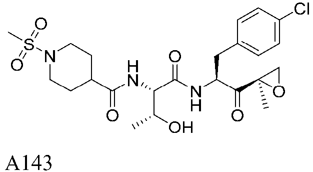 | LCMS-A143: (ESI, *m*/*z*): 530.15 [M+H]⁺. ¹H NMR-A143: (400 MHz, Methanol-*d*₄) *δ* 7.31 - 7.20 (m, 4H), 4.79 (dd, *J =* 9.0, 4.4 Hz, 1H), 4.31 - 4.24 (m, 1H), 4.07 - 4.01 (m, 1H), 3.78 - 3.73 (m, 2H), 3.28 (d*, J* = 5.0 Hz, 1H), 3.12 (dd*, J* = 13.9, 4.4 Hz, 1H), 2.98 (d, *J* = 5.0 Hz, 1H), 2.85 (s, 3H), 2.84 - 2.71 (m, 3H), 2.48 - 2.41 (m 1H), 1.95 - 1.82 (m, 2H), 1.82 - 1.69 (m, 2H), 1.47 (s, 3H), 1.35 - 1.27 (m, 1H), 1.15 (d, *J =* 6.4 Hz, 3H). |
| 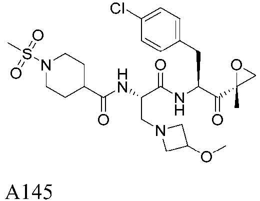 | LCMS-A145: (ESI, *m*/*z):* 585.30 [M+H]⁺. ¹H NMR-A145: (400 MHz, Methanol-*d*₄) *δ* 7.36 - 7.29 (m, 2H), 7.24 (dd, *J =* 8.6, 7.0 Hz, 2H), 4.75 (ddd, *J* = 10.1, 5.9, 4.1 Hz, 1H), 4.32 (d, *J* = 7.3 Hz, 1H), 4.00 (dt, *J* = 27.2, 5.7 Hz, 1H), 3.83 - 3.66 (m, 3H), 3.64 - 3.42 (m, 1H), 3.28 (s, 3H), 3.26 (s, 1H), 3.21 - 2.96 (m, 4H), 2.89 - 2.56 (m, 8H), 2.36 (qt, *J =* 11.6, 3.9 Hz, 1H), 1.86 (td, *J =* 17.5, 15.8, 6.9 Hz, 2H), 1.79 - 1.62 (m, 2H), 1.50 (s, 3H). |
| 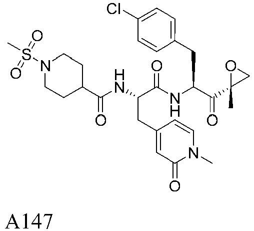 | LCMS-A147: (ESI, *m*/*z*): 607.15 [M+H]⁺. ¹H NMR-A147: (400 MHz, DMSO-*d*₆) *δ* 8.45 (d, *J =* 7.4 Hz, 1H), 7.99 (d, *J* = 8.7 Hz, 1H), 7.56 (d, *J =* 6.9 Hz, 1H), 7.39 - 7.32 (m, 2H), 7.29 - 7.23 (m, 2H), 6.18 (d, *J =* 1.8 Hz, 1H), 6.10 - 6.08 (m, 1H), 4.60 - 4.49 (m, 2H), 3.55 - 3.39 (m, 2H), 3.36 (s, 3H), 3.22 (d, *J* = 5.2 Hz, 1H), 3.02 (d, *J =* 5.2 Hz, 1H), 2.97 - 2.93 (m, 1H), 2.84 (s, 3H), 2.79 - 2.60 (m, 4H), 2.48 (d, *J =* 14.8 Hz, 1H), 2.25 - 2.17 (m, 1H), 1.69 - 1.58 (m, 2H), 1.55 - 1.41 (m, 2H), 1.38 (s, 3H). |
| 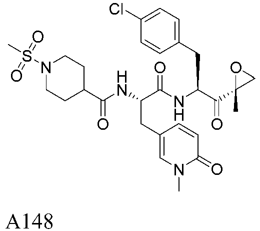 | LCMS-A148: (ESI, *m*/*z):* 607.05 [M+H]⁺. ¹H NMR-A148: (400 MHz, DMSO-*d*₆) *δ* 8.44 (d, *J =* 7.3 Hz, 1H), 7.91 (d, *J =* 8.5 Hz, 1H), 7.37 - 7.33 (m, 3H), 7.29 - 7.21 (m, 3H), 6.32 - 6.19 (m, 1H), 4.59 - 4.40 (m, 2H), 3.54 - 3.45 (m, 2H), 3.35 (s, 3H), 3.22 (d, *J =* 5.2 Hz, 1H), 3.02 (d, *J =* 5.2 Hz, 1H), 2.97 - 2.92 (m, 1H), 2.84 (s, 3H), 2.71 - 2.62 (m, 4H), 2.46 - 2.41 (m, 1H), 2.29 - 2.21(m, 1H), 1.67 - 1.62 (m, 2H), 1.55 - 1.41 (m, 2H), 1.38 (s, 3H). |
| 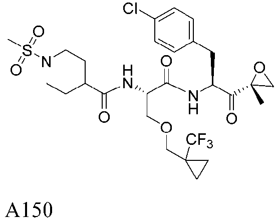 | LCMS-A150: (ESI, *m*/*z*): 638.25 [M+H]⁺. ¹H NMR-A150: (400 MHz, DMSO-*d*₆) *δ* 8.40 (d, *J =* 7.5 Hz, 1H), 7.92 (d, *J =* 8.3 Hz, 1H), 7.38 - 7.29 (m, 2H), 7.29 - 7.18 (m, 2H), 4.59 - 4.43 (m, 2H), 3.59 - 3.51 (m, 5H), 3.43 (dd, *J =* 10.2, 7.4 Hz, 1H), 3.20 (d, *J* = *5.2* Hz, 1H), 3.01 (d, *J* = 5.1 Hz, 1H), 2.93 (dd, *J* = 13.9, 4.3 Hz, 1H), 2.85 (s, 3H), 2.68 (t, *J =* 12.5 Hz, 3H), 2.30 (s, 1H), 1.72 (t*, J* = 15.7 Hz, 2H), 1.54 (t, *J =* 12.2 Hz, 2H), 1.37 (s, 3H), 0.92 (t, *J* = 2.7 Hz, 2H), 0.81 (d, *J =* 6.0 Hz, 2H). |
| 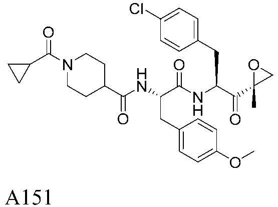 | LCMS-A151: (ESI, *m*/*z):* 596.25 [M+H]⁺. ¹H NMR-A151: (400 MHz, DMSO-*d*₆) *δ* 8.37 (d, *J =* 7.3 Hz, 1H), 7.87 (d, *J =* 8.6 Hz, 1H), 7.38 - 7.31 (m, 2H), 7.30 - 7.23 (m, 2H), 7.13 - 7.06 (m, 2H), 6.83 - 6.71 (m, 2H), 4.61 - 4.40 (m, 2H), 4.20 (d, *J =* 27.8 Hz, 2H), 3.70 (s, 3H), 3.21 (d*, J* = 5.2 Hz, 1H), 3.01 (d*, J* = 5.2 Hz, 2H), 2.96 - 2.85 (m, 2H), 2.74 - 2.57 (m, 3H), 2.44 - 2.31 (m, 1H), 1.95 - 1.89 (m, 1H), 1.65 - 1.40 (m, 2H), 1.38 (s, 3H), 1.32 (s, 2H), 0.69 - 1.64 (m, 4H). |
| 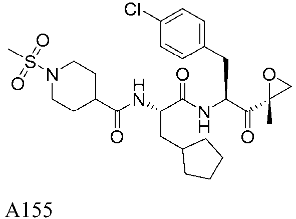 | LCMS-A155: (ESI, *m*/*z*): 568.20 [M+H]⁺. ¹H NMR-A155: (400 MHz, Methanol-*d*₄) *δ* 7.31 - 7.21 (m, 4H), 4.76 (dd, *J =* 9.6, 4.0 Hz, 1H), 4.31 (dd, *J* = 9.1, 5.9 Hz, 1H), 3.75 (dd, *J =* 12.2, 3.4 Hz, 2H), 3.27 (d, *J* = 5.0 Hz, 1H), 3.12 (dd*, J =* 14.0, 4.1 Hz, 1H), 2.98 (d, *J* = 5.0 Hz, 1H), 2.84 (s, 3H), 2.78 (tt, *J =* 12.0, 2.2 Hz, 2H), 2.70 (dd, *J* = 13.9, 9.6 Hz, 1H), 2.33 (tt*, J* = 11.4, 3.9 Hz, 1H), 1.92 - 1.52 (m, 13H), 1.48 (s, 3H), 1.16 (d, *J =* 8.6 Hz, 2H). |
| 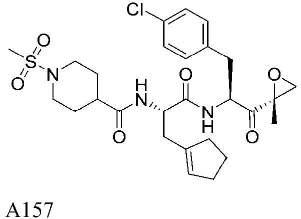 | LCMS-A157: (ESI, *m*/*z):* 566.20 [M+H]⁺. ¹H NMR-A157: (400 MHz, Methanol-*d*₄) *δ* 7.32 - 7.26 (m, 2H), 7.23 (d, *J* = 8.5 Hz, 2H), 5.41 (s, 1H), 4.75 (dd, *J* = 9.5, 4.1 Hz, 1H), 4.50 (dd, *J* = 9.5, 5.5 Hz, 1H), 3.81 - 3.69 (m, 2H), 3.26 (d, *J* = 5.0 Hz, 1H), 3.11 (dd, *J =* 14.0, 4.2 Hz, 1H), 2.98 (d, *J =* 5.0 Hz, 1H), 2.84 (s, 3H), 2.82 - 2.65 (m, 3H), 2.48 (dd, *J =* 14.7, 5.5 Hz, 1H), 2.39 - 2.21 (m, 6H), 1.94 - 1.63 (m, 6H), 1.48 (s, 3H). |
| 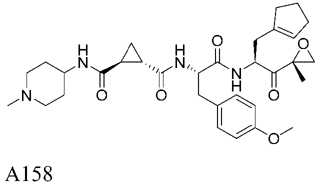 | LCMS-A158: (ESI, *m*/*z):* 581.45 [M+H]⁺. ¹H NMR-A158: (400 MHz, Methanol-*d*₄) *δ* 8.51 (d, *J* = 11.2 Hz, 1H), 7.14 (dd, *J =* 8.8, 2.3 Hz, 2H), 6.88 - 6.75 (m, 2H), 5.44 (s, 1H), 4.65 (dd, *J =* 8.8, 5.1 Hz, 1H), 4.55 (dd, *J* = 9.0, 5.8 Hz, 1H), 3.95 (s, 1H), 3.78 (d, *J* = 1.9 Hz, 3H), 3.19 (d*, J* = 5.1 Hz, 2H), 3.10 (s, 1H), 3.00 (d, *J* = 5.8 Hz, 1H), 2.93 - 2.89 (m, 1H), 2.78 (dd, *J =* 13.9, 9.0 Hz, 3H), 2.66 (s, 3H), 2.54 - 2.46 (m, 1H), 2.29 (t, *J* = 7.3 Hz, 5H), 2.15 - 1.94 (m, 4H), 1.87 (p, *J* = 7.5 Hz, 2H), 1.70 (d, *J* = 12.2 Hz, 2H), 1.45 (s, 3H), 1.20 (dd, *J =* 8.9, 5.7, 3.6 Hz, 1H), 1.13 (d, *J* = 8.8, 5.6, 3.5 Hz, 1H). |
| 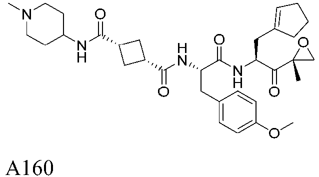 | LCMS-A160: (ESI, *m*/*z*): 595.45 [M+H]⁺. ¹H NMR-A160: (400 MHz, Methanol-*d*₄) *δ* 7.12 (d, *J =* 8.1 Hz, 2H), 6.83 (d, *J =* 8.1 Hz, 2H), 5.39 (s, 1H), 4.60 (dd, *J* = 9.2, 4.2 Hz, 2H), 3.77 (s, 4H), 3.33 (s, 3H), 3.29 (d, *J =* 6.2 Hz, 6H), 3.05 - 2.92 (m, 4H), 2.89 (d, *J* = 11.2 Hz, 1H), 2.42 - 2.17 (m, 9H), 2.11 - 1.99 (m, 2H), 1.86 (p, *J* = 7.2 Hz, 2H), 1.71 (q, *J =* 12.9, 12.3 Hz, 2H), 1.46 (s, 3H). |
| 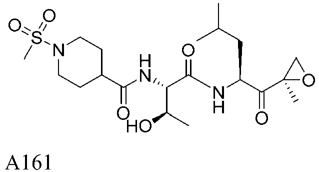 | LCMS-A161: (ESI, *m*/*z*): 462.25 [M+H]⁺. ¹H NMR-A161: (400 MHz, DMSO-*d*₆) *δ* 7.98 (d, *J =* 7.4 Hz, 1H), 7.69 (d, *J =* 8.6 Hz, 1H), 4.77 (d, *J =* 5.3 Hz, 1H), 4.44 - 4.38 (m, 1H), 4.19 (dd, *J =* 8.6, 4.8 Hz, 1H), 3.91-3.83 (m, 1H), 3.62 - 3.52 (m, 2H), 3.20 (d, *J* = 5.3 Hz, 1H), 3.01 (d, *J* = 5.2 Hz, 1H), 2.86 (s, 3H), 2.71 (td, *J* = 12.0, 2.7 Hz, 2H), 2.45 - 2.38 (m, 1H), 1.86 -1.75 (m, 2H), 1.71 - 1.50 (m, 3H), 1.41 (s, 3H), 1.37 - 1.28 (m, 2H), 1.03 (d, *J =* 6.3 Hz, 3H), 0.88 (dd, *J* = 21.0, 6.6 Hz, 6H). |
| 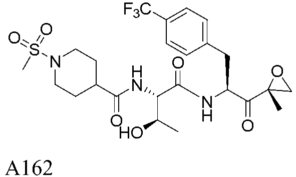 | LCMS-A162: (ESI, *m*/*z):* 564.15 [M+H]⁺. ¹H NMR-A162: (400 MHz, DMSO-*d*₆) *δ* 8.20 (d, *J =* 7.6 Hz, 1H), 7.65 (dd, *J =* 8.4, 6.5 Hz, 3H), 7.46 (d, *J =* 8.0 Hz, 2H), 4.79 (d, *J =* 5.4 Hz, 1H), 4.71 - 4.60 (m, 1H), 4.17 (dd, *J =* 8.6, 4.9 Hz, 1H), 3.85 (p, *J =* 5.8 Hz, 1H), 3.56 (d, *J =* 11.8 Hz, 2H), 3.23 (d, *J =* 5.1 Hz, 1H), 3.09 - 2.99 (m, 2H), 2.86 (s, 4H), 2.74 - 2.63 (m, 2H), 2.43 - 2.32 (m, 1H), 1.76 (dd, *J =* 32.3, 13.1 Hz, 2H), 1.60 - 1.48 (m, 2H), 1.38 (s, 3H), 0.98 (d, *J =* 6.3 Hz, 3H). |
| 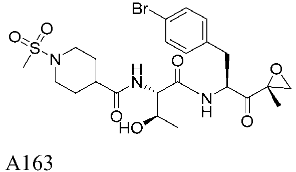 | LCMS-A163: (ESI, *m*/*z*): 574.10 [M+H]⁺. ¹H NMR-A163: (400 MHz, DMSO-*d*₆) *δ* 8.13 (d, *J =* 7.5 Hz, 1H), 7.66 (d, *J =* 8.7 Hz, 1H), 7.47 (d, *J =* 8.0 Hz, 2H), 7.19 (d, *J =* 8.1 Hz, 2H), 4.79 (d, *J* = 5.2 Hz, 1H), 4.61 - 4.56 (m, 1H), 4.18 (dd*, J* = 8.6, 4.7 Hz, 1H), 3.97 - 3.76 (m, 1H), 3.67 - 3.50 (m, 2H), 3.22 (d, *J =* 5.2 Hz, 1H), 3.00 (d, *J* = 5.2 Hz, 1H), 2.88 (d*, J* = 16.0 Hz, 4H), 2.79 - 2.63 (m, 3H), 2.51 - 2.32 (m, 1H), 1.77 (dd, *J =* 26.4, 13.1 Hz, 2H), 1.55 (q, *J* = 12.4 Hz, 2H), 1.37 (s, 3H), 0.99 (d, *J =* 6.3 Hz, 3H). |
| 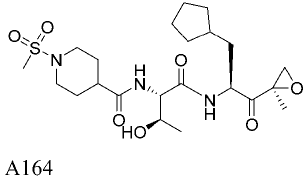 | LCMS-A164: (ESI, *m*/*z):* 488.25 [M+H]⁺. ¹H NMR-A164: (400 MHz, DMSO-*d*₆) *δ* 7.85 (dd, *J =* 115.9, 8.0 Hz, 1H), 4.79 (d, *J =* 5.3 Hz, 1H), 4.39 - 4.34 (m, 1H), 4.25 - 4.15 (m, 1H), 3.93 - 3.82 (m, 1H), 3.57 (dd*, J* = 11.4, 4.1 Hz, 2H), 3.31 (s, 1H), 3.20 (d,*J* = 5.3 Hz, 1H), 3.01 (d, *J* = 5.2 Hz, 1H), 2.86 (s, 3H), 2.80 - 2.66 (m, 2H), 2.42 (tt, *J =* 11.3, 3.7 Hz, 1H), 1.92 - 1.63 (m, 5H), 1.63 - 1.43 (m, 8H), 1.41 (s, 3H), 1.16 - 1.08 (m, 2H), 1.03 (d, *J =* 6.3 Hz, 3H). |
| 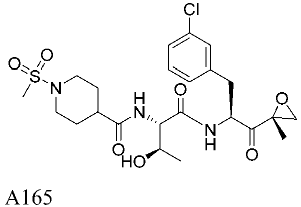 | LCMS-A165: (ESI, *m*/*z):* 530.20 [M+H]⁺. ¹H NMR-A165: (400 MHz, DMSO-*d*₆) *δ* 8.17 (d, *J =* 7.4 Hz, 1H), 7.66 (d, *J =* 8.6 Hz, 1H), 7.37 - 7.26 (m, 3H), 7.21 - 7.19 (m, 1H), 4.78 (d, *J* = 5.3 Hz, 1H), 4.64 - 4.54 (m, 1H), 4.17 (dd, *J =* 8.6, 4.9 Hz, 1H), 3.88 - 3.80 (m, 1H), 3.56 (d, *J =* 11.7 Hz, 2H), 3.24 (d, *J =* 5.2 Hz, 1H), 3.00 (d, *J* = 5.2 Hz, 1H), 2.94 (dd, *J =* 14.0, 4.8 Hz, 1H), 2.86 (s, 3H), 2.84 - 2.60 (m, 3H), 2.44 - 2.34 (m, 1H), 1.86 - 1.71 (m, 2H), 1.60 - 1.45 (m, *J =* 11.9 Hz, 2H), 1.36 (s, 3H), 0.99 (d, *J* = 6.3 Hz, 3H). |
| 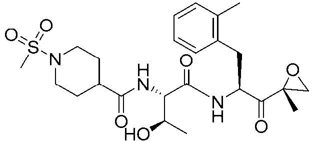 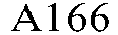 | LCMS-A166: (ESI, *m*/*z):* 510.25 [M+H]⁺. ¹H NMR-A166: (400 MHz, DMSO-*d*₆) *δ* 8.16 (d, *J =* 7.7 Hz, 1H), 7.64 (d, *J =* 8.6 Hz, 1H), 7.21 - 7.06 (m, 4H), 4.84 (d, *J* = 5.3 Hz, 1H), 4.76 - 4.66 (m, 1H), 4.18 (dd, *J =* 8.7, 4.8 Hz, 1H), 3.87 - 3.79 (m, 1H), 3.55 (s, 2H), 3.23 (d, *J =* 5.3 Hz, 1H), 3.00 - 2.89 (m, 2H), 2.86 (s, 3H), 2.70 (dd, *J =* 13.5, 9.5 Hz, 3H), 2.36 (d, *J =* 17.7 Hz, 1H), 2.33 (s, 3H), 1.83 - 1.72 (m, 2H), 1.55 (t, *J =* 12.2 Hz, 2H), 1.34 (s, 1H), 1.28 (s, 2H), 0.92 (dd, *J =* 44.5, 6.3 Hz, 3H). |
| 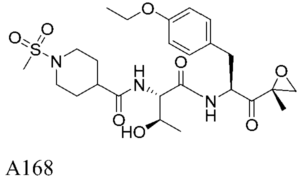 | LCMS-A168: (ESI, *m*/*z*): 540.30 [M+H]⁺. ¹H NMR-A168: (400 MHz, DMSO-*d*₆) *δ* 8.07 (d, *J =* 7.3 Hz, 1H), 7.67 (d, *J =* 8.7 Hz, 1H), 7.15 - 7.08 (m, 2H), 6.86 - 6.77 (m, 2H), 4.80 (d, *J =* 5.3 Hz, 1H), 4.56 (d, *J* = 8.0, 4.8 Hz, 1H), 4.19 (dd, *J =* 8.7, 4.7 Hz, 1H), 3.98 (q, *J* = 7.0 Hz, 2H), 3.85 (d, *J =* 11.6, 5.8 Hz, 1H), 3.56 (d, *J* = 11.6 Hz, 2H), 3.24 (dd*, J* = 20.8, 5.4 Hz, 1H), 3.00 (dd, *J =* 15.6, 5.3 Hz, 1H), 2.86 (d, *J =* 2.3 Hz, 4H), 2.75 - 2.62 (m, 3H), 2.52 - 2.39 (m, 1H), 1.77 (dd, *J =* 26.1, 13.1 Hz, 2H), 1.55 (tq, *J =* 11.6, 4.8 Hz, 2H), 1.37 (d, *J =* 17.4 Hz, 3H), 1.31 (t, *J =* 6.9 Hz, 3H), 0.94 (dd, *J* = 43.6, 6.3 Hz, 3H). |
| 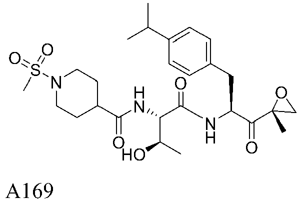 | LCMS-A169: (ESI, *m*/*z):* 538.30 [M+H]⁺. ¹H NMR-A169: (400 MHz, DMSO-*d*₆) *δ* 8.11 (d, *J =* 7.3 Hz, 1H), 7.68 (d, *J =* 8.7 Hz, 1H), 7.15 (s, 4H), 4.79 (d, *J =* 5.3 Hz, 1H), 4.58 (s, 1H), 4.19 (dd, *J* = 8.6, 4.9 Hz, 1H), 3.85 (q, *J =* 5.6 Hz, 1H), 3.56 (d, *J =* 11.7 Hz, 2H), 3.21 (d, *J* = 5.3 Hz, 1H), 2.99 (d, *J =* 5.3 Hz, 1H), 2.94 - 2.79 (m, 5H), 2.69 (dd, *J =* 13.2, 9.7 Hz, 3H), 2.39 (s, 1H), 1.77 (dd, *J* = 23.1, 13.1 Hz, 2H), 1.54 (d, *J =* 12.5 Hz, 2H), 1.35 (s, 3H), 1.19 (s, 3H), 1.17 (s, 3H), 0.98 (d, *J =* 6.3 Hz, 3H). |
| 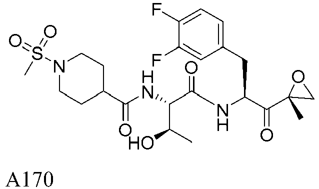 | LCMS-A170: (ESI, *m*/*z*): 532.20 [M+H]⁺. ¹H NMR-A170: (400 MHz, DMSO-*d*₆) *δ* 8.17 (d, *J =* 7.4 Hz, 1H), 7.67 (d, *J =* 8.5 Hz, 1H), 7.39 - 7.25 (m, 2H), 7.05 (s, 1H), 4.79 (d, *J =* 5.3 Hz, 1H), 4.59 (d, *J* = 8.1, 4.9 Hz, 1H), 4.17 (dd, *J =* 8.5, 4.9 Hz, 1H), 3.84 (q, *J* = 5.7 Hz, 1H), 3.56 (d, *J =* 11.7 Hz, 2H), 3.24 (d, *J =* 5.2 Hz, 1H), 3.00 (d, *J* = 5.2 Hz, 1H), 2.93 (dd*, J =* 14.0, 4.8 Hz, 1H), 2.86 (s, 3H), 2.82 - 2.74 (m, 1H), 2.73 - 2.65 (m, 2H), 2.44 - 2.34 (m, 1H), 1.78 (dd, *J =* 23.6, 13.4 Hz, 2H), 1.55 (d, *J* = 12.7 Hz, 2H), 1.35 (s, 3H), 0.99 (d, *J* = 6.3 Hz, 3H). |
| 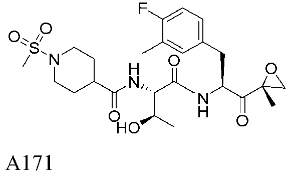 | LCMS-A171: (ESI, *m*/*z*): 528.25 [M+H]⁺. ¹H NMR-A171: (400 MHz, Chloroform-d) *δ* 7.00 (d, *J =* 7.4 Hz, 1H), 6.97 - 6.92 (m, 2H), 6.88 (d, *J* = 7.3 Hz, 1H), 6.32 (d, *J* = 7.3 Hz, 1H), 4.73 - 4.68 (m, 1H), 4.35 - 4.30 (m, 2H), 3.83 - 3.78 (m, 2H), 3.34 (d, *J* = 4.9 Hz, 1H), 3.16 - 3.11 (m, 1H), 2.98 (d, *J =* 5.0 Hz, 1H), 2.82 (s, 3H), 2.80 - 2.73 (m, 2H), 2.68 - 2.63 (m, 1H), 2.30 - 2.26 (m, 3H), 2.25 - 2.17 (m, 1H), 1.95 - 1.67 (m, 5H), 1.55 (s, 3H), 1.13 (d, *J =* 6.5 Hz, 3H). |
| 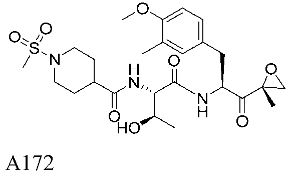 | LCMS-A172: (ESI, *m*/*z):* 540.30 [M+H]⁺. ¹H NMR-A172: (400 MHz, Chloroform-d) *δ* 6.94 (d, *J =* 7.1 Hz, 2H), 6.82 (d, *J =* 6.8 Hz, 1H), 6.79 - 6.72 (m, 1H), 6.31 (d, *J* = 7.4 Hz, 1H), 4.71 - 4.66 (m, 1H), 4.35 - 4.29 (m, 2H), 3.83 (s, 3H), 3.81 - 3.72 (m, 2H), 3.34 (d, *J* = 5.0 Hz, 1H), 3.15 - 3.10 (m, 1H), 3.03 (d, *J* = 3.4 Hz, 1H), 2.97 (d, *J* = 5.0 Hz, 1H), 2.83 - 2.71 (m, 5H), 2.66 - 2.61 (m, 1H), 2.21 (s, 4H), 1.92 - 1.87 (m, 1H), 1.87 - 1.67 (m, 2H), 1.61 (s, 1H), 1.56 (s, 3H), 1.13 (d, *J =* 6.4 Hz, 3H). |
| 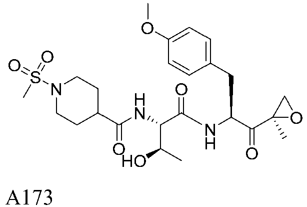 | LCMS-A173: (ESI, *m*/*z):* 526.20 [M+H]⁺. ¹H NMR-A173: (400 MHz, DMSO-*d*₆) *δ* 8.14 (dd, *J =* 63.4, 7.4 Hz, 1H), 7.70 (dd, *J =* 25.6, 8.8 Hz, 1H), 7.14 (dd*, J =* 8.6, 4.2 Hz, 2H), 6.83 (dd*, J = 8.4,* 5.7 Hz, 2H), 4.74 (dd, *J =* 49.1, 5.3 Hz, 1H), 4.61 - 4.44 (m, 1H), 4.34 - 4.15 (m, 1H), 3.94 - 3.67 (m, 4H), 3.56 (d, *J =* 11.8 Hz, 2H), 3.24 (dd, *J =* 20.5, 5.3 Hz, 1H), 3.00 (dd, *J =* 13.8, 5.3 Hz, 1H), 2.86 (s, 4H), 2.78 - 2.64 (m, 3H), 2.45 - 2.29 (m, 1H), 1.84 - 1.70 (m, 2H), 1.54 (d, *J =* 11.8 Hz, 2H), 1.37 (d, *J =* 16.1 Hz, 3H), 0.94 (dd, *J =* 47.9, 6.3 Hz, 3H). |
| 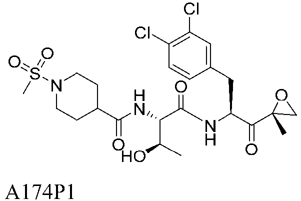 | LCMS-A174P1: (ESI, *m*/*z*): 564.10 [M+H]⁺. ¹H NMR-A174P1: (400 MHz, DMSO-*d*₆) *δ* 8.29 - 8.17 (m, 1H), 7.67 - 7.61 (m, 1H), 7.55 - 7.53 (m, 1H), 7.49 (t, *J =* 2.1 Hz, 1H), 7.24 - 7.20 (m, 1H), 6.04 (s, 1H), 4.78 - 4.69 (m, 1H), 4.64 - 4.47 (m, 1H), 4.19 - 4.15 (m, 1H), 3.82 (s, 1H), 3.57 (d, *J =* 11.6 Hz, 2H), 3.29 - 3.23 (m, 1H), 3.05 - 3.00 (m, 1H), 2.98 - 2.92 (m, 1H), 2.86 (s, 3H), 2.80 - 2.70 (m, 1H), 2.69 - 2.65 (m, 1H), 2.44 - 2.31 (m, 1H), 1.83 - 1.72 (m, 2H), 1.59 - 1.50 (m, 2H), 1.39 (d, *J =* 18.0 Hz, 3H), 0.99 - 0.86 (m, 3H). |
| 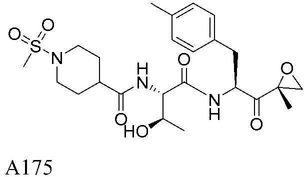 | LCMS-A175: (ESI, *m*/*z):* 510.20 [M+H]⁺. ¹H NMR-A175: (400 MHz, DMSO-*d*₆) *δ* 8.14 (dd, *J =* 60.4, 7.4 Hz, 1H), 7.70 (dd, *J =* 24.6, 8.8 Hz, 1H), 7.20 - 7.01 (m, 4H), 4.74 (dd, *J* = 49.1, 5.3 Hz, 1H), 4.63 - 4.47 (m, 1H), 4.35 - 4.15 (m, 1H), 3.93 - 3.70 (m, 1H), 3.56 (d, *J =* 11.6 Hz, 2H), 3.24 (dd, *J =* 19.3, 5.4 Hz, 1H), 3.01 (dd, *J =* 13.1, 5.3 Hz, 1H), 2.93 - 2.81 (m, 4H), 2.69 (dd, *J =* 13.5, 9.8 Hz, 3H), 2.46 - 2.32 (m, 1H), 2.26 (s, 3H), 1.77 (dd, *J =* 26.8, 13.2 Hz, 2H), 1.54 (d, *J =* 12.8 Hz, 2H), 1.38 (d, *J =* 15.8 Hz, 3H), 0.93 (dd, *J =* 48.5, 6.3 Hz, 3H). |
| 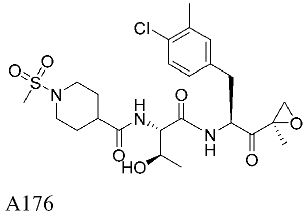 | LCMS-A176: (ESI, *m*/*z):* 544.20 [M+H]⁺. ¹H NMR-A176: (400 MHz, DMSO-*d*₆) *δ* 8.13 (d, *J =* 7.4 Hz, 1H), 7.67 (d, *J =* 8.6 Hz, 1H), 7.30 (d*, J =* 8.2 Hz, 1H), 7.19 (d, *J =* 2.2 Hz, 1H), 7.07 (dd, *J* = 8.2, 2.2 Hz, 1H), 4.80 (s, 1H), 4.63 - 4.53 (m, 1H), 4.18 (dd, *J* = 8.6, 4.9 Hz, 1H), 3.85 (p, *J =* 6.0 Hz, 1H), 3.60 - 3.53 (m, 2H), 3.22 (d, *J =* 5.2 Hz, 1H), 3.06 - 2.97 (m, 1H), 2.86 (s, 4H), 2.84 - 2.5 (m, 3H), 2.45-2.37 (m*, J =* 11.3, 3.7 Hz, 1H), 2.30 (s, 3H), 1.8 -1.75 (m, *J =* 26.0, 14.4, 3.7 Hz, 2H), 1.62 - 1.47 (m, 2H), 1.37 (s, 3H), 0.99 (d, *J =* 6.3 Hz, 3H). |
| 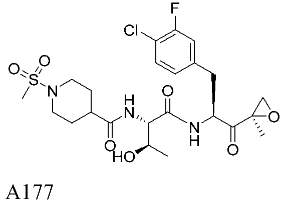 | LCMS-A177: (ESI, *m*/*z*): 548.250 [M+H]⁺. ¹H NMR-A177: (400 MHz, DMSO-*d*₆) *δ* 8.17 (d, *J =* 7.5 Hz, 1H), 7.66 (d, *J =* 8.5 Hz, 1H), 7.49 (t, *J* = 8.0 Hz, 1H), 7.28 (dd*, J* = 10.3, 1.9 Hz, 1H), 7.14 - 7.04 (m, 1H), 4.78 (d, *J =* 5.3 Hz, 1H), 4.60 (q, *J =* 7.3, 6.7 Hz, 1H), 4.16 (dd, *J =* 8.6, 4.9 Hz, 1H), 3.84 (q, *J =* 5.7 Hz, 1H), 3.56 (d, *J* = 11.6 Hz, 2H), 3.24 (d, *J =* 5.2 Hz, 1H), 3.07 - 2.92 (m, 2H), 2.90 - 2.84 (m, 3H), 2.74 (ddd, *J =* 31.9, 13.0, 9.1 Hz, 3H), 2.37 (d, *J* = 11.9 Hz, 1H), 1.77 (dd, *J =* 26.0, 13.0 Hz, 2H), 1.56 (t, *J =* 12.2 Hz, 2H), 1.37 (s, 2H), 0.99 (d, *J =* 6.3 Hz, 3H). |
| 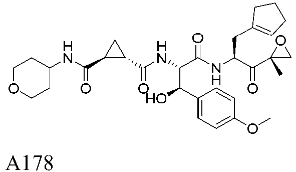 | LCMS-A178: (ESI, *m*/*z*): 584.25 [M+H]⁺. ¹H NMR-A178: (400 MHz, DMSO-*d*₆) *δ* 8.20 (dd, *J =* 17.0, 8.2 Hz, 2H), 8.07 (d, *J =* 7.9 Hz, 1H), 7.25 (d, *J* = 8.6 Hz, 2H), 6.82 (d, *J =* 8.6 Hz, 2H), 5.37 (d, *J =* 5.4 Hz, 2H), 4.86 (t, *J =* 4.6 Hz, 1H), 4.56 (q, *J =* 7.4 Hz, 1H), 4.44 (dd*, J =* 8.9, 4.2 Hz, 1H), 3.81 (d*, J =* 11.9 Hz, 3H), 3.73 (s, 3H), 3.60 (s, 1H), 3.36 (s, 1H), 3.10 (d, *J =* 5.2 Hz, 1H), 2.93 (d, *J* = 5.2 Hz, 1H), 2.47 - 2.36 (m, 1H), 2.22 (s, 4H), 2.17 (dd, *J* = 8.6, 4.7 Hz, 2H), 1.87 (dt, *J* = 9.2*,* 4.9 Hz, 1H), 1.79 (q, *J* = 7.5 Hz, 2H), 1.68 (s, 2H), 1.38 (d, *J =* 3.9 Hz, 2H), 1.34 (s, 3H), 0.96 (d, *J* = 5.7 Hz, 1H), 0.78 (d, *J =* 5.7 Hz, 1H). |
| 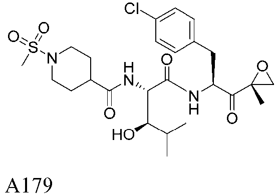 | LCMS-A179: (ESI, *m*/*z*): 558.15 [M+H]⁺. ¹H NMR-A179: (400 MHz, Methanol-*d*₄) *δ* 7.33 - 7.15 (m, 4H), 4.82 (dd, *J =* 8.4, 4.5 Hz, 1H), 4.51 (d, *J* = 3.4 Hz, 1H), 3.76 (d, *J =* 12.3 Hz, 2H), 3.60 (dd, *J* = 7.7, 3.4 Hz, 1H), 3.25 (d, *J =* 5.0 Hz, 1H), 3.12 (dd, *J* = 14.0, 4.5 Hz, 1H), 2.97 (d, *J =* 5.0 Hz, 1H), 2.88 - 2.75 (m, 6H), 2.45 (tt, *J =* 11.4, 3.8 Hz, 1H), 1.88 (t, *J* = 11.6 Hz, 2H), 1.80 - 1.67 (m, 2H), 1.61 (dq, *J =* 13.6, 6.8 Hz, 1H), 1.47 (s, 3H), 1.01 (d, *J* = 6.6 Hz, 3H), 0.96 - 0.80 (m, 3H). |
| 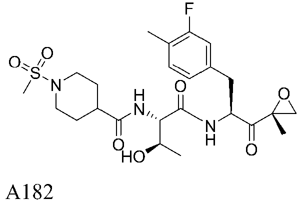 | LCMS-A182: (ESI, *m*/*z*): 528.30 [M+H]⁺. ¹H NMR-A182: (400 MHz, DMSO-*d*₆) *δ* 8.11 (d, *J =* 7.4 Hz, 1H), 7.67 (d, *J =* 8.6 Hz, 1H), 7.18 (t, *J* = 8.0 Hz, 1H), 7.05 - 6.90 (m, 2H), 4.79 (d, *J* = 5.3 Hz, 1H), 4.58 (td, *J =* 8.1, 4.6 Hz, 1H), 4.18 (dd, *J =* 8.6, 4.8 Hz, 1H), 3.85 (q, *J =* 5.7 Hz, 1H), 3.56 (dt, *J =* 12.0, 3.7 Hz, 2H), 3.23 (d, *J =* 5.2 Hz, 1H), 3.00 (d, *J* = 5.2 Hz, 1H), 2.91 (dd, *J =* 14.0, 4.6 Hz, 1H), 2.86 (s, 3H), 2.77 - 2.65 (m, 3H), 2.43 - 2.34 (m, 1H), 2.19 (d, *J* = 1.9 Hz, 3H), 1.77 (dd, *J* = 24.6, 13.1 Hz, 2H), 1.54 (dq, *J =* 12.5, 6.2, 4.6 Hz, 2H), 1.36 (s, 3H), 0.99 (d, *J =* 6.3 Hz, 3H). |
| 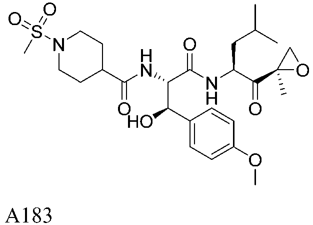 | LCMS-A183: (ESI, *m*/*z*): 554.15 [M+H]⁺. ¹H NMR-A183: (400 MHz, DMSO-*d*₆) *δ* 7.84 (dd, *J =* 60.7, 8.7 Hz, 2H), 7.31 - 7.21 (m, 2H), 6.91 - 6.78 (m, 2H), 5.46 (d, *J =* 5.1 Hz, 1H), 5.03 - 4.92 (m, 1H), 4.49 (ddt, *J =* 12.7, 9.2, 4.4 Hz, 2H), 3.72 (s, 3H), 3.50 (d*, J* = 12.0 Hz, 1H), 3.42 - 3.34 (m, 1H), 3.14 (d, *J =* 5.1 Hz, 1H), 2.99 (d, *J* = 5.1 Hz, 1H), 2.83 (s, 3H), 2.68 (qd, *J =* 11.5, 2.8 Hz, 2H), 2.43 - 2.32 (m, 1H), 1.79 - 1.57 (m, 3H), 1.54 - 1.45 (m, 1H), 1.40 (s, 3H), 1.38 - 1.21 (m, 3H), 0.89 (dd, *J* = 19.4, 6.6 Hz, 6H). |
| 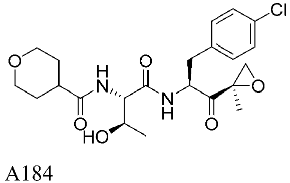 | LCMS-A184: (ESI, *m*/*z*): 453.15 [M+H]⁺. ¹H NMR-A184: (400 MHz, DMSO-*d*₆) *δ* 8.10 (d, *J* = 7.5 Hz, 1H), 7.55 (d, *J* = 8.6 Hz, 1H), 7.39 - 7.30 (m, 2H), 7.28 - 7.20 (m, 2H), 4.78 (d, *J* = 5.4 Hz, 1H), 4.59 (td, *J* = 8.2, 4.7 Hz, 1H), 4.16 (dd, *J* = 8.6, 4.7 Hz, 1H), 3.93 - 3.80 (m, 3H), 3.32 - 3.25 (m, 3H), 3.22 (d, *J* = 5.2 Hz, 1H), 3.00 (d, *J* = 5.2 Hz, 1H), 2.93 (dd, *J* = 13.9, 4.7 Hz, 1H), 2.75 (dd, *J* = 13.9, 8.7 Hz, 1H), 1.55 (ddt, *J =* 12.3, 8.8, 4.0 Hz, 4H), 1.36 (s, 3H), 0.98 (d, *J =* 6.3 Hz, 3H). |
| 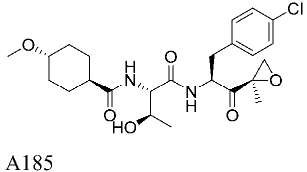 | LCMS-A185: (ESI, *m*/*z*): 481.20 [M+H]⁺ |
| | ¹H NMR-A185 (400 MHz, DMSO-*d*₆) *δ* 8.05 (d, *J =* 7.6 Hz, 1H), 7.49 (d, *J* = 8.7 Hz, 1H), 7.37 - 7.30 (m, 2H), 7.27 - 7.20 (m, 2H), 4.75 (d, *J* = 5.4 Hz, 1H), 4.59 (m, 1H), 4.14 (dd, *J =* 8.7, 4.6 Hz, 1H), 3.84 (h, *J =* 6.1 Hz, 1H), 3.23 (m, 4H), 3.06 (tt, *J =* 10.6, 4.1 Hz, 1H), 3.00 (d, *J* = 5.1 Hz, 1H), 2.93 (dd, *J =* 13.9, 4.7 Hz, 1H), 2.80 - 2.66 (m, 1H), 2.21 - 2.19 (m, 1H), 2.01 (dd, *J* = 11.9, 4.4 Hz, 2H), 1.80 - 1.62 (m, 2H), 1.42 - 1.22 (m, 5H), 1.07 (q, *J* = 12.0 Hz, 2H), 0.97 (d, *J =* 6.3 Hz, 3H). |
| 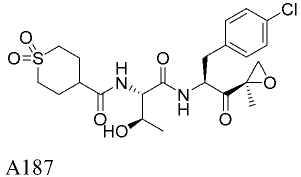 | LCMS-A187: (ESI, m/z): 501.05 [M+H]⁺ |
| | ¹H NMR-A187 (400 MHz, Methanol-*d*₄) *δ* 7.32 - 7.20 (m, 4H), 4.79 (dd, *J =* 9.0, 4.4 Hz, 1H), 4.26 (d, *J =* 4.8 Hz, 1H), 4.02 (qd, *J =* 6.3, 4.8 Hz, 1H), 3.28 (d, *J =* 4.9 Hz, 5H), 3.14 (tdd, *J =* 13.9, 6.6, 3.3 Hz, 5H), 2.98 (d, *J =* 4.9 Hz, 1H), 2.75 (dd, *J =* 14.0, 9.0 Hz, 1H), 2.63 (tq, *J =* 9.9, 5.6, 5.2 Hz, 1H), 2.29 - 2.10 (m, 4H), 1.47 (s, 3H), 1.15 (d, *J =* 6.3 Hz, 3H). |
| 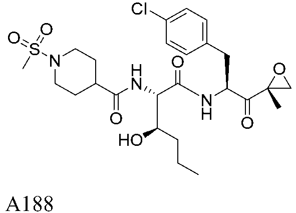 | LCMS-A188: (ESI, *m*/*z*):558.20 [M+H]⁺ |
| | ¹H NMR-A188: ¹H NMR (400 MHz, DMSO-*d*₆) δ 8.07 (d, *J* = 7.6 Hz, 1H), 7.64 (d*, J =* 8.8 Hz, 1H), 7.37 - 7.30 (m, 2H), 7.28 - 7.19 (m, 2H), 4.69 (d, *J =* 6.2 Hz, 1H), 4.62 (dd, *J =* 8.3, 4.8 Hz, 1H), 4.22 (dd, *J* = 8.8, 4.1 Hz, 1H), 3.72 (d, *J* = 7.3 Hz, 1H), 3.57 (d, *J* = 11.7 Hz, 2H), 3.22 (d, *J =* 5.2 Hz, 1H), 3.00 (d, *J =* 5.1 Hz, 1H), 2.95 (d, *J =* 4.8 Hz, 1H), 2.87 (s, 3H), 2.81 - 2.65 (m, 3H), 2.44 - 2.32 (m, 1H), 1.85 - 1.69 (m, 2H), 1.55 (q, *J =* 11.8 Hz, 2H), 1.36 (s, 4H), 1.31 - 1.15 (m, 3H), 0.84 (t, *J* = 7.0 Hz, 3H). |
| 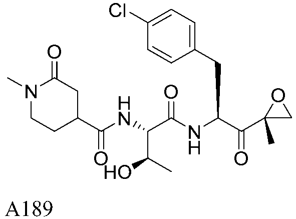 | LCMS-A189: (ESI, *m*/*z*):480.15 [M+H]⁺ |
| | ¹H NMR-A189 (400 MHz, DMSO-*d*₆) *δ* 8.17 (d, *J* = 7.4 Hz, 1H), 7.78 (d, *J* = 8.6 Hz, 1H), 7.38 - 7.30 (m, 2H), 7.30 - 7.18 (m, 2H), 4.79 (d*, J =* 5.2 Hz, 1H), 4.58 (td, *J* = 8.0, 7.4 Hz, 1H), 4.19 (dd, *J* = 8.6, 4.8 Hz, 1H), 3.86 (h, *J =* 5.9 Hz, 1H), 3.30 - 3.20 (m, 3H), 3.07 - 2.87 (m, 2H), 2.84 - 2.62 (m, 5H), 2.33 - 2.23 (m, 2H), 1.98 - 1.89 (m, 1H), 1.82 - 1.68 (m, 1H), 1.36 (s, 3H), 0.99 (d, *J* = 6.3 Hz, 3H) |
| 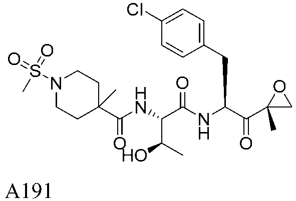 | LCMS-A191: (ESI, *m*/*z*):544.25 [M+H]⁺ |
| | ¹H NMR-A191 (400 MHz, DMSO-*d*₆) *δ* 8.14 (d, *J* = 7.5 Hz, 1H), 7.37 - 7.28 (m, 2H), 7.26 (s, 2H), 7.18 (d, *J* = 8.1 Hz, 1H), 4.82 (d, *J =* 6.3 Hz, 1H), 4.60 (d, *J =* 8.2 Hz, 1H), 4.19 (dd, *J =* 8.3, 5.0 Hz, 1H), 3.93 - 3.86 (m, 1H), 3.26 (d, *J =* 11.7 Hz, 2H), 3.19 (d, *J* = 5.2 Hz, 1H), 3.00 (d*, J =* 5.1 Hz, 1H), 2.95 - 2.82 (m, 2H), 2.78 (s, 4H), 2.08 (s, 2H), 1.48 - 1.38 (m, 2H), 1.36 (s, 3H), 1.13 (s, 3H), 1.02 - 0.86 (m, 3H). |
| 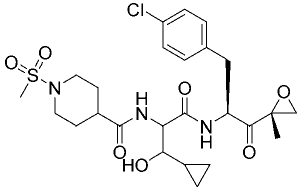 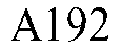 | LCMS-A192: (ESI, *m*/*z*): 556.15 [M+H]⁺ |
| | ¹H NMR-A192 (400 MHz, DMSO-*d*₆) δ 8.09 (d, *J =* 7.8 Hz, 1H), 7.69 (d*, J =* 8.8 Hz, 1H), 7.40 - 7.29 (m, 2H), 7.28 - 7.14 (m, 2H), 4.82 (d, *J* = 5.5 Hz, 1H), 4.62 (td, J = 8.2, 5.0 Hz, 1H), 4.36 (dd, *J* = 8.8, 4.0 Hz, 1H), 3.61 - 3.51 (m, 2H), 3.21 (d, *J* = 5.2 Hz, 1H), 3.16 (dd, *J* = 7.9, 4.2 Hz, 1H), 3.01 (dd, *J* = 11.6, 5.1 Hz, 1H), 2.92 (dd, *J =* 13.9, 5.0 Hz, 1H), 2.87 (s, 3H), 2.81 - 2.64 (m, 3H), 2.42 (tt, *J =* 11.3, 3.8 Hz, 1H), 1.85 - 1.71 (m, 2H), 1.55 (qt, *J =* 11.8, 3.6 Hz, 2H), 1.37 (d, *J =* 15.8 Hz, 3H), 0.86 - 0.70 (m, 1H), 0.36 (q, *J =* 5.9 Hz, 1H), 0.27 (h, *J =* 6.5, 6.0 Hz, 2H), 0.17 - 0.07 (m, 1H). |
| 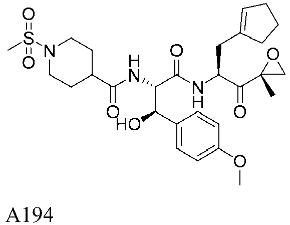 | LCMS-A194: (ESI, *m*/*z*): 578.2 [M+H]⁺ |
| | ¹H NMR-A194 (400 MHz, DMSO-*d*₆) δ 7.97 (d, J = 7.8 Hz, 1H), 7.75 (d, J = 9.2 Hz, 1H), 7.23 (d, J = 8.6 Hz, 2H), 6.82 (d, J = 8.7 Hz, 2H), 5.46 (d, J = 5.1 Hz, 1H), 5.40 (s, 1H), 4.93 (t, J = 4.4 Hz, 1H), 4.59 (dd, J = 13.9, 7.5 Hz, 1H), 4.45 (dd, J = 9.1, 3.5 Hz, 1H), 3.71 (s, 3H), 3.49 (d, J = 11.9 Hz, 1H), 3.38 (d, J = 12.4 Hz, 1H), 3.14 (d, J = 5.2 Hz, 1H), 2.95 (d, J = 5.1 Hz, 1H), 2.82 (s, 3H), 2.69 (dd, J = 11.8, 2.7 Hz, 1H), 2.64 (dd, J = 11.5, 2.5 Hz, 1H), 2.39 (ddd, J = 9.4, 7.5, 4.0 Hz, 2H), 2.29 - 2.15 (m, 5H), 1.85 - 1.74 (m, 2H), 1.71 (d, J = 11.2 Hz, 1H), 1.60 (d, J = 12.6 Hz, 1H), 1.54 - 1.43 (m, 1H), 1.36 (s, 3H), 1.27 (d, J = 31.7 Hz, 1H). |
| 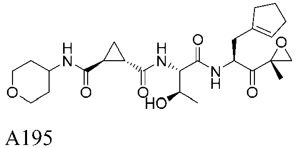 | LCMS-A195: (ESI, *m*/*z*): 492.2 [M+H]⁺ |
| | ¹H NMR-A195 (400 MHz, DMSO-*d*₆) δ 8.19 (dd, J = 9.9, 8.2 Hz, 2H), 8.06 (d, J = 7.0 Hz, 1H), 5.40 (s, 1H), 4.85 (d, J = 4.6 Hz, 1H), 4.50 (d, J = 6.6 Hz, 1H), 4.22 (dd, J = 8.5, 5.2 Hz, 1H), 3.78 (t, J = 17.7 Hz, 4H), 3.36 (s, 1H), 3.30 (s, 1H), 3.24 (d, J = 5.3 Hz, 1H), 2.99 (d, J = 5.3 Hz, 1H), 2.43 - 2.37 (m, 1H), 2.27 - 2.13 (m, 6H), 1.95 - 1.90 (m, 1H), 1.83 - 1.75 (m, 2H), 1.69 (dd, J = 8.3, 4.0 Hz, 2H), 1.42 - 1.33 (m, 5H), 1.05 - 0.96 (m, 5H). |
| 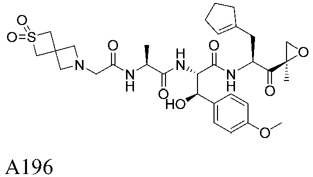 | LCMS-A196: (ESI, *m*/*z*): 647.3 [M+H]⁺ |
| | ¹H NMR-A196 (400 MHz, DMSO-*d*₆) δ 7.92 (d, *J =* 7.8 Hz, 1H), 7.75 (t, *J =* 7.9 Hz, 2H), 7.23 (d, *J =* 8.7 Hz, 2H), 6.80 (d, *J =* 8.7 Hz, 2H), 5.54 (d, *J =* 4.6 Hz, 1H), 5.39 (s, 1H), 4.89 (t*, J* = 4.1 Hz, 1H), 4.56 (dd*, J =* 13.9, 7.5 Hz, 1H), 4.37 (dd, *J* = 8.7, 3.7 Hz, 1H), 4.32 (d, *J =* 9.8 Hz, 5H), 3.72 (s, 3H), 3.43 (s, 3H), 3.29 (s, 1H), 3.12 (d, *J =* 5.1 Hz, 1H), 3.04 (s, 2H), 2.95 (d, *J =* 5.1 Hz, 1H), 2.41 (dd*, J =* 14.1, 5.5 Hz, 1H), 2.29 - 2.14 (m, 5H), 1.79 (dd*, J =* 14.7, 7.4 Hz, 2H), 1.35 (s, 3H), 1.13 (d, *J =* 7.0 Hz, 3H). |
| 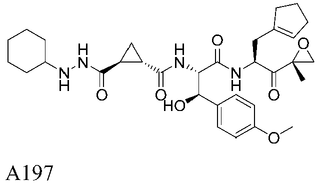 | LCMS-A197: (ESI, *m*/*z*): 597.4 [M+H]⁺ |
| | ¹H NMR-A197 (400 MHz, DMSO-*d*₆) δ 9.53 (d, J = 6.0 Hz, 1H), 8.26 (d, J = 8.8 Hz, 1H), 8.05 (d, J = 7.4 Hz, 1H), 7.24 (d, J = 8.6 Hz, 2H), 6.81 (d, J = 8.6 Hz, 2H), 5.37 (d, J = 4.8 Hz, 2H), 4.84 (t, J = 4.7 Hz, 1H), 4.66 - 4.60 (m, 1H), 4.59 - 4.50 (m, 1H), 4.43 (dd, J = 8.9, 4.3 Hz, 1H), 3.72 (s, 3H), 3.09 (d, J = 5.2 Hz, 1H), 2.92 (d, J = 5.1 Hz, 1H), 2.57 (dd, J = 11.2, 3.5 Hz, 1H), 2.39 (d, J = 8.9 Hz, 1H), 2.24 - 2.16 (m, 5H), 1.86 - 1.64 (m, 7H), 1.52 (d, J = 5.8 Hz, 1H), 1.35 (d, J = 17.6 Hz, 3H), 1.27 - 0.89 (m, 7H), 0.85 - 0.76 (m, 1H). |
| 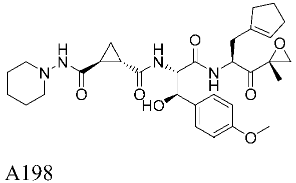 | LCMS-A198: (ESI, *m*/*z*): 583.3 [M+H]⁺ |
| | ¹H NMR-A198 (400 MHz, DMSO-*d*₆) δ 8.81 (d, J = 208.9 Hz, 1H), 8.22 (dd, J = 17.1, 8.9 Hz, 1H), 8.11 - 7.94 (m, 1H), 7.24 (d, J = 8.7 Hz, 2H), 6.81 (d, J = 8.7 Hz, 2H), 5.37 (s, 2H), 4.84 (d, J = 4.4 Hz, 1H), 4.56 (q, J = 7.4 Hz, 1H), 4.50 - 4.31 (m, 1H), 3.72 (s, 3H), 3.09 (t, J = 5.3 Hz, 1H), 2.94 - 2.87 (m, 1H), 2.71 - 2.56 (m, 3H), 2.53 (d, J = 5.6 Hz, 1H), 2.49 - 2.34 (m, 2H), 2.29 - 2.09 (m, 6H), 1.87 - 1.68 (m, 3H), 1.52 (s, 4H), 1.33 (d, J = 2.7 Hz, 4H), 0.88 (ddd, J = 55.2, 25.6, 4.1 Hz, 2H). |
| 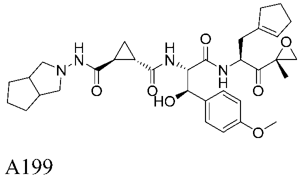 | LCMS-A199: (ESI, *m*/*z*): 609.3 [M+H]⁺ |
| | ¹H NMR-A199 (400 MHz, DMSO-*d*₆) δ 8.65 (d, J = 232.6 Hz, 1H), 8.21 (dd, J = 16.2, 9.2 Hz, 1H), 8.05 (t, J = 8.4 Hz, 1H), 7.28 - 7.19 (m, 2H), 6.81 (d, J = 8.7 Hz, 2H), 5.37 (d, J = 4.7 Hz, 2H), 4.84 (s, 1H), 4.56 (t, J = 6.5 Hz, 1H), 4.42 (dd, J = 8.4, 4.2 Hz, 1H), 3.72 (s, 3H), 3.10 (d, J = 4.6 Hz, 1H), 2.97 - 2.90 (m, 2H), 2.65 - 2.51 (m, 1H), 2.49 - 2.37 (m, 5H), 2.27 - 2.08 (m, 6H), 1.84 - 1.68 (m, 3H), 1.59 (dd, J = 17.3, 7.5 Hz, 3H), 1.50 - 1.35 (m, 2H), 1.33 (s, 4H), 1.00 - 0.70 (m, 2H). |
| 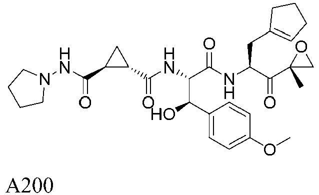 | LCMS-A200: (ESI, *m*/*z*): 569.3 [M+H]⁺ |
| | ¹H NMR-A200 (400 MHz, DMSO-*d*₆) δ 9.06 - 8.43 (m, 1H), 8.22 (t, J = 8.5 Hz, 1H), 8.04 (dd, J = 22.6, 7.9 Hz, 1H), 7.24 (d, J = 8.6 Hz, 2H), 6.81 (d, J = 8.6 Hz, 2H), 5.37 (s, 2H), 4.84 (s, 1H), 4.56 (dd, J = 14.2, 7.4 Hz, 1H), 4.43 (dd, J = 8.9, 4.1 Hz, 1H), 3.71 (d, J = 6.2 Hz, 3H), 3.09 (t, J = 4.8 Hz, 1H), 2.92 (d, J = 5.2 Hz, 1H), 2.76 (s, 4H), 2.65 - 2.53 (m, 1H), 2.43 - 2.35 (m, 1H), 2.17 (ddd, J = 13.7, 11.1, 6.2 Hz, 6H), 1.75 (ddd, J = 21.6, 10.6, 5.1 Hz, 6H), 1.33 (d, J = 2.1 Hz, 3H), 0.97 - 0.75 (m, 2H). |
| 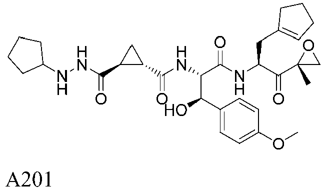 | LCMS-A201: (ESI, *m*/*z*): 583.4 [M+H]⁺ |
| | ¹H NMR-A201 (400 MHz, DMSO-*d*₆) δ 9.57 (d, J = 5.8 Hz, 1H), 8.26 (d, J = 8.7 Hz, 1H), 8.06 (d, J = 8.0 Hz, 1H), 7.24 (d, J = 8.7 Hz, 2H), 6.81 (d, J = 8.7 Hz, 2H), 5.37 (s, 2H), 4.84 (dd, J = 10.1, 5.7 Hz, 1H), 4.63 (dd, J = 9.7, 4.9 Hz, 1H), 4.59 - 4.51 (m, 1H), 4.43 (dd, J = 8.7, 4.2 Hz, 1H), 3.72 (s, 3H), 3.09 (d, J = 5.1 Hz, 1H), 2.92 (d, J = 5.0 Hz, 1H), 2.40 (dd, J = 14.9, 4.4 Hz, 1H), 2.27 - 2.12 (m, 6H), 1.86 - 1.71 (m, 3H), 1.68 - 1.36 (m, 9H), 1.33 (s, 3H), 0.96 (s, 1H), 0.81 (s, 1H). |
| 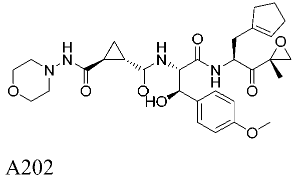 | LCMS-A202: (ESI, *m*/*z*): 585.3 [M+H]⁺ |
| | ¹H NMR-A202 (400 MHz, DMSO-*d*₆) δ 9.23 (s, 1H), 8.67 (s, 1H), 8.22 (dd, J = 23.3, 8.8 Hz, 1H), 8.05 (dd, J = 13.0, 7.9 Hz, 1H), 7.24 (dd, J = 8.8, 2.4 Hz, 2H), 6.89 - 6.76 (m, 2H), 5.37 (s, 2H), 4.85 (d, J = 4.0 Hz, 1H), 4.56 (d, J = 6.9 Hz, 1H), 4.43 (dd, J = 8.9, 4.2 Hz, 1H), 3.72 (s, 3H), 3.69 - 3.48 (m, 4H), 3.09 (t, J = 4.1 Hz, 1H), 2.94 (dd, J = 12.0, 5.8 Hz, 1H), 2.75 - 2.69 (m, 3H), 2.64 - 2.52 (m, 2H), 2.48 - 2.35 (m, 1H), 2.18 (ddd, J = 12.5, 11.0, 4.2 Hz, 6H), 1.81 - 1.74 (m, 2H), 1.34 (s, 3H), 1.02 - 0.84 (m, 2H). |
| 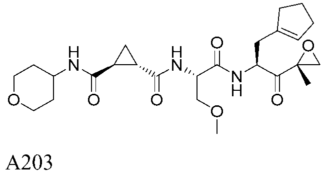 | LCMS-A203: (ESI, *m*/*z*): 492.2 [M+H]⁺ |
| | ¹H NMR-A203 (400 MHz, DMSO-*d*₆) δ 8.45 (d, J = 8.1 Hz, 1H), 8.26 (d, J = 7.0 Hz, 1H), 8.20 (d, J = 7.5 Hz, 1H), 5.39 (s, 1H), 4.56 - 4.42 (m, 2H), 3.86 - 3.68 (m, 3H), 3.44 (dd, J = 9.9, 4.8 Hz, 1H), 3.37 (dd, J = 9.8, 7.4 Hz, 5H), 3.30 (s, 2H), 3.22 (d, J = 4.9 Hz, 3H), 3.19 (d, J = 5.2 Hz, 1H), 2.99 (d, J = 5.3 Hz, 1H), 2.38 (d, J = 14.2 Hz, 1H), 2.29 - 2.14 (m, 5H), 2.13 - 2.05 (m, 1H), 1.96 - 1.87 (m, 1H), 1.86 - 1.74 (m, 2H), 1.68 (d, J = 11.1 Hz, 2H), 1.46 - 1.30 (m, 5H), 1.06 - 0.94 (m, 2H). |
| 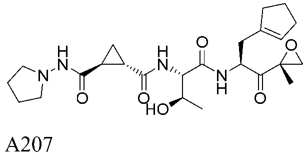 | LCMS-A207: (ESI, *m*/*z*): 477.2 [M+H]⁺ |
| | ¹H NMR-A207 (400 MHz, DMSO-*d*₆) δ 8.78 (d, *J* = 235.3 Hz, 1H), 8.18 (t, *J =* 8.4 Hz, 1H), 8.05 (dd, *J =* 12.4, 7.0 Hz, 1H), 5.40 (s, 1H), 4.85 (dd, *J =* 7.1, 5.1 Hz, 1H), 4.50 (dd, *J =* 12.5, 7.9 Hz, 1H), 4.22 (dd*, J* = 8.5, 5.2 Hz, 1H), 3.78 (dd*, J =* 11.4, 5.2 Hz, 1H), 3.24 (d, *J* = 5.5 Hz, 1H), 2.99 (d, *J* = 5.3 Hz, 1H), 2.77 (s, 4H), 2.39 (d, *J =* 12.7 Hz, 1H), 2.18 (ddd, *J =* 18.0, 12.4, 6.6 Hz, 6H), 1.88 - 1.66 (m, 7H), 1.37 (s, 3H), 1.07 - 0.94 (m, 5H). |
| 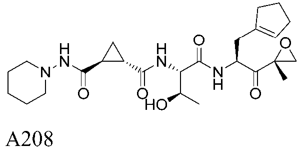 | LCMS-A208: (ESI, *m*/*z*): 491.3 [M+H]⁺ |
| | ¹H NMR-A208 (400 MHz, DMSO-*d*₆) δ 8.82 (d, *J* = 205.3 Hz, 1H), 8.17 (dd, *J =* 14.8, 8.6 Hz, 1H), 8.05 (t, *J =* 6.2 Hz, 1H), 5.40 (s, 1H), 4.84 (dd*, J =* 13.1, 5.1 Hz, 1H), 4.57 - 4.44 (m, 1H), 4.28 - 4.17 (m, 1H), 3.78 (dd*, J =* 11.4, 5.4 Hz, 1H), 3.23 (t, *J =* 5.5 Hz, 1H), 2.99 (d, *J =* 5.3 Hz, 1H), 2.69 - 2.63 (m, 3H), 2.54 (dd, *J =* 7.0, 4.4 Hz, 1H), 2.43 - 2.37 (m, 1H), 2.31 - 2.10 (m, 7H), 1.86 - 1.74 (m, 3H), 1.58 - 1.48 (m, 4H), 1.37 (s, 3H), 1.34 - 1.30 (m, 1H), 1.06 - 0.95 (m, 5H). |
| 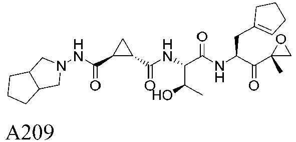 | LCMS-A209: (ESI, m/z): 517.4 [M+H]⁺ |
| | ¹H NMR-A209 (400 MHz, DMSO-*d*₆) δ 8.67 (d, *J* = 236.4 Hz, 1H), 8.17 (dd, *J =* 15.4, 8.6 Hz, 1H), 8.05 (t, *J* = 7.4 Hz, 1H), 5.40 (s, 1H), 4.86 (s, 1H), 4.50 (dd, *J =* 13.8, 6.7 Hz, 1H), 4.28 - 4.16 (m, 1H), 3.84 - 3.72 (m, 1H), 3.24 (d, *J* = 5.3 Hz, 1H), 2.97 (dd, *J* = 14.0, 5.9 Hz, 2H), 2.65 (dd, *J =* 8.6, 5.4 Hz, 1H), 2.41 (dd, *J =* 21.8, 6.0 Hz, 3H), 2.22 (s, 5H), 2.16 - 2.11 (m, 1H), 1.87 - 1.72 (m, 3H), 1.60 (s, 3H), 1.52 - 1.23 (m, 7H), 1.14 - 0.90 (m, 6H). |
| 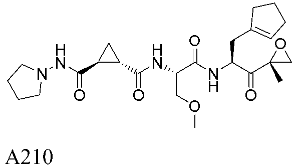 | LCMS-A210: (ESI, *m*/*z*): 477.4 [M+H]⁺ |
| | ¹H NMR-A210 (400 MHz, DMSO-*d*₆) δ 9.50 - 8.40 (m, 2H), 8.25 (dd, *J =* 22.0, 7.1 Hz, 1H), 5.39 (s, 1H), 4.67 - 4.35 (m, 2H), 3.47 - 3.41 (m, 2H), 3.24 - 3.17 (m, 4H), 2.99 (d, *J =* 5.3 Hz, 1H), 2.84 (s, 4H), 2.40 - 2.33 (m, 1H), 2.28 - 2.10 (m, 6H), 1.85 - 1.63 (m, 7H), 1.38 (d, *J* = 8.3 Hz, 3H), 1.02 (dt, *J =* 11.2, 5.6 Hz, 2H). |
| 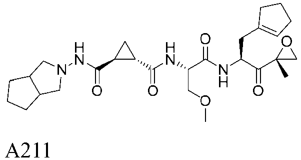 | LCMS-A211: (ESI, *m*/*z):* 517.3 [M+H]⁺ |
| | ¹H NMR-A211 (400 MHz, DMSO-*d*₆) δ 9.02 - 8.35 (m, 2H), 8.30 - 8.18 (m, 1H), 5.39 (s, 1H), 4.60 - 4.43 (m, 2H), 3.44 (dd, *J =* 10.0, 4.6 Hz, 1H), 3.39 (d, *J =* 4.0 Hz, 1H), 3.37 (s, 2H), 3.29 (s, 1H), 3.22 (s, 3H), 3.19 (d, *J =* 5.2 Hz, 1H), 2.97 (dd, *J =* 14.5, 5.8 Hz, 2H), 2.43 (dd, *J =* 15.4, 6.6 Hz, 3H), 2.20 (dt, *J =* 16.1, 8.2 Hz, 5H), 2.11 - 2.05 (m, 1H), 1.87 - 1.73 (m, 3H), 1.60 (s, 3H), 1.48 - 1.33 (m, 6H), 1.08 - 0.94 (m, 2H). |
| 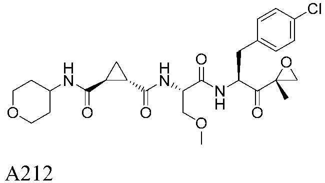 | LCMS-A212: (ESI, *m*/*z*): 536.2 [M+H]⁺ |
| | ¹H NMR-A212 (400 MHz, DMSO-*d*₆) δ 8.46 (dd, *J* = 10.0, 7.9 Hz, 2H), 8.18 (d, *J =* 7.5 Hz, 1H), 7.38 - 7.31 (m, 2H), 7.26 (d, *J* = 8.5 Hz, 2H), 4.51 (dt, *J* = 12.8, 6.3 Hz, 2H), 3.84 - 3.67 (m, 3H), 3.43 (dd, *J* = 9.9, 4.7 Hz, 1H), 3.38 - 3.34 (m, 2H), 3.29 (d, *J =* 2.0 Hz, 1H), 3.24 - 3.17 (m, 4H), 3.01 (d, *J =* 5.1 Hz, 1H), 2.92 (dd, *J =* 14.0, 4.1 Hz, 1H), 2.66 (dd, *J =* 13.9, 9.5 Hz, 1H), 2.12 - 2.05 (m, 1H), 1.96 - 1.86 (m, 1H), 1.68 (d*, J* = 12.8 Hz, 2H), 1.42 - 1.28 (m, 5H), 1.00 (tdd, *J =* 8.4, 5.5, 2.8 Hz, 2H). |
| 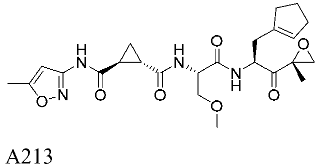 | LCMS-A213: (ESI, *m*/*z*): 489.1 [M+H]⁺ |
| | ¹H NMR-A213 (400 MHz, DMSO-*d*₆) δ 11.24 (s, 1H), 8.53 (d, *J* = 8.2 Hz, 1H), 8.27 (d, *J* = 7.1 Hz, 1H), 6.58 (s, 1H), 5.39 (s, 1H), 4.63 - 4.41 (m, 2H), 3.48 - 3.37 (m, 2H), 3.23 (s, 3H), 3.19 (d, *J* = 5.3 Hz, 1H), 2.98 (d, *J =* 5.2 Hz, 1H), 2.41 (d*, J =* 5.1 Hz, 1H), 2.36 (d, *J* = 0.6 Hz, 3H), 2.29 - 2.25 (m, 1H), 2.25 - 2.16 (m, 6H), 1.82 - 1.74 (m, 2H), 1.37 (s, 3H), 1.18 - 1.13 (m, 2H). |
| 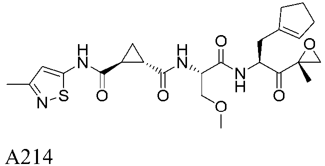 | LCMS-A214: (ESI, *m*/*z*): 505.3 [M+H]⁺ |
| | ¹H NMR-A214 (400 MHz, DMSO-*d*₆) δ 12.16 (s, 1H), 8.56 (d, *J* = 8.0 Hz, 1H), 8.27 (d, *J =* 7.2 Hz, 1H), 6.67 (s, 1H), 5.39 (s, 1H), 4.52 (dt, *J* = 14.1, 8.2 Hz, 2H), 3.46 (dd, *J =* 9.9, 4.7 Hz, 1H), 3.43 - 3.37 (m, 1H), 3.23 (s, 3H), 3.18 (d*, J* = 5.1 Hz, 1H), 2.98 (d, *J =* 5.3 Hz, 1H), 2.36 (ddd, *J* = 9.5, 8.4, 3.3 Hz, 2H), 2.30 (s, 3H), 2.19 (ddd, *J* = 9.3, 6.8, 3.1 Hz, 6H), 1.82 - 1.72 (m, 2H), 1.38 (d, *J* = 7.0 Hz, 3H), 1.29 - 1.20 (m, 2H). |
| 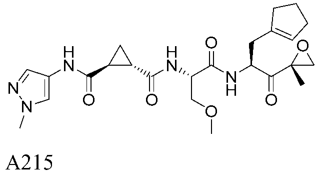 | LCMS-A215: (ESI, *m*/*z*): 488.2 [M+H]⁺ |
| | ¹H NMR-A215 (400 MHz, DMSO-*d*₆) δ 10.26 (s, 1H), 8.50 (d, *J* = 8.1 Hz, 1H), 8.26 (d*, J* = 7.1 Hz, 1H), 7.80 (s, 1H), 7.36 (s, 1H), 5.39 (s, 1H), 4.50 (ddd, *J =* 20.8, 14.2, 6.5 Hz, 2H), 3.77 (s, 3H), 3.47 - 3.36 (m, 2H), 3.23 (s, 3H), 3.19 (d, *J =* 5.3 Hz, 1H), 2.98 (d, *J* = 5.3 Hz, 1H), 2.43 - 2.35 (m, 1H), 2.26 - 2.16 (m, 6H), 2.06 - 2.00 (m, 1H), 1.82 - 1.74 (m, 2H), 1.37 (s, 3H), 1.14 - 1.07 (m, 2H). |
| 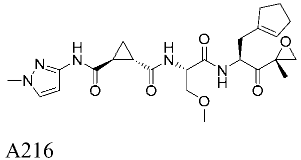 | LCMS-A216: (ESI, *m*/*z*): 488.2 [M+H]⁺ |
| | ¹H NMR-A216 (400 MHz, DMSO-*d*₆) δ 10.69 (s, 1H), 8.49 (d, *J* = 8.2 Hz, 1H), 8.26 (d, *J =* 7.0 Hz, 1H), 7.51 (d, *J =* 2.2 Hz, 1H), 6.39 (d, *J =* 2.2 Hz, 1H), 5.40 (s, 1H), 4.59 - 4.40 (m, 2H), 3.72 (s, 3H), 3.45 (dd, *J =* 10.0, 4.8 Hz, 1H), 3.38 (dd, *J* = 9.9*,* 7.4 Hz, 1H), 3.23 (d, *J =* 1.7 Hz, 3H), 3.20 (d, *J =* 5.8 Hz, 1H), 2.98 (d, *J* = 5.2 Hz, 1H), 2.38 (d*, J =* 14.5 Hz, 1H), 2.28 - 2.12 (m, 7H), 1.78 (p, *J =* 7.6 Hz, 2H), 1.37 (s, 3H), 1.09 (dd, *J =* 10.2, 4.0 Hz, 2H). |
| 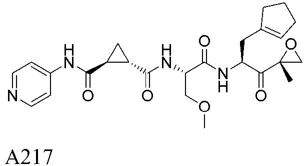 | LCMS-A217: (ESI, *m*/*z*): 485.2 [M+H]⁺ |
| | ¹H NMR-A217 (400 MHz, DMSO-*d*₆) δ 10.70 (s, 1H), 8.55 (d, *J* = 8.0 Hz, 1H), 8.41 (d, *J =* 6.2 Hz, 2H), 8.26 (d, *J =* 7.0 Hz, 1H), 7.53 (d, *J =* 6.3 Hz, 2H), 5.39 (s, 1H), 4.51 (ddd, *J =* 25.0, 12.7, 7.6 Hz, 2H), 3.46 (dd, *J =* 10.0, 4.7 Hz, 1H), 3.42 - 3.38 (m, 1H), 3.24 (d, *J =* 6.2 Hz, 3H), 3.18 (d, *J =* 5.2 Hz, 1H), 2.98 (d, *J =* 5.3 Hz, 1H), 2.39 (d, *J =* 10.3 Hz, 1H), 2.28 (dd, *J =* 7.8, 4.6 Hz, 1H), 2.25 - 2.13 (m, 6H), 1.81 - 1.72 (m, 2H), 1.37 (s, 3H), 1.20 (td, *J* = 8.9, 5.5 Hz, 2H). |
| 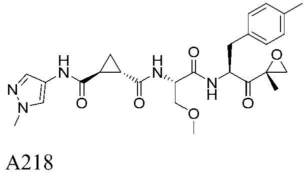 | LCMS-A218: (ESI, *m*/*z*): 512.2 [M+H]⁺ |
| 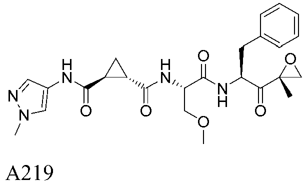 | LCMS-A219: (ESI, *m*/*z*): 498.2 [M+H]⁺ |
| 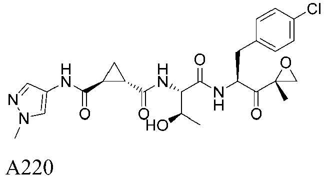 | LCMS-A220: (ESI, *m*/*z*): 532.2 [M+H]⁺ |
| 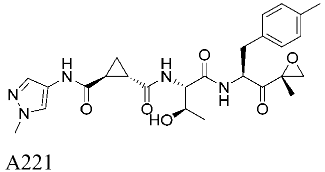 | LCMS-A221: (ESI, *m*/*z*): 512.3 [M+H]⁺ |
| 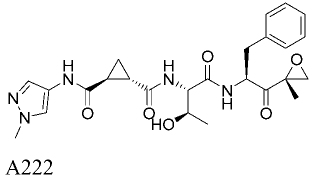 | LCMS-A222: (ESI, *m*/*z*): 498.1 [M+H]⁺ |

### Biological activity test examples

Test of proteasome inhibition activity

### Experimental instruments:

| **Name** | **Model** | **Manufacturer** |
|---|---|---|
| Centrifuge | 5810R | Eppendorf |
| Liquid handler | ECHO 555 | Labcyte |
| Microplate reader | Envision2105 | PerkinElmer |

### Reagents and materials:

| **Name** | **Article number** | **Manufacturer** |
|---|---|---|
| Human cPS | E-360 | R&D |
| Human iPS | BML-PW9645 | Enzolife |
| Ac-nLPnLD-AMC | 4030084.0025 | Bachem |
| Ac-Arg-Leu-Arg-AMC | 4053458.0005 | Bachem |
| (Ac-PAL)2R110 | ABD-13467 | Aatbio |
| Suc-LLVY-AMC | 4011369.0025 | Bachem |
| Assay plate | 4514 | Corning |
| HEPES | 15630-080 | Invitrogen |
| DTT | 43815-1g | Sigma |
| 10% SDS | AM9822 | Invitrogen |

### Experimental steps and methods

1. The compounds were dissolved to give a concentration of 10 mM, and all the test compounds were diluted to 6.67 mM (6 uL of 10 mM stock solution+3 ul of DMSO) on the compound plate respectively.
2. An intermediate dilution plate and a test plate were prepared, and 3-fold dilutions were prepared (the highest concentration was 30 uM) with ECHO according to the plate distribution. 50 nL compound/DMSO was transferred to the test plate.
3. Preparation of reaction buffer

| | Final concentration of reaction solution | Concentration of stock solution | Volume (uL) |
|---|---|---|---|
| HEPES | 100 mM | 1000 mM | 3000 |
| NaCl | 50 mM | 5000 mM | 300 |
| SDS | 0.035% | 10% | 105 |
| DTT | 1 mM | 1000 mM | 30 |
| H₂O | - | - | 26565 |
| Total | pH | 7.50 | 30000 |

4. Protease preparation

| Name | Total volume (ul) | Buffer (ul) | Enzyme stock solution (ul) | Concentration of enzyme stock solution (nM) | Prepared enzyme concentration (nM) | Final enzyme concentration in reaction (nM) |
|---|---|---|---|---|---|---|
| Human cPS | 3000 | 2995.8 | 4.25 | 1412.86 | 2.00 | 1.00 |
| Human iPS | 3000 | 2995.8 | 4.20 | 1428.57 | 2.00 | 1.00 |

5. Substrate preparation

| Substrate name | Total volume (ul) | Buffer (ul) | Substrate stock solution (uL) | Concentration of substrate stock solution (uM) | Prepared substrate concentration (uM) | Substrate concentration in reaction (uM) | Name of experiment |
|---|---|---|---|---|---|---|---|
| Ac-nLPnLD-AMC | 2009.50 | 2005.00 | 4.50 | 50000.00 | 112.00 | 56.00 | Human cPS β1 Inhibition Assay |
| Ac-Arg-Leu-Arg-AMC | 2000.00 | 1996.00 | 4.00 | 10000.00 | 20.00 | 10.00 | Human cPS β2 Inhibition Assay |
| Suc-LLVY-AMC | 2212.00 | 2210.00 | 2.00 | 50000.00 | 45.20 | 22.60 | Human cPS β5 Inhibition Assay |
| (Ac-PAL)2R110 | 2049.00 | 2048.00 | 1.00 | 10000.00 | 4.88 | 2.44 | Human iPS β1 Inhibition Assay |
| Ac-Arg-Leu-Arg-AMC | 2000.00 | 1992.00 | 8.00 | 10000.00 | 40.00 | 20.00 | Human iPS β2 Inhibition Assay |
| Suc-LLVY-AMC | 2051.20 | 2048.00 | 3.20 | 50000.00 | 78.00 | 39.00 | Human iPS β5 Inhibition Assay |

### Experimental stage:

1. According to the test plate distribution, 5 uL of the reaction buffer was added to each Low control wells of the test plate by an electronic pipette.
2. According to the test plate distribution, 5 uL/well of the corresponding 2 nM enzyme reaction solution was added to other wells than the Low control wells of the test plate by an electronic pipette, and centrifuged at 1000 rpm for 1 min.
3. According to the test plate distribution, 5 uL/well of the corresponding substrate solution prepared was added to all the wells of the test plate by an electronic pipette, and centrifuged at 1000 rpm for 1 min.
4. The plate was sealed with aluminum foil, and incubated in an incubator at 37°C for 1 hr.

**Detection:** After incubation at 37°C for 1 hr, the fluorescence intensity was measured on a microplate reader (excitation at 380 nm and emission at 460 nm of the substrate with AMC, excitation at 485 nm and emission at 535 nm of the substrate with R110). The data was analyzed by Prism. The results are shown in Table 1.

**Table 1. Activity data (IC₅₀)**

| Compound | β5c (nM) | β5i (nM) | β1c (nM) | β1i (nM) |
|---|---|---|---|---|
| A001 | 30000 | 4521 | 30000 | 2100 |
| A002 | 24530 | 2626 | 30000 | 68 |
| A004 | 631 | 249 | 30000 | 188 |
| A006 | 4882 | 717 | 30000 | 555 |
| A008 | 2543 | 206 | 30000 | 411 |
| A009 | 30000 | 2512 | 30000 | 4922 |
| A010 | 574 | 42 | 30000 | 339 |
| A012 | 1221 | 53 | 30000 | 378 |
| A013 | 11328 | 532 | 30000 | 871 |
| A015 | 1740 | 207 | 30000 | 453 |
| A017 | 16871 | 3696 | 30000 | 1362 |
| A019 | 30000 | 2599 | 30000 | 89 |
| A020 | 2090 | 617 | 30000 | 15129 |
| A022 | 150 | 30 | 30000 | 513 |
| A023 | 2009 | 849 | 30000 | 161 |
| A028 | 29534 | 3214 | 30000 | 371 |
| A029 | 30000 | 7152 | 30000 | 248 |
| A030 | 3227 | 1038 | 30000 | 490 |
| A031 | 1659 | 162 | 30000 | 254 |
| A032 | 5630 | 2450 | 30000 | 1140 |
| A034 | 2363 | 457 | 30000 | 1984 |
| A036 | 973 | 68 | 30000 | 747 |
| A038 | 22784 | 1667 | 30000 | 1367 |
| A040 | 27616 | 1301 | 30000 | 78 |
| A042 | 404 | 103 | 20849 | 720 |
| A043 | 2311 | 495 | 30000 | 188 |
| A053 | 30000 | 3086 | 30000 | 3119 |
| A055 | 4538 | 382 | 30000 | 12898 |
| A057 | 481 | 66 | 30000 | 111 |
| A059 | 14825 | 147 | 30000 | 90 |
| A061 | 945 | 17 | 30000 | 72 |
| A062 | 6595 | 132 | 30000 | 692 |
| A064 | 566 | 86 | 30000 | 197 |
| A066 | 968 | 240 | 30000 | 167 |
| A068 | 6181 | 497 | 30000 | 604 |
| A070 | 30000 | 1664 | 30000 | 85 |
| A073 | 4960 | 245 | 30000 | 679 |
| A075 | 18675 | 1085 | 30000 | 1593 |
| A077 | 850 | 234 | 30000 | 353 |
| A079 | 25755 | 9342 | 30000 | 823 |
| A080 | 20765 | 3953 | 30000 | 421 |
| A095 | 17756 | 1171 | 30000 | 976 |
| A098 | 1169 | 90 | 30000 | 30000 |
| A100 | 1026 | 168 | 30000 | 21811 |
| A102 | 30000 | 13173 | 30000 | 2166 |
| A103 | 30000 | 9161 | 30000 | 15660 |
| A105 | 15181 | 2056 | 30000 | 150 |
| A107 | 8190 | 1359 | 30000 | 262 |
| A109 | 30000 | 2497 | 30000 | 224 |
| A110 | 3081 | 984 | 30000 | 74 |
| A115 | 30000 | 964 | 30000 | 145 |
| A117 | 30000 | 279 | 30000 | 270 |
| A119 | 642 | 161 | 30000 | 311 |
| A120 | 30000 | 429 | 30000 | 158 |
| A121 | 30000 | 30000 | 30000 | 536 |
| A122 | 30000 | 8596 | 30000 | 460 |
| A135 | 30000 | 5422 | 30000 | 344 |
| A137 | 30000 | 291 | 30000 | 30000 |
| A141 | 30000 | 9232 | 30000 | 51 |
| A142 | 10292 | 12086 | 30000 | 2312 |
| A143 | 2785 | 1667 | 30000 | 250 |
| A145 | 6299 | 7051 | 30000 | 4660 |
| A147 | 2366 | 2382 | 30000 | 294 |
| A148 | 8985 | 6148 | 30000 | 1053 |
| A150 | 2667 | 1642 | 30000 | 1403 |
| A151 | 938 | 322 | 30000 | 738 |
| A155 | 30000 | 3218 | 30000 | 6866 |
| A157 | 30000 | 3765 | 30000 | 835 |
| A158 | 30000 | 2081 | / | 16174 |
| A160 | 30000 | 1056 | / | 30000 |
| A161 | 30000 | 1401 | / | 104 |
| A162 | 2386 | 1657 | / | 30000 |
| A163 | 2614 | 254 | / | 354 |
| A164 | 30000 | 8283 | / | 98 |
| A165 | 30000 | 7873 | / | 1522 |
| A168 | 3933 | 2303 | / | 3123 |
| A169 | 30000 | 1811 | / | 30000 |
| A170 | 13392 | 1702 | / | 334 |
| A171 | 30000 | 4406 | / | 5971 |
| A172 | 30000 | 4880 | / | 934 |
| A173 | 8593 | 3060 | / | 499 |
| A174P1 | 9288 | 1765 | / | 30000 |
| A175 | 4567 | 1575 | / | 405 |
| A176 | 30000 | 2947 | / | 30000 |
| A177 | 2096 | 707 | / | 1339 |
| A178 | 768 | 54 | / | 1300 |
| A179 | 12062 | 2105 | / | 1589 |
| A182 | 1092 | 141 | / | 510 |
| A183 | 7767 | 3107 | / | 182 |
| A184 | 3158 | 2422 | / | 199 |
| A194 | / | 681 | / | / |
| A195 | 1044 | 95 | / | 368 |
| A196 | 1086 | 317 | / | 1031 |
| A197 | >30000 | 160 | / | 238 |
| A198 | 759 | 16 | / | 1105 |
| A199 | 3011 | 30 | / | 556 |
| A200 | 399 | 11 | / | 357 |
| A201 | 1748 | 30 | / | 96 |
| A202 | 2277 | 69 | / | 446 |
| A203 | 2635 | 140 | / | 568 |
| A207 | 336 | 23 | / | 2094 |
| A208 | 579 | 10 | / | 610 |
| A209 | 5806 | 33 | / | 315 |
| A210 | 1044 | 35 | / | 2555 |
| A211 | 17998 | 115 | / | 863 |
| A212 | 804 | 99 | / | 248 |
| A214 | 3242 | 390 | / | 13 |
| A215 | >30000 | 29 | / | 70 |
| A217 | 6721 | 21 | / | 3 |

| | | | | |
|---|---|---|---|---|
| Note: "/"means no measurement or the value is greater than 30000. | | | | |

The above results show that the compound of the present invention has high inhibitory activity on β5i and β1i, but low inhibitory level on β5c and β1c. Therefore, the compound of the present invention has high selectivity.

In-vivo pharmacodynamic experiment: The inhibitory effect of the compound on cytokines in C57BL/6J mice after in-vivo administration and in-vitro stimulation was evaluated.

Experimental method: Female C57BL/6J mice aged 6-8 weeks were randomly divided into 6 groups (n=3), including a control group (given blank vehicle) and compound groups (subcutaneously injecting 2.5 mpk or orally taking 40 mpk). 4 hrs after administration, all animals were anesthetized with 3-5% isoflurane gas, blood was taken from the heart, and > 500 µl of whole blood anticoagulated with heparin sodium was taken. BMC was extracted from whole blood by Ficoll density gradient centrifugation. After the extraction, appropriate amount of PRMI 1640 complete medium was added to re-suspend the cell precipitate. The cell suspension prepared above was into a 24-well plate, 10 µL of 10 µg/mL LPS working solution (final concentration 100 ng/mL) was added, and gently blown and mixed at 37°C. The cells were then cultured at 5% CO₂ for 12 hrs. After the culture, the supernatant was taken and the concentrations of cytokines IL-6 and TNF-α were detected by flow CBA. The data was processed in Office Excel 2013 and GraphPad Prism 9.0, and the values were expressed as a percentage relative to the vehicle control group.

Experimental results: The compound of the present invention can effectively inhibit the cytokines IL-6 and TNF-α after subcutaneous injection or oral administration.

## Claims

1. An epoxy compound of Formula I, or a pharmaceutically acceptable salt or a stereoisomer thereof, 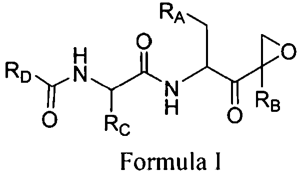 wherein in Formula I,
R_{A} is C₁₋₆ alkyl, cyclopentenyl, cyclohexenyl, cyclohexyl, cyclopentyl, or C₆₋₁₄ aryl, in which the cyclopentenyl, cyclohexenyl, cyclohexyl, cyclopentyl and C₆₋₁₄ aryl are optionally substituted with one or more R_{A1};
R_{A1} is independently C₁₋₆ alkyl, C₁₋₆ alkoxy, halogen, hydroxyl, or C₁₋₆ haloalkyl;
R_{B} is C₁₋₆ alkyl or C₁₋₆ haloalkyl;
R_{C} is 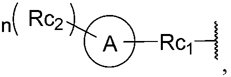 C₁₋₆ alkyl, or C₁₋₆ alkyl substituted with one or more Rc₃;
ring A is phenyl, 4- to 7-membered heterocycloalkyl, 8- to 10-membered heterocycloalkyl, 5- to 8-membered heteroaryl, C₄₋₇ cycloalkyl, pyridinonyl or C₄₋₇ cycloalkenyl;
Rc₁ is C₁₋₆ alkylene optionally substituted with one or more R_{C1-1}; R_{C1-1} is hydroxyl, C₁₋₆ alkoxy, or halogen;
Rc₂ is C₁₋₆ alkyl, C₁₋₆ alkoxy, halogen, hydroxyl, C₃₋₆ cycloalkoxy, NH(C₁₋₆ alkyl), or N(C₁₋₆ alkyl)₂; or two adjacent Rc₂ form, together with the atoms to which they are attached, a 4- to 6-membered ring;
Rc₃ is independently hydroxyl, halogen, C₁₋₆ alkoxy, or C₁₋₆ alkoxy substituted with one or more Rc₃₋₁;
Rc₃₋₁ is independently C₁₋₆ haloalkyl or C₃₋₈cycloalkyl;
R_{D} is 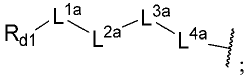
L^{4a} is a bond, C₁₋₆ alkylene, C₂₋₆ alkenylene, C₃₋₈ cycloalkylene, or 3- to 8-membered heterocycloalkylene, in which the C₁₋₆ alkylene, C₂₋₆ alkenylene, C₃₋₈ cycloalkylene, and 3- to 8-membered heterocycloalkylene are optionally substituted with one or more L^{4a-1};
L^{4a-1} is hydroxyl, halogen, oxo (=O), C₁₋₆ alkylsulfonamido, or C₁₋₆ alkoxy;
L^{3a}, L^{2a}, and L^{1a} are each independently a bond, -O-, -NH-, -N(C₁₋₆ alkyl)-, carbonyl (-C=O-), C₁₋₆ alkylene, sulfonyl (-SO₂-), -C(=O)NH-, -NHC(=O)-, or -NHC(=O)NH-;
R_{d1} is C₆₋₁₄ aryl, C₁₋₆ alkyl, C₃₋₁₀ cycloalkyl, 3- to 10-membered heterocycloalkyl, C₃₋₆cycloalkyloxy, 3- to 8-membered heterocycloalkyloxy, 5- to 8-membered heteroaryl, or C₁₋₆ alkylsulfonyl, in which the C₆₋₁₄ aryl, C₁₋₆ alkyl, C₃₋₁₀ cycloalkyl, 3- to 10-membered heterocycloalkyl, C₃₋₆cycloalkyloxy, 3- to 8-membered heterocycloalkyloxy, 5- to 8-membered heteroaryl, and C₁₋₆ alkylsulfonyl are optionally substituted with one or more R_{d1-1};
R_{d1-1} is hydroxyl, halogen, C₁₋₆ alkyl, C₁₋₆ alkoxy, C₁₋₆ haloalkyl, C₁₋₆ haloalkoxy, oxo (=O), C₁₋₆ alkylcarbonyl, C₁₋₆ alkoxycarbonyl, C₁₋₆ alkylsulfonyl, C₃₋₆cycloalkylsulfonyl, 3- to 6-membered heterocycloalkyl, 3- to 6-membered heterocycloalkylmethylene, C₃₋₆cycloalkyl, or C₆₋₁₄ aryl-C₁₋₆ alkylene-;
n is 0, 1, 2 or 3; and
in the 3- to 8-membered heterocycloalkylene, 3- to 10-membered heterocycloalkyl, 3- to 8-membered heterocycloalkyloxy, 4- to 7-membered heterocycloalkyl, 8- to 10-membered heterocycloalkyl, 3- to 6-membered heterocycloalkyl, 3- to 6-membered heterocycloalkylmethylene, and 5- to 8-membered heteroaryl, the number of the heteroatom or heteroatom group is independently 1, 2, 3, or 4, and the heteroatom or heteroatom group is independently one or more of N, O, S, and -SO₂-.

2. The epoxy compound of Formula I according to claim 1, or the pharmaceutically acceptable salt or stereoisomer thereof, wherein R_{A} is cyclopentenyl, cyclohexenyl, cyclohexyl, cyclopentyl, or C₆₋₁₄ aryl, in which the cyclopentenyl, cyclohexenyl, cyclohexyl, cyclopentyl and C₆₋₁₄ aryl are optionally substituted with one or more R_{A1};
R_{A1} is C₁₋₆ alkyl, C₁₋₆ alkoxy, halogen, or hydroxyl;
R_{B} is C₁₋₆ alkyl or C₁₋₆ haloalkyl;
R_{C} is 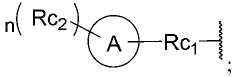
ring A is phenyl, 4- to 7-membered heterocycloalkyl, 5- to 8-membered heteroaryl, or C₄₋₇cycloalkyl;
Rc₁ is C₁₋₆ alkylene optionally substituted with one or more R_{C1-1}; R_{C1-1} is hydroxyl, C₁₋₆ alkoxy, or halogen;
Rc₂ is C₁₋₆ alkyl, C₁₋₆ alkoxy, halogen, hydroxyl, C₃₋₆ cycloalkoxy, NH(C₁₋₆ alkyl), or N(C₁₋₆ alkyl)₂; or two adjacent Rc₂ form, together with the atoms to which they are attached, a 4- to 6-membered ring;
R_{D} is 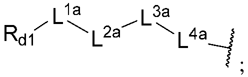
L^{4a} is C₁₋₆ alkylene, C₂₋₆ alkenylene, C₃₋₈ cycloalkylene, or 3- to 8-membered heterocycloalkylene, in which the C₁₋₆ alkylene, C₂₋₆ alkenylene, C₃₋₈ cycloalkylene, and 3- to 8-membered heterocycloalkylene are optionally substituted with one or more L^{4a-1};
L^{4a-1} is hydroxyl, halogen, oxo (=O), C₁₋₆ alkylsulfonamido, or C₁₋₆ alkoxy;
L^{3a}, L^{2a}, and L^{1a} are each independently a bond, -O-, -NH-, -N(C₁₋₆ alkyl)-, carbonyl (-C=O-), C₁₋₆ alkylene, sulfonyl (-SO₂-), -C(=O)NH-, -NHC(=O)-, or -NHC(=O)NH-;
R_{d1} is C₆₋₁₄ aryl, C₁₋₆ alkyl, C₃₋₁₀ cycloalkyl, 3- to 10-membered heterocycloalkyl, C₃₋₆cycloalkyloxy, 3- to 8-membered heterocycloalkyloxy, 5- to 8-membered heteroaryl, or C₁₋₆ alkylsulfonyl, in which the C₆₋₁₄ aryl, C₁₋₆ alkyl, C₃₋₁₀ cycloalkyl, 3- to 10-membered heterocycloalkyl, C₃₋₆cycloalkyloxy, 3- to 8-membered heterocycloalkyloxy, 5- to 8-membered heteroaryl, and C₁₋₆ alkylsulfonyl are optionally substituted with one or more R_{d1-1};
R_{d1-1} is hydroxyl, halogen, C₁₋₆ alkyl, C₁₋₆ alkoxy, C₁₋₆ haloalkyl, C₁₋₆ haloalkoxy, oxo (=O), C₁₋₆ alkylcarbonyl, C₁₋₆ alkoxycarbonyl, C₁₋₆ alkylsulfonyl, C₃₋₆cycloalkylsulfonyl, 3- to 6-membered heterocycloalkyl, 3- to 6-membered heterocycloalkylmethylene, C₃₋₆cycloalkyl, or C₆₋₁₄ aryl-C₁₋₆ alkylene-;
n is 0, 1, 2 or 3; and
in the 3- to 8-membered heterocycloalkylene, 3- to 10-membered heterocycloalkyl, 3- to 8-membered heterocycloalkyloxy, 3- to 6-membered heterocycloalkyl, and 3- to 6-membered heterocycloalkylmethylene, the number of the heteroatom or heteroatom group is independently 1, 2, 3, or 4, and the heteroatom or heteroatom group is independently one or more of N, O, S, and -SO₂-.

3. The epoxy compound of Formula I according to claim 1, or the pharmaceutically acceptable salt or stereoisomer thereof, wherein the epoxy compound of Formula I or the pharmaceutically acceptable salt or stereoisomer thereof meets one of the following conditions:
(1) R_{A} is cyclopentenyl, cyclohexenyl, cyclohexyl, cyclopentyl or phenyl, in which the cyclopentenyl, cyclohexenyl, cyclohexyl, cyclopentyl and phenyl are optionally substituted with one or more R_{A1}; R_{A1} is C₁₋₆ haloalkyl; or R_{A} is C₁₋₆ alkyl;
(2) R_{C} is C₁₋₆ alkyl, or C₁₋₆ alkyl substituted with one or more Rc₃; Rc₃ is independently hydroxyl, halogen, C₁₋₆ alkoxy, or C₁₋₆ alkoxy substituted with one or more Rc₃₋₁; and Rc₃₋₁ is independently C₁₋₆ haloalkyl or C₃₋₈cycloalkyl;
(3) ring A is 8- to 10-membered heterocycloalkyl, pyridinonyl or C₄₋₇ cycloalkenyl; and
(4) L^{4a} is a bond.

4. The epoxy compound of Formula I according to claim 1 or 2, or the pharmaceutically acceptable salt or stereoisomer thereof, wherein the epoxy compound of Formula I or the pharmaceutically acceptable salt or stereoisomer thereof meets one of the following conditions:
(1) R_{A} is cyclopentenyl, cyclohexenyl, cyclohexyl, cyclopentyl or phenyl, in which the cyclopentenyl, cyclohexenyl, cyclohexyl, cyclopentyl and phenyl are optionally substituted with one or more R_{A1}; and R_{A1} is C₁₋₆ alkyl, C₁₋₆ alkoxy, halogen, or hydroxyl;
(2) R_{B} is methyl, ethyl, or trifluoromethyl;
(3) R_{C} is 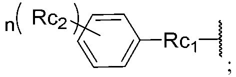
(4) when L^{4a} is C₃₋₈ cycloalkylene optionally substituted with one or more L^{4a-1}, the C₃₋₈ cycloalkylene is C₃₋₆ monocycloalkylene or C₅₋₈ spirocycloalkylene;
(5) when L^{4a} is 3- to 8-membered heterocycloalkylene optionally substituted with one or more L^{4a-1}, the 3- to 8-membered heterocycloalkylene is 3- to 6-membered heteromonocycloalkylene or 5- to 8-membered heterospirocycloalkylene;
(6) when R_{d1} is C₃₋₁₀cycloalkyl optionally substituted with one or more R_{d1-1}, the C₃₋₁₀cycloalkyl is C₃₋₆ monocycloalkyl or C₅₋₁₀ spirocycloalkyl; and
(7) when R_{d1} is 3- to 10-membered heterocycloalkyl optionally substituted with one or more R_{d1-1}, the 3- to 10-membered heterocycloalkyl is 3- to 8-membered heteromonocycloalkyl or 5- to 10-membered heterospirocycloalkyl.

5. The epoxy compound of Formula I according to claim 1, or the pharmaceutically acceptable salt or stereoisomer thereof, wherein
(1) R_{A} is isopropyl, cyclopentenyl, cyclohexenyl, cyclohexyl, cyclopentyl or phenyl, in which the cyclopentenyl, cyclohexenyl, cyclohexyl, cyclopentyl and phenyl are optionally substituted with one or more R_{A1}; and R_{A1} is ethyl, isopropyl, methoxy, ethoxy, or trifluoromethyl;
(2) R_{C} is methyl, ethyl, or t-butyl, in which the methyl, ethyl and t-butyl are optionally substituted with one or more Rc₃; R_{C3} is independently hydroxyl, or methoxy substituted with one or more R_{C3-1}; R_{C3-1} is independently trifluoromethyl or cyclopropyl; and
(3) L^{4a} is a bond, cyclopentylene, or azacyclohexylene substituted with one or more L^{4a-1}.

6. The epoxy compound of Formula I according to claim 1 or 2, or the pharmaceutically acceptable salt or stereoisomer thereof, wherein the epoxy compound of Formula I or the pharmaceutically acceptable salt or stereoisomer thereof meets one of the following conditions:
(1) R_{A} is cyclopentenyl, cyclohexenyl, cyclohexyl, cyclopentyl or phenyl, in which the cyclopentenyl, cyclohexenyl, cyclohexyl, cyclopentyl and phenyl are optionally substituted with one or more R_{A1}; and R_{A1} is methyl, halogen, or hydroxyl;
(2) Rc₁ is methylene optionally substituted with one or more R_{C1-1}, in which the R_{C1-1} is hydroxyl or halogen;
(3) Rc₂ is methyl, methoxy, halogen, hydroxyl, NH(C₁₋₆ alkyl), or N(C₁₋₆ alkyl)₂; or two Rc₂ form, together with the atoms to which they are attached, a 4-membered ring, a 5-membered ring or a 6-membered ring;
(4) L^{4a} is cyclopropylene, cyclobutylene, oxacyclobutylene, azacyclobutylene, azacyclohexylene, C₅₋₈ spirocycloalkylene, or 5- to 8-membered heterospirocycloalkylene; and
(5) L^{3a}, L^{2a}, and L^{1a} are each independently selected from a bond, -O-, -NH-, -N(CH₃)-, carbonyl, C₁₋₆ alkylene, sulfonyl, -C(=O)NH-, or -NHC(=O)NH-.

7. The epoxy compound of Formula I according to claim 1, or the pharmaceutically acceptable salt or stereoisomer thereof, wherein the epoxy compound of Formula I or the pharmaceutically acceptable salt or stereoisomer thereof meets one of the following conditions:
(1) R_{A} is 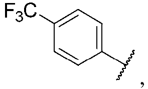 cyclopentyl, 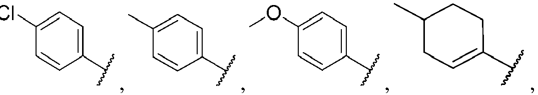 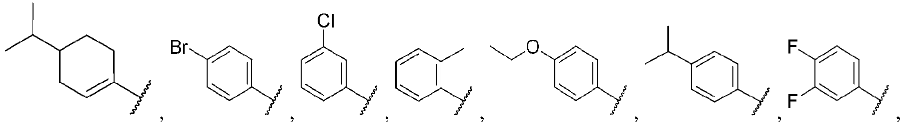 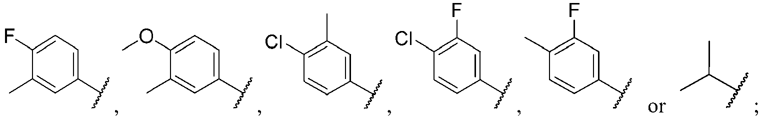
(2) R_{C} is 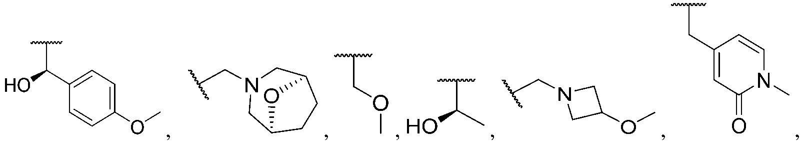 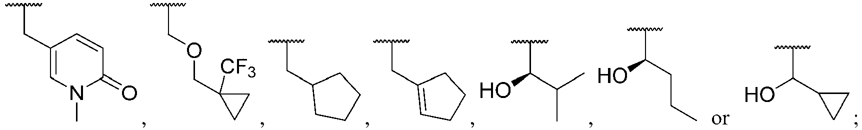
(3) L^{4a} is a bond, 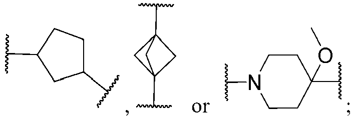
(4) R_{d1} is 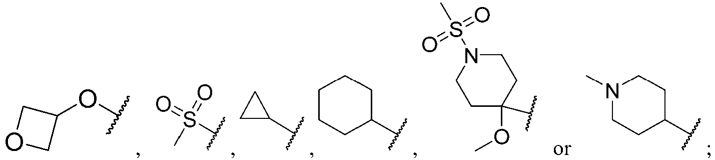 and
(5) the structural unit 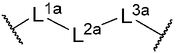 is methylene or

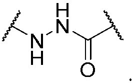

8. The epoxy compound of Formula I according to claim 1 or 2, or the pharmaceutically acceptable salt or stereoisomer thereof, wherein the epoxy compound of Formula I or the pharmaceutically acceptable salt or stereoisomer thereof meets one of the following conditions:
(1) R_{A} is 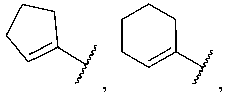 cyclohexyl, phenyl, 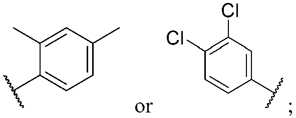
(2) R_{C} is 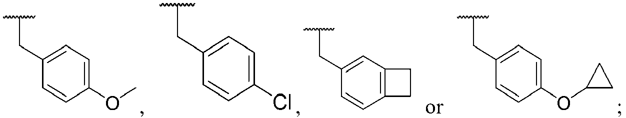
(3) R_{d1} is 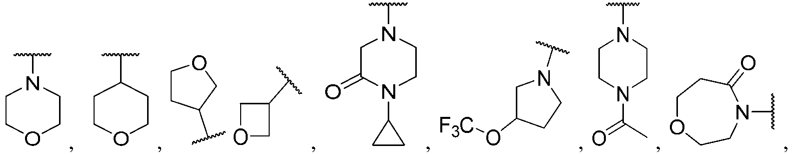 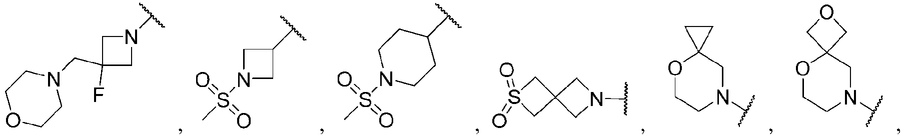 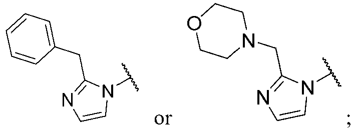 and
(4) the structural unit 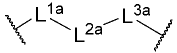 is a bond, NH-,

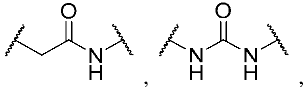

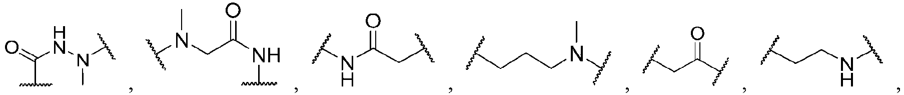

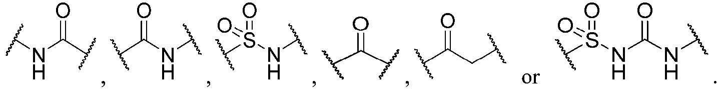

9. The epoxy compound of Formula I according to claim 1, or the pharmaceutically acceptable salt or stereoisomer thereof, wherein Formula I has a structure of Formula I-d1: 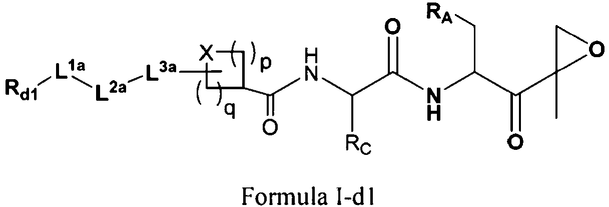 wherein
R_{A}, R_{C}, L^{3a}, L^{2a}, L^{1a} and R_{d1} are as defined in claim 1;
p and q are independently 0, 1, 2 or 3, and p and q are not both 0; and X is CH₂, NH, O or S.

10. The epoxy compound of Formula I according to claim 9, or the pharmaceutically acceptable salt or stereoisomer thereof, wherein Formula I has a structure of Formula I-d1b: 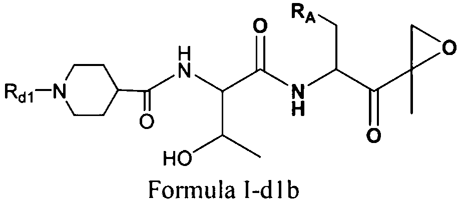 wherein R_{A} and R_{d1} are as defined in claim 9.

11. The epoxy compound of Formula I according to claim 1, or the pharmaceutically acceptable salt or stereoisomer thereof, wherein Formula I has a structure of Formula I-d2: 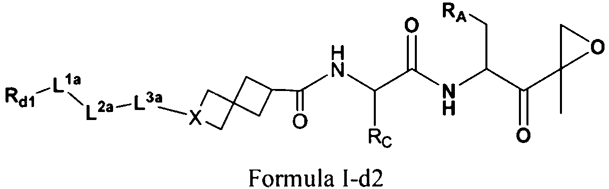
wherein R_{A}, R_{C}, L^{3a}, L^{2a}, L^{1a} and R_{d1} are as defined in claim 1; and
X is CH or N.

12. The epoxy compound of Formula I according to claim 1, or the pharmaceutically acceptable salt or stereoisomer thereof, wherein Formula I has a structure of Formula I-d3: 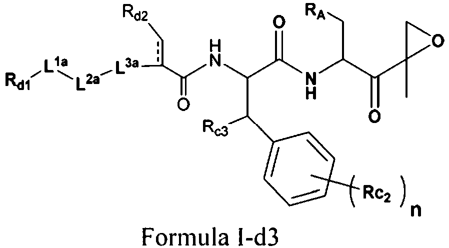
wherein R_{A}, Rc₂, L^{3a}, L^{2a}, L^{1a}, R_{d1} and n are as defined in claim 1;
R_{d2} is hydrogen, hydroxyl, halogen, oxo, C₁₋₆ alkylsulfonamido, or C₁₋₆ alkoxy;
Rc₃ is hydrogen, hydroxyl, C₁₋₆ alkoxy, or halogen; and
"-̅ -̅ -̅" represents a single bond or a double bond.

13. The epoxy compound of Formula I according to claim 1, or the pharmaceutically acceptable salt or stereoisomer thereof, wherein the epoxy compound of Formula I is selected from any compound in Table A.

14. A pharmaceutical composition, comprising the epoxy compound of Formula I according to any one of claims 1 to 13, or the pharmaceutically acceptable salt or stereoisomer thereof, and a pharmaceutically acceptable adjuvant.

15. Use of the epoxy compound of Formula I according to any one of claims 1 to 13 or the pharmaceutically acceptable salt or stereoisomer thereof in the preparation of drugs for inhibiting immunoproteasome in cells, wherein the drugs are applicable to the treatment of autoimmune diseases.
